# EUROPEAN PATENT SPECIFICATION

(11) **EP 1 651 667 B1**
(45) Date of publication and mention of the grant of the patent: **28.03.2012**
(21) Application number: 04725350.5
(22) Date of filing: 02.04.2004
(51) Int. Cl.: C07K 14/15, A61K 35/00, A61K 48/00

(54) **PROTEIN WITH FUSOGENIC ACTIVITY, NUCLEIC ACID SEQUENCES ENCODING SAID PROTEIN AND PHARMACEUTICAL COMPOSITIONS COMPRISING THE SAME**
PROTEIN MIT FUSOGENER WIRKUNG, NUKLEINSÄURESEQUENZEN, DIE FÜR DIESES PROTEIN CODIEREN UND PHARMAZEUTISCHE ZUSAMMENSETZUNGEN, DIE DIESES ENTHALTEN
PROTEINE A ACTIVITE FUSOGENE, SEQUENCES D'ACIDES NUCLEIQUES CODANT CETTE PROTEINE ET COMPOSITIONS PHARMACEUTIQUES LES CONTENANT

(30) Priority: 04.04.2003 US 459974 P
(43) Date of publication of application: 03.05.2006
(73) Proprietor: CENTRE NATIONAL DE LA RECHERCHE SCIENTIFIQUE, 75794 Paris Cedex 16 (FR); I.N.S.E.R.M. Institut National de la Santé et de la Recherche Médicale, 75654 Paris Cedex 13 (FR); Institut Gustave Roussy, 94805 Villejuif Cédex (FR)
(72) Inventor: HEIDMANN, Thierry, F-75017 Paris (FR); DEWANNIEUX, Marie, F-75013 Paris (FR); BLAISE, Sandra, F-91600 Savigny Sur Orge (FR); BENIT, Laurence, F-91120 Palaiseau (FR); DE PARSEVAL, Nathalie, F-75011 Paris (FR); LAZAR, Vladimir, F-94800 Villejuif (FR); MANGENEY, Marianne, F-75014 Paris (FR)
(74) Representative: Grosset-Fournier, Chantal Catherine
(86) International application number: PCT/EP2004/003552
(87) International publication number: WO 2004/087748

(56) References cited:
- WO-A-01/16171
- WO-A-01/75067
- WO-A-02/068579
- WO-A-02/070539
- BLAISE SANDRA ET AL: "Genomewide screening for fusogenic human endogenous retrovirus envelopes identifies syncytin 2, a gene conserved on primate evolution." PROCEEDINGS OF THE NATIONAL ACADEMY OF SCIENCES OF THE UNITED STATES OF AMERICA, vol. 100, no. 22, 28 October 2003 (2003-10-28), pages 13013-13018, XP002289560 ISSN: 0027-8424
- LAVILLETTE DIMITRI ET AL: "The envelope glycoprotein of human endogenous retrovirus type W uses a divergent family of amino acid transporters/cell surface receptors" JOURNAL OF VIROLOGY, vol. 76, no. 13, July 2002 (2002-07), pages 6442-6452, XP002289561 ISSN: 0022-538X
- BENIT LAURENCE ET AL: "Identification, phylogeny, and evolution of retroviral elements based on their envelope genes" JOURNAL OF VIROLOGY, vol. 75, no. 23, December 2001 (2001-12), pages 11709-11719, XP002289562 ISSN: 0022-538X

### SEQUENCE LISTING

<110> INSTITUT GUSTAVE ROUSSY
<120> PROTEIN WITH FUSOGENIC ACTIVITY, NUCLEIC ACID SEQUENCES ENCODING SAID PROTEIN AND PHARMACEUTICAL COMPOSITIONS COMPRISING THE SAME
<130> WOB 03 BB IGR HERV
<160> 69
<170> PatentIn version 3.1
<210> 1
   <211> 1617
   <212> DNA
   <213> Homo sapiens
<220>
   <221> CDS
   <222> (1)..(1617)
   <223>
<400> 1
   atg ggc ctg ctc ctg ctg gtt ctc att ctc acg cct tca cta gca gcc      48
   Met Gly Leu Leu Leu Leu Val Leu Ile Leu Thr Pro Ser Leu Ala Ala
   1               5                   10                  15
   tac cgc cat cct gat ttc ccg tta ttg gaa aaa gct cag caa ctg ctc      96
   Tyr Arg His Pro Asp Phe Pro Leu Leu Glu Lys Ala Gln Gln Leu Leu
               20                  25                  30
   caa agt aca gga tcc cct tac tcc acc aat tgc tgg tta tgt act agc     144
   Gln Ser Thr Gly Ser Pro Tyr Ser Thr Asn Cys Trp Leu Cys Thr Ser
           35                  40                  45
   tct tcc act gaa aca cca ggg aca gct tat cca gcc tcg ccc aga gaa     192
   Ser Ser Thr Glu Thr Pro Gly Thr Ala Tyr Pro Ala Ser Pro Arg Glu
       50                  55                  60
   tgg aca agc ata gag gcg gaa tta cat att tcc tat cga tgg gac cct     240
   Trp Thr Ser Ile Glu Ala Glu Leu His Ile Ser Tyr Arg Trp Asp Pro
   65                  70                  75                  80
   aat ctg aaa gga ctg atg agg cct gca aat agt ctt ctt tca aca gta     288
   Asn Leu Lys Gly Leu Met Arg Pro Ala Asn Ser Leu Leu Ser Thr Val
                   85                  90                  95
   aag caa gat ttc cct gat atc cgc cag aaa cct ccc att ttc gga ccc     336
   Lys Gln Asp Phe Pro Asp Ile Arg Gln Lys Pro Pro Ile Phe Gly Pro
               100                 105                 110
   atc ttt act aat atc aac cta atg gga ata gcc cct att tgt gtt atg     384
   Ile Phe Thr Asn Ile Asn Leu Met Gly Ile Ala Pro Ile Cys Val Met
           115                 120                 125
   gcc aaa agg aaa aat gga aca aat gta ggc act ctt cca agt aca gtc     432
   Ala Lys Arg Lys Asn Gly Thr Asn Val Gly Thr Leu Pro Ser Thr Val
       130                 135                 140
   tgt aat gtt act ttc act gta gat tct aac caa cag act tac caa aca     480
   Cys Asn Val Thr Phe Thr Val Asp Ser Asn Gln Gln Thr Tyr Gln Thr
   145                 150                 155                 160
   tac acc cac aac caa ttc cgc cat caa cca aga ttc ccc aaa cct cca     528
   Tyr Thr His Asn Gln Phe Arg His Gln Pro Arg Phe Pro Lys Pro Pro
                   165                 170                 175
   aat att act ttt cct cag gga act ttg cta gat aaa tcc agc cgg ttt      576
   Asn Ile Thr Phe Pro Gln Gly Thr Leu Leu Asp Lys Ser Ser Arg Phe
               180                 185                 190
   tgc cag gga cgc cca agc tca tgc agt act cga aac ttc tgg ttc cgg      624
   Cys Gln Gly Arg Pro Ser Ser Cys Ser Thr Arg Asn Phe Trp Phe Arg
           195                 200                 205
   cct gct gat tat aac caa tgt ctg caa att tcc aac ctc agc tct aca      672
   Pro Ala Asp Tyr Asn Gln Cys Leu Gln Ile Ser Asn Leu Ser Ser Thr
       210                 215                 220
   gcg gaa tgg gtt cta ttg gac caa act cga aat tct ctt ttt tgg gaa      720
   Ala Glu Trp Val Leu Leu Asp Gln Thr Arg Asn Ser Leu Phe Trp Glu
   225                 230                 235                 240
   aat aaa acc aag gga gct aac cag agc caa aca ccc tgc gtc caa gtc      768
   Asn Lys Thr Lys Gly Ala Asn Gln Ser Gln Thr Pro Cys Val Gln Val
                   245                 250                 255
   tta gca ggc atg act ata gcc acc agc tac ctg ggc ata tca gca gtc      816
   Leu Ala Gly Met Thr Ile Ala Thr Ser Tyr Leu Gly Ile Ser Ala Val
               260                 265                 270
   tca gaa ttt ttt gga acc tcc ctc acc ccc tta ttt cat ttc cat atc      864
   Ser Glu Phe Phe Gly Thr Ser Leu Thr Pro Leu Phe His Phe His Ile
           275                 280                 285
   tct aca tgc ctt aaa act caa gga gcc ttt tat att tgt ggc cag tcg      912
   Ser Thr Cys Leu Lys Thr Gln Gly Ala Phe Tyr Ile Cys Gly Gln Ser
       290                 295                 300
   att cac caa tgc ctc ccc agt aac tgg act gga act tgt acc ata ggc      960
   Ile His Gln Cys Leu Pro Ser Asn Trp Thr Gly Thr Cys Thr Ile Gly
   305                 310                 315                 320
   tat gta acc cca gac atc ttc ata gcc cct ggc aat ctc tct ctt cca     1008
   Tyr Val Thr Pro Asp Ile Phe Ile Ala Pro Gly Asn Leu Ser Leu Pro
                   325                 330                 335
   ata cca atc tat ggg aat tcc ccg ttg ccc agg gtg agg agg gca atc     1056
   Ile Pro Ile Tyr Gly Asn Ser Pro Leu Pro Arg Val Arg Arg Ala Ile
               340                 345                 350
   cat ttc att ccc ctt ctc gcg gga ctc ggc att cta gct ggt acg gga     1104
   His Phe Ile Pro Leu Leu Ala Gly Leu Gly Ile Leu Ala Gly Thr Gly
           355                 360                 365
   acc gga att gct gga atc aca aaa gct tcc ctc acc tat agc cag ctc     1152
   Thr Gly Ile Ala Gly Ile Thr Lys Ala Ser Leu Thr Tyr Ser Gln Leu
       370                 375                 380
   tca aag gaa ata gcc aac aac att gac acc atg gct aaa gcc tta acg     1200
   Ser Lys Glu Ile Ala Asn Asn Ile Asp Thr Met Ala Lys Ala Leu Thr
   385                 390                 395                 400
   acc atg caa gaa caa atc gac tct tta gca gcc gta gtc ctt caa aat     1248
   Thr Met Gln Glu Gln Ile Asp Ser Leu Ala Ala Val Val Leu Gln Asn
                   405                 410                 415
   cgt cga gga cta gac atg tta acg gca gca cag gga gga att tgt ttg     1296
   Arg Arg Gly Leu Asp Met Leu Thr Ala Ala Gln Gly Gly Ile Cys Leu
                   420                     425                     430
   gcc tta gat gaa aaa tgt tgc ttt tgg gta aat caa tca gga aaa gta      1344
   Ala Leu Asp Glu Lys Cys Cys Phe Trp Val Asn Gln Ser Gly Lys Val
               435                     440                     445
   caa gac aac atc aga caa ctc cta aat caa gcc tcc agt tta cgg gaa      1392
   Gln Asp Asn Ile Arg Gln Leu Leu Asn Gln Ala Ser Ser Leu Arg Glu
           450                     455                     460
   cga gcc act cag ggt tgg tta aat tgg gaa gga act tgg aaa tgg ttc      1440
   Arg Ala Thr Gln Gly Trp Leu Asn Trp Glu Gly Thr Trp Lys Trp Phe
   465                     470                     475                 480
   tct tgg gtt ctt ccc ctt aca ggc cca ctt gtt agt ctc cta ctt ttg      1488
   Ser Trp Val Leu Pro Leu Thr Gly Pro Leu Val Ser Leu Leu Leu Leu
                   485                     490                     495
   ctc ctt ttt ggt cca tgt ctc cta aat cta ata acc caa ttt gtc tcc      1536
   Leu Leu Phe Gly Pro Cys Leu Leu Asn Leu Ile Thr Gln Phe Val Ser
               500                     505                     510
   tct cgc ctt cag gcc ata aag ctc cag acg aat ctc agt gca gga cgc      1584
   Ser Arg Leu Gln Ala Ile Lys Leu Gln Thr Asn Leu Ser Ala Gly Arg
           515                     520                     525
   cat cct cgc aat att caa gag tca ccc ttc taa                          1617
   His Pro Arg Asn Ile Gln Glu Ser Pro Phe
       530                     535
<210> 2
   <211> 538
   <212> PRT
   <213> Homo sapiens
<400> 2
   Met Gly Leu Leu Leu Leu Val Leu Ile Leu Thr Pro Ser Leu Ala Ala
   1               5                   10                  15
   Tyr Arg His Pro Asp Phe Pro Leu Leu Glu Lys Ala Gln Gln Leu Leu
               20                  25                  30
   Gln Ser Thr Gly Ser Pro Tyr Ser Thr Asn Cys Trp Leu Cys Thr Ser
           35                  40                  45
   Ser Ser Thr Glu Thr Pro Gly Thr Ala Tyr Pro Ala Ser Pro Arg Glu
       50                  55                  60
   Trp Thr Ser Ile Glu Ala Glu Leu His Ile Ser Tyr Arg Trp Asp Pro
   65                  70                  75                  80
   Asn Leu Lys Gly Leu Met Arg Pro Ala Asn Ser Leu Leu Ser Thr Val
                   85                  90                  95
   Lys Gln Asp Phe Pro Asp Ile Arg Gln Lys Pro Pro Ile Phe Gly Pro
               100                 105                 110
   Ile Phe Thr Asn Ile Asn Leu Met Gly Ile Ala Pro Ile Cys Val Met
   115 120 125
   Ala Lys Arg Lys Asn Gly Thr Asn Val Gly Thr Leu Pro Ser Thr Val
   130 135 140
   Cys Asn Val Thr Phe Thr Val Asp Ser Asn Gln Gln Thr Tyr Gln Thr
   145 150 155 160
   Tyr Thr His Asn Gln Phe Arg His Gln Pro Arg Phe Pro Lys Pro Pro
   165 170 175
   Asn Ile Thr Phe Pro Gln Gly Thr Leu Leu Asp Lys Ser Ser Arg Phe
   180 185 190
   Cys Gln Gly Arg Pro Ser Ser Cys Ser Thr Arg Asn Phe Trp Phe Arg
   195 200 205
   Pro Ala Asp Tyr Asn Gln Cys Leu Gln Ile Ser Asn Leu Ser Ser Thr
   210 215 220
   Ala Glu Trp Val Leu Leu Asp Gln Thr Arg Asn Ser Leu Phe Trp Glu
   225 230 235 240
   Asn Lys Thr Lys Gly Ala Asn Gln Ser Gln Thr Pro Cys Val Gln Val
   245 250 255
   Leu Ala Gly Met Thr Ile Ala Thr Ser Tyr Leu Gly Ile Ser Ala Val
   260 265 270
   Ser Glu Phe Phe Gly Thr Ser Leu Thr Pro Leu Phe His Phe His Ile
   275 280 285
   Ser Thr Cys Leu Lys Thr Gln Gly Ala Phe Tyr Ile Cys Gly Gln Ser
   290 295 300
   Ile His Gln Cys Leu Pro Ser Asn Trp Thr Gly Thr Cys Thr Ile Gly
   305 310 315 320
   Tyr Val Thr Pro Asp Ile Phe Ile Ala Pro Gly Asn Leu Ser Leu Pro
   325 330 335
   Ile Pro Ile Tyr Gly Asn Ser Pro Leu Pro Arg Val Arg Arg Ala Ile
   340 345 350
   His Phe Ile Pro Leu Leu Ala Gly Leu Gly Ile Leu Ala Gly Thr Gly
   355 360 365
   Thr Gly Ile Ala Gly Ile Thr Lys Ala Ser Leu Thr Tyr Ser Gln Leu
   370 375 380
   Ser Lys Glu Ile Ala Asn Asn Ile Asp Thr Met Ala Lys Ala Leu Thr
   385 390 395 400
   Thr Met Gln Glu Gln Ile Asp Ser Leu Ala Ala Val Val Leu Gln Asn
   405 410 415
   Arg Arg Gly Leu Asp Met Leu Thr Ala Ala Gln Gly Gly Ile Cys Leu
   420 425 430
   Ala Leu Asp Glu Lys Cys Cys Phe Trp Val Asn Gln Ser Gly Lys Val
   435 440 445
   Gln Asp Asn Ile Arg Gln Leu Leu Asn Gln Ala Ser Ser Leu Arg Glu
   450 455 460
   Arg Ala Thr Gln Gly Trp Leu Asn Trp Glu Gly Thr Trp Lys Trp Phe
   465 470 475 480
   Ser Trp Val Leu Pro Leu Thr Gly Pro Leu Val Ser Leu Leu Leu Leu
   485 490 495
   Leu Leu Phe Gly Pro Cys Leu Leu Asn Leu Ile Thr Gln Phe Val Ser
   500 505 510
   Ser Arg Leu Gln Ala Ile Lys Leu Gln Thr Asn Leu Ser Ala Gly Arg
   515 520 525
   His Pro Arg Asn Ile Gln Glu Ser Pro Phe
   530 535
<210> 3
   <211> 20
   <212> DNA
   <213> Artificial sequence
<220>
   <223> PCR primer
<400> 3
   gcctgcaaat agtcttcttt 20
<210> 4
   <211> 20
   <212> DNA
   <213> Artificial sequence
<220>
   <223> PCR primer
<400> 4
   ataggggcta ttcccattag 20
<210> 5
   <211> 55
   <212> DNA
   <213> Artificial sequence
<220>
   <223> PCR primer
<400> 5
   gctaatacga ctcactatag gaacagacca ccatgcacca cagtatcaac cttac 55
<210> 6
   <211> 23
   <212> DNA
   <213> Artificial sequence
<220>
   <223> PCR primer
<400> 6
   ttctgtttca gctacaactc tgt 23
<210> 7
   <211> 20
   <212> DNA
   <213> Artificial sequence
<220>
   <223> PCR primer
<400> 7
   agcaatagtt tgttaaattc 20
<210> 8
   <211> 55
   <212> DNA
   <213> Artificial sequence
<220>
   <223> PCR primer
<400> 8
   gctaatacga ctcactatag gaacagacca ccatgagggc accctccaat acttc 55
<210> 9
   <211> 20
   <212> DNA
   <213> Artificial sequence
<220>
   <223> PCR primer
<400> 9
   accccatgtt ctagtcttcc 20
<210> 10
   <211> 53
   <212> DNA
   <213> Artificial sequence
<220>
   <223> PCR primer
<400> 10
   gctaatacga ctcactatag gaacagacca ccatggagat gcaaagaaaa gca 53
<210> 11
   <211> 25
   <212> DNA
   <213> Artificial sequence
<220>
   <223> PCR primer
<400> 11
   gtgaacaaag gtctttgcat catag 25
<210> 12
   <211> 23
   <212> DNA
   <213> Artificial sequence
<220>
   <223> PCR primer
<400> 12
   gaattaggct ttcgggactt gaa 23
<210> 13
   <211> 18
   <212> DNA
   <213> Artificial sequence
<220>
   <223> PCR primer
<220>
   <221> misc feature
   <222> (1)..(1)
   <223> c or t
<220>
   <221> misc_feature
   <222> (7)..(7)
   <223> g or a
<220>
   <221> misc feature
   <222> (6)..(6)
   <223> c or g or a
<220>
   <221> misc feature
   <222> (17)..(17)
   <223> c or a
<400> 13
   nttaannagc atgctgnc 18
<210> 14
   <211> 53
   <212> DNA
   <213> Artificial sequence
<220>
   <223> PCR primer
<400> 14
   gctaatacga ctcactatag gaacagacca ccatgttgga ttcatcactc cca 53
<210> 15
   <211> 21
   <212> DNA
   <213> Artificial sequence
<220>
   <223> PCR primer
<400> 15
   ctgaagggag ttcctcctag g 21
<210> 16
   <211> 50
   <212> DNA
   <213> Artificial sequence
<220>
   <223> PCR primer
<400> 16
   gctaatacga ctcactatag gaacagacca ccatggccct cccttatcat 50
<210> 17
   <211> 18
   <212> DNA
   <213> Artificial sequence
<220>
   <223> PCR primer
<400> 17
   acagccaagc aggtacag 18
<210> 18
   <211> 54
   <212> DNA
   <213> Artificial sequence
<220>
   <223> PCR primer
<400> 18
   gctaatacga ctcactatag gaacagacca ccatgctcct gctggttctc attc 54
<210> 19
   <211> 21
   <212> DNA
   <213> Artificial sequence
<220>
   <223> PCR primer
<400> 19
   ctgcagcaga ctccatcctt g 21
<210> 20
   <211> 55
   <212> DNA
   <213> Artificial sequence
<220>
   <223> PCR primer
<400> 20
   gctaatacga ctcactatag gaacagacca ccatgactaa aaccctgttg tatca 55
<210> 21
   <211> 20
   <212> DNA
   <213> Artificial sequence
<220>
   <223> PCR primer
<400> 21
   gttaatactt agttagggcc 20
<210> 22
   <211> 55
   <212> DNA
   <213> Artificial sequence
<220>
   <223> PCR primer
<400> 22
   gctaatacga ctcactatag gaacagacca ccatggatcc actacacacg attga 55
<210> 23
   <211> 20
   <212> DNA
   <213> Artificial sequence
<220>
   <223> PCR primer
<400> 23
   tgttttggga caccacgaat 20
<210> 24
   <211> 50
   <212> DNA
   <213> Artificial sequence
<220>
   <223> PCR primer
<400> 24
   gctaatacga ctcactatag gaacagacca ccatgaattc tccatgtgac 50
<210> 25
   <211> 20
   <212> DNA
   <213> Artificial sequence
<220>
   <223> PCR primer
<400> 25
   gacacttaat agttgcgaca 20
<210> 26
   <211> 56
   <212> DNA
   <213> Artificial sequence
<220>
   <223> PCR primer
<400> 26
   gctaatacga ctcactatag gaacagacca ccatggccag accttcccca ctatgc 56
<210> 27
   <211> 21
   <212> DNA
   <213> Artificial sequence
<220>
   <223> PCR primer
<400> 27
   gccttggcaa ctaaaccatt c 21
<210> 28
   <211> 20
   <212> DNA
   <213> Artificial sequence
<220>
   <223> PCR primer
<400> 28
   ttcactccat ccttggctat 20
<210> 29
   <211> 21
   <212> DNA
   <213> Artificial sequence
<220>
   <223> PCR primer
<400> 29
   cgtcgagtat ctacgagcaa t 21
<210> 30
   <211> 23
   <212> DNA
   <213> Artificial sequence
<220>
   <223> PCR primer
<400> 30
   actacacaca tcactgaaac aaa 23
<210> 31
   <211> 20
   <212> DNA
   <213> Artificial sequence
<220>
   <223> PCR primer
<400> 31
   ggatggagtg aaatacagga 20
<210> 32
   <211> 20
   <212> DNA
   <213> Artificial sequence
<220>
   <223> PCR primer
<400> 32
   ccctcctcca catttatttg 20
<210> 33
   <211> 18
   <212> DNA
   <213> Artificial sequence
<220>
   <223> PCR primer
<400> 33
   gttgggcttt ggagatgg 18
<210> 34
   <211> 20
   <212> DNA
   <213> Artificial sequence
<220>
   <223> PCR primer
<400> 34
   cacaactaaa gaagctgacg 20
<210> 35
   <211> 20
   <212> DNA
   <213> Artificial sequence
<220>
   <223> PCR primer
<400> 35
   cataggccca gttggtatag 20
<210> 36
   <211> 18
   <212> DNA
   <213> Artificial sequence
<220>
   <223> PCR primer
<400> 36
   ccaggatttg atgttggg 18
<210> 37
   <211> 21
   <212> DNA
   <213> Artificial sequence
<220>
   <223> PCR primer
<400> 37
   ggggtgaggt taaggagatg g 21
<210> 38
   <211> 20
   <212> DNA
   <213> Artificial sequence
<220>
   <223> PCR primer
<400> 38
   ccccatcgta taggagtctt 20
<210> 39
   <211> 20
   <212> DNA
   <213> Artificial sequence
<220>
   <223> PCR primer
<400> 39
   ccccatcaga cataccagtt 20
<210> 40
   <211> 20
   <212> DNA
   <213> Artificial sequence
<220>
   <223> PCR primer
<400> 40
   ccatgggaag caagggaact 20
<210> 41
   <211> 20
   <212> DNA
   <213> Artificial sequence
<220>
   <223> PCR primer
<400> 41
   ctttccccag cgagcaatac 20
<210> 42
   <211> 20
   <212> DNA
   <213> Artificial sequence
<220>
   <223> PCR primer
<400> 42
   ggacagtgcc gacatactat 20
<210> 43
   <211> 20
   <212> DNA
   <213> Artificial sequence
<220>
   <223> PCR primer
<400> 43
   tagagtgcag catcctaacc 20
<210> 44
   <211> 22
   <212> DNA
   <213> Artificial sequence
<220>
   <223> PCR primer
<400> 44
   atggaggact atatgagcac aa 22
<210> 45
   <211> 22
   <212> DNA
   <213> Artificial sequence
<220>
   <223> PCR primer
<400> 45
   attaaagtta accacgagaa gc 22
<210> 46
   <211> 21
   <212> DNA
   <213> Artificial sequence
<220>
   <223> PCR primer
<400> 46
   gggccactaa gttactaggt c 21
<210> 47
   <211> 21
   <212> DNA
   <213> Artificial sequence
<220>
   <223> PCR primer
<400> 47
   agttaggagg gagttactgg g 21
<210> 48
   <211> 2097
   <212> DNA
   <213> Homo sapiens
<220>
   <221> CDS
   <222> (1)..(2097)
   <223>
<400> 48
   atg cac cca tcg gag atg caa aga aaa gca cct ccg cgg aga cgg aga        48
   Met His Pro Ser Glu Met Gln Arg Lys Ala Pro Pro Arg Arg Arg Arg
   1               5                   10                  15
   cac cgc aat cga gca ccg ttg act cac aag atg aac aaa atg gtg aca        96
   His Arg Asn Arg Ala Pro Leu Thr His Lys Met Asn Lys Met Val Thr
               20                  25                  30
   tca gaa cag atg aag ttg cca tcc acc aag aag gca gag ccg ccg act       144
   Ser Glu Gln Met Lys Leu Pro Ser Thr Lys Lys Ala Glu Pro Pro Thr
           35                  40                  45
   tgg gca caa cta aag aag ctg acg cag tta gct aca aaa tat cta gag       192
   Trp Ala Gln Leu Lys Lys Leu Thr Gln Leu Ala Thr Lys Tyr Leu Glu
       50                  55                  60
   aac aca aag gtg aca caa acc cca gag agt atg ctg ctt gca gcc ttg       240
   Asn Thr Lys Val Thr Gln Thr Pro Glu Ser Met Leu Leu Ala Ala Leu
   65                  70                  75                  80
   atg att gta tca atg gtg gta agt ctc cct atg cct gca gga gca gct       288
   Met Ile Val Ser Met Val Val Ser Leu Pro Met Pro Ala Gly Ala Ala
                   85                  90                  95
   gca gct aac tat acc aac tgg gcc tat gtg cct ttc ccg ccc tta att       336
   Ala Ala Asn Tyr Thr Asn Trp Ala Tyr Val Pro Phe Pro Pro Leu Ile
               100                 105                 110
   cgg gca gtc aca tgg atg gat aat cct ata gaa gta tat gtt aat gat       384
   Arg Ala Val Thr Trp Met Asp Asn Pro Ile Glu Val Tyr Val Asn Asp
           115                 120                 125
   agt gta tgg gta cat ggc ccc ata gat gat cgc tgc cct gcc aaa cct      432
   Ser Val Trp Val His Gly Pro Ile Asp Asp Arg Cys Pro Ala Lys Pro
       130                 135                 140
   gag gaa gaa ggg atg atg ata aat atc tcc att ggg tat cat tat cct      480
   Glu Glu Glu Gly Met Met Ile Asn Ile Ser Ile Gly Tyr His Tyr Pro
   145                 150                 155                 160
   cct att tgc cta ggg aga gca cca gga tgt tta atg cct gca gtc caa      528
   Pro Ile Cys Leu Gly Arg Ala Pro Gly Cys Leu Met Pro Ala Val Gln
                   165                 170                 175
   aat tgg ttg gta gaa gta cct act gtc agt ccc atc agt aga ttc act      576
   Asn Trp Leu Val Glu Val Pro Thr Val Ser Pro Ile Ser Arg Phe Thr
               180                 185                 190
   tat aac atg gta agc ggg atg tca ctc agg cca cgg gta aat tat tta      624
   Tyr Asn Met Val Ser Gly Met Ser Leu Arg Pro Arg Val Asn Tyr Leu
           195                 200                 205
   caa gac ttt tct tat caa aga tca tta aaa ttt aga cct aaa ggg aaa      672
   Gln Asp Phe Ser Tyr Gln Arg Ser Leu Lys Phe Arg Pro Lys Gly Lys
       210                 215                 220
   cct tgc ccc aag gaa att ccc aaa gaa tca aaa aat aca gaa gtt tta      720
   Pro Cys Pro Lys Glu Ile Pro Lys Glu Ser Lys Asn Thr Glu Val Leu
   225                 230                 235                 240
   gtt tgg gaa gaa tgt gtg gcc aat agt gtg gtg ata tta caa aac aat      768
   Val Trp Glu Glu Cys Val Ala Asn Ser Val Val Ile Leu Gln Asn Asn
                   245                 250                 255
   gaa ttt gga act att ata gat tgg gca cct cga ggt caa ttc tac cac      816
   Glu Phe Gly Thr Ile Ile Asp Trp Ala Pro Arg Gly Gln Phe Tyr His
               260                 265                 270
   aat tgc tca gga caa act cag tcg tgt cca agt gca caa gtg agt cca      864
   Asn Cys Ser Gly Gln Thr Gln Ser Cys Pro Ser Ala Gln Val Ser Pro
           275                 280                 285
   gct gtt gat agt gac tta aca gaa agt tta gac aaa cat aag cat aaa      912
   Ala Val Asp Ser Asp Leu Thr Glu Ser Leu Asp Lys His Lys His Lys
       290                 295                 300
   aaa ttg cag tct ttc tac cct tgg gaa tgg gga gaa aaa gga atc tct      960
   Lys Leu Gln Ser Phe Tyr Pro Trp Glu Trp Gly Glu Lys Gly Ile Ser
   305                 310                 315                 320
   acc cca aga cca aaa ata ata agt cct gtt tct ggt cct gaa cat cca     1008
   Thr Pro Arg Pro Lys Ile Ile Ser Pro Val Ser Gly Pro Glu His Pro
                   325                 330                 335
   gaa tta tgg agg ctt act gtg gcc tca cac cac att aga att tgg tct     1056
   Glu Leu Trp Arg Leu Thr Val Ala Ser His His Ile Arg Ile Trp Ser
               340                 345                 350
   gga aat caa act tta gaa aca aga gat cgt aag cca ttt tat act gtt     1104
   Gly Asn Gln Thr Leu Glu Thr Arg Asp Arg Lys Pro Phe Tyr Thr Val
           355                 360                 365
   gac cta aat tcc agt cta aca gtt cct tta caa agt tgc gta aag ccc     1152
   Asp Leu Asn Ser Ser Leu Thr Val Pro Leu Gln Ser Cys Val Lys Pro
       370                 375                 380
   cct tat atg cta gtt gta gga aat ata gtt att aaa cca gac tct caa      1200
   Pro Tyr Met Leu Val Val Gly Asn Ile Val Ile Lys Pro Asp Ser Gln
   385                 390                 395                 400
   act ata acc tgt gaa aat tgt aga ttg ctt act tgc att gat tca act      1248
   Thr Ile Thr Cys Glu Asn Cys Arg Leu Leu Thr Cys Ile Asp Ser Thr
                   405                 410                 415
   ttt aat tgg caa cac cgt att ctg ctg gtg aga gca aga gag ggc gtg      1296
   Phe Asn Trp Gln His Arg Ile Leu Leu Val Arg Ala Arg Glu Gly Val
               420                 425                 430
   tgg atc cct gtg tcc atg gac cga ccg tgg gag gcc tcg cca tcc atc      1344
   Trp Ile Pro Val Ser Met Asp Arg Pro Trp Glu Ala Ser Pro Ser Ile
           435                 440                 445
   cat att ttg act gaa gta tta aaa ggt gtt tta aat aga tcc aaa aga      1392
   His Ile Leu Thr Glu Val Leu Lys Gly Val Leu Asn Arg Ser Lys Arg
       450                 455                 460
   ttc att ttt act tta att gca gtg att atg gga tta att gca gtc aca      1440
   Phe Ile Phe Thr Leu Ile Ala Val Ile Met Gly Leu Ile Ala Val Thr
   465                 470                 475                 480
   gct atg gct gct gta gca gga gtt gca ttg cac tct ttt gtt cag tca      1488
   Ala Met Ala Ala Val Ala Gly Val Ala Leu His Ser Phe Val Gln Ser
                   485                 490                 495
   gta aac ttt gtt aat gat tgg caa aaa aat tct aca aga ttg tgg aat      1536
   Val Asn Phe Val Asn Asp Trp Gln Lys Asn Ser Thr Arg Leu Trp Asn
               500                 505                 510
   tca caa tct agt att gat caa aaa ttg gca aat caa att aat gat ctt      1584
   Ser Gln Ser Ser Ile Asp Gln Lys Leu Ala Asn Gln Ile Asn Asp Leu
           515                 520                 525
   aga caa act gtc att tgg atg gga gac agg ctc atg agc tta gaa cat      1632
   Arg Gln Thr Val Ile Trp Met Gly Asp Arg Leu Met Ser Leu Glu His
       530                 535                 540
   cgt ttc cag tta cag tgt gac tgg aat acg tca gat ttt tgt att aca      1680
   Arg Phe Gln Leu Gln Cys Asp Trp Asn Thr Ser Asp Phe Cys Ile Thr
   545                 550                 555                 560
   ccc caa att tat aat gag tct gag cat cac tgg gac atg gtt aga cgc      1728
   Pro Gln Ile Tyr Asn Glu Ser Glu His His Trp Asp Met Val Arg Arg
                   565                 570                 575
   cat cta cag gga aga gaa gat aat ctc act tta gac att tcc aaa tta      1776
   His Leu Gln Gly Arg Glu Asp Asn Leu Thr Leu Asp Ile Ser Lys Leu
               580                 585                 590
   aaa gaa caa att ttc gaa gca tca aaa gcc cat tta aat ttg gtg cca      1824
   Lys Glu Gln Ile Phe Glu Ala Ser Lys Ala His Leu Asn Leu Val Pro
           595                 600                 605
   gga act gag gca att gca gga gtt gct gat ggc ctc gca aat ctt aac      1872
   Gly Thr Glu Ala Ile Ala Gly Val Ala Asp Gly Leu Ala Asn Leu Asn
       610                 615                 620
   cct gtc act tgg gtt aag acc att gga agt act acg att ata aat ctc      1920
   Pro Val Thr Trp Val Lys Thr Ile Gly Ser Thr Thr Ile Ile Asn Leu
   625                 630                 635                 640
   ata tta atc ctt gtg tgc ctg ttt tgt cta ttg tta gtc tgc agg ttt      1968
   Ile Leu Ile Leu Val Cys Leu Phe Cys Leu Leu Leu Val Cys Arg Phe
                   645                     650                 655
   acc caa cag ctc cga aga gac agc tac cat cga gaa cgg gcc atg atg      2016
   Thr Gln Gln Leu Arg Arg Asp Ser Tyr His Arg Glu Arg Ala Met Met
               660                 665                     670
   acg atg gtg gtt ttg tcg aaa aga aaa ggg gga aat gtg ggg aaa agc      2064
   Thr Met Val Val Leu Ser Lys Arg Lys Gly Gly Asn Val Gly Lys Ser
           675                 680                     685
   aag aga gat cag att gtt act gtg tct gtg tag                          2097
   Lys Arg Asp Gln Ile Val Thr Val Ser Val
       690                     695
<210> 49
   <211> 698
   <212> PRT
   <213> Homo sapiens
<400> 49
   Met His Pro Ser Glu Met Gln Arg Lys Ala Pro Pro Arg Arg Arg Arg
   1               5                   10                  15
   His Arg Asn Arg Ala Pro Leu Thr His Lys Met Asn Lys Met Val Thr
               20                  25                  30
   Ser Glu Gln Met Lys Leu Pro Ser Thr Lys Lys Ala Glu Pro Pro Thr
           35                  40                  45
   Trp Ala Gln Leu Lys Lys Leu Thr Gln Leu Ala Thr Lys Tyr Leu Glu
       50                  55                  60
   Asn Thr Lys Val Thr Gln Thr Pro Glu Ser Met Leu Leu Ala Ala Leu
   65                  70                  75                  80
   Met Ile Val Ser Met Val Val Ser Leu Pro Met Pro Ala Gly Ala Ala
                   85                  90                  95
   Ala Ala Asn Tyr Thr Asn Trp Ala Tyr Val Pro Phe Pro Pro Leu Ile
               100                 105                 110
   Arg Ala Val Thr Trp Met Asp Asn Pro Ile Glu Val Tyr Val Asn Asp
           115                 120                 125
   Ser Val Trp Val His Gly Pro Ile Asp Asp Arg Cys Pro Ala Lys Pro
       130                 135                 140
   Glu Glu Glu Gly Met Met Ile Asn Ile Ser Ile Gly Tyr His Tyr Pro
   145                 150                 155                 160
   Pro Ile Cys Leu Gly Arg Ala Pro Gly Cys Leu Met Pro Ala Val Gln
                   165                 170                 175
   Asn Trp Leu Val Glu Val Pro Thr Val Ser Pro Ile Ser Arg Phe Thr
   180 185 190
   Tyr Asn Met Val Ser Gly Met Ser Leu Arg Pro Arg Val Asn Tyr Leu
   195 200 205
   Gln Asp Phe Ser Tyr Gln Arg Ser Leu Lys Phe Arg Pro Lys Gly Lys
   210 215 220
   Pro Cys Pro Lys Glu Ile Pro Lys Glu Ser Lys Asn Thr Glu Val Leu
   225 230 235 240
   Val Trp Glu Glu Cys Val Ala Asn Ser Val Val Ile Leu Gln Asn Asn
   245 250 255
   Glu Phe Gly Thr Ile Ile Asp Trp Ala Pro Arg Gly Gln Phe Tyr His
   260 265 270
   Asn Cys Ser Gly Gln Thr Gln Ser Cys Pro Ser Ala Gln Val Ser Pro
   275 280 285
   Ala Val Asp Ser Asp Leu Thr Glu Ser Leu Asp Lys His Lys His Lys
   290 295 300
   Lys Leu Gln Ser Phe Tyr Pro Trp Glu Trp Gly Glu Lys Gly Ile Ser
   305 310 315 320
   Thr Pro Arg Pro Lys Ile Ile Ser Pro Val Ser Gly Pro Glu His Pro
   325 330 335
   Glu Leu Trp Arg Leu Thr Val Ala Ser His His Ile Arg Ile Trp Ser
   340 345 350
   Gly Asn Gln Thr Leu Glu Thr Arg Asp Arg Lys Pro Phe Tyr Thr Val
   355 360 365
   Asp Leu Asn Ser Ser Leu Thr Val Pro Leu Gln Ser Cys Val Lys Pro
   370 375 380
   Pro Tyr Met Leu Val Val Gly Asn Ile Val Ile Lys Pro Asp Ser Gln
   385 390 395 400
   Thr Ile Thr Cys Glu Asn Cys Arg Leu Leu Thr Cys Ile Asp Ser Thr
   405 410 415
   Phe Asn Trp Gln His Arg Ile Leu Leu Val Arg Ala Arg Glu Gly Val
   420 425 430
   Trp Ile Pro Val Ser Met Asp Arg Pro Trp Glu Ala Ser Pro Ser Ile
           435                 440                 445
   His Ile Leu Thr Glu Val Leu Lys Gly Val Leu Asn Arg Ser Lys Arg
       450                 455                 460
   Phe Ile Phe Thr Leu Ile Ala Val Ile Met Gly Leu Ile Ala Val Thr
   465                 470                 475                 480
   Ala Met Ala Ala Val Ala Gly Val Ala Leu His Ser Phe Val Gln Ser
                   485                 490                 495
   Val Asn Phe Val Asn Asp Trp Gln Lys Asn Ser Thr Arg Leu Trp Asn
               500                 505                 510
   Ser Gln Ser Ser Ile Asp Gln Lys Leu Ala Asn Gln Ile Asn Asp Leu
           515                 520                 525
   Arg Gln Thr Val Ile Trp Met Gly Asp Arg Leu Met Ser Leu Glu His
       530                 535                 540
   Arg Phe Gln Leu Gln Cys Asp Trp Asn Thr Ser Asp Phe Cys Ile Thr
   545                 550                 555                 560
   Pro Gln Ile Tyr Asn Glu Ser Glu His His Trp Asp Met Val Arg Arg
                   565                 570                 575
   His Leu Gln Gly Arg Glu Asp Asn Leu Thr Leu Asp Ile Ser Lys Leu
               580                 585                 590
   Lys Glu Gln Ile Phe Glu Ala Ser Lys Ala His Leu Asn Leu Val Pro
           595                 600                 605
   Gly Thr Glu Ala Ile Ala Gly Val Ala Asp Gly Leu Ala Asn Leu Asn
       610                 615                 620
   Pro Val Thr Trp Val Lys Thr Ile Gly Ser Thr Thr Ile Ile Asn Leu
   625                 630                 635                 640
   Ile Leu Ile Leu Val Cys Leu Phe Cys Leu Leu Leu Val Cys Arg Phe
                   645                 650                 655
   Thr Gln Gln Leu Arg Arg Asp Ser Tyr His Arg Glu Arg Ala Met Met
               660                 665                 670
   Thr Met Val Val Leu Ser Lys Arg Lys Gly Gly Asn Val Gly Lys Ser
           675                 680                 685
   Lys Arg Asp Gln Ile Val Thr Val Ser Val
       690                 695
<210> 50
   <211> 1881
   <212> DNA
   <213> Homo sapiens
<220>
   <221> CDS
   <222> (1) .. (1881)
   <223>
<400> 50
   atg ggt ccc gaa gcc tgg gtc agg ccc ctt aaa act gcg cct aag ccg      48
   Met Gly Pro Glu Ala Trp Val Arg Pro Leu Lys Thr Ala Pro Lys Pro
   1               5                   10                  15
   ggt gaa gcc att aga tta att ctt ttt att tac ctc tct tgt ttc ttt      96
   Gly Glu Ala Ile Arg Leu Ile Leu Phe Ile Tyr Leu Ser Cys Phe Phe
               20                  25                  30
   ttg cct gtt atg tcc tct gag cct tcc tac tcc ttt ctc ctc acc tct      144
   Leu Pro Val Met Ser Ser Glu Pro Ser Tyr Ser Phe Leu Leu Thr Ser
           35                  40                  45
   ttc aca aca gga cgt gta ttc gca aac act act tgg agg gcc ggt acc      192
   Phe Thr Thr Gly Arg Val Phe Ala Asn Thr Thr Trp Arg Ala Gly Thr
       50                  55                  60
   tcc aag gaa gtc tcc ttt gca gtt gat tta tgt gta ctg ttc cca gag      240
   Ser Lys Glu Val Ser Phe Ala Val Asp Leu Cys Val Leu Phe Pro Glu
   65                  70                  75                  80
   cca gct cgt acc cat gaa gag caa cat aat ttg ccg gtc ata gga gca      288
   Pro Ala Arg Thr His Glu Glu Gln His Asn Leu Pro Val Ile Gly Ala
                   85                  90                  95
   gga agt gtc gac ctt gca gca gga ttt gga cac tct ggg agc caa act      336
   Gly Ser Val Asp Leu Ala Ala Gly Phe Gly His Ser Gly Ser Gln Thr
               100                 105                 110
   gga tgt gga agc tcc aaa ggt gca gaa aaa ggg ctc caa aat gtt gac      384
   Gly Cys Gly Ser Ser Lys Gly Ala Glu Lys Gly Leu Gln Asn Val Asp
           115                 120                 125
   ttt tac ctc tgt cct gga aat cac cct gac gct agc tgt aga gat act      432
   Phe Tyr Leu Cys Pro Gly Asn His Pro Asp Ala Ser Cys Arg Asp Thr
       130                 135                 140
   tac cag ttt ttc tgc cct gat tgg aca tgt gta act tta gcc acc tac      480
   Tyr Gln Phe Phe Cys Pro Asp Trp Thr Cys Val Thr Leu Ala Thr Tyr
   145                 150                 155                 160
   tct ggg gga tca act aga tct tca act ctt tcc ata agt cgt gtt cct      528
   Ser Gly Gly Ser Thr Arg Ser Ser Thr Leu Ser Ile Ser Arg Val Pro
                   165                 170                 175
   cat cct aaa tta tgt act aga aaa aat tgt aat cct ctt act ata act      576
   His Pro Lys Leu Cys Thr Arg Lys Asn Cys Asn Pro Leu Thr Ile Thr
               180                 185                 190
   gtc cat gac cct aat gca gct caa tgg tat tat ggc atg tca tgg gga      624
   Val His Asp Pro Asn Ala Ala Gln Trp Tyr Tyr Gly Met Ser Trp Gly
           195                 200                 205
   tta aga ctt tat atc cca gga ttt gat gtt ggg act atg ttc acc atc      672
   Leu Arg Leu Tyr Ile Pro Gly Phe Asp Val Gly Thr Met Phe Thr Ile
       210                 215                 220
   caa aag aaa atc ttg gtc tca tgg agc tcc ccc aag cca atc ggg cct      720
   Gln Lys Lys Ile Leu Val Ser Trp Ser Ser Pro Lys Pro Ile Gly Pro
   225                 230                 235                 240
   tta act gat cta ggt gac cct ata ttc cag aaa cac cct gac aaa gtt      768
   Leu Thr Asp Leu Gly Asp Pro Ile Phe Gln Lys His Pro Asp Lys Val
                   245                 250                 255
   gat tta act gtt cct ctg cca ttc tta gtt cct aga ccc cag cta caa      816
   Asp Leu Thr Val Pro Leu Pro Phe Leu Val Pro Arg Pro Gln Leu Gln
               260                 265                 270
   caa caa cat ctt caa ccc agc cta atg tct ata cta ggt gga gta cac      864
   Gln Gln His Leu Gln Pro Ser Leu Met Ser Ile Leu Gly Gly Val His
           275                 280                 285
   cat ctc ctt aac ctc acc cag cct aaa cta gcc caa gat tgt tgg cta      912
   His Leu Leu Asn Leu Thr Gln Pro Lys Leu Ala Gln Asp Cys Trp Leu
       290                 295                 300
   tgt tta aaa gca aaa ccc cct tat tat gta gga tta gga gta gaa gcc      960
   Cys Leu Lys Ala Lys Pro Pro Tyr Tyr Val Gly Leu Gly Val Glu Ala
   305                 310                 315                 320
   aca ctt aaa cgt ggc cct cta tct tgt cat aca cga ccc cgt gct ctc     1008
   Thr Leu Lys Arg Gly Pro Leu Ser Cys His Thr Arg Pro Arg Ala Leu
                   325                 330                 335
   aca ata gga gat gtg tct gga aat gct tcc tgt ctg att agt acc ggg     1056
   Thr Ile Gly Asp Val Ser Gly Asn Ala Ser Cys Leu Ile Ser Thr Gly
               340                 345                 350
   tat aac tta tct gct tct cct ttt cag gct act tgt aat cag tcc ctg     1104
   Tyr Asn Leu Ser Ala Ser Pro Phe Gln Ala Thr Cys Asn Gln Ser Leu
           355                 360                 365
   ctt act tcc ata agc acc tca gtc tct tac caa gca ccc aac aat acc     1152
   Leu Thr Ser Ile Ser Thr Ser Val Ser Tyr Gln Ala Pro Asn Asn Thr
       370                 375                 380
   tgg ttg gcc tgc acc tca ggt ctc act cgc tgc att aat gga act gaa     1200
   Trp Leu Ala Cys Thr Ser Gly Leu Thr Arg Cys Ile Asn Gly Thr Glu
   385                 390                 395                 400
   cca gga cct ctc ctg tgc gtg tta gtt cat gta ctt ccc cag gta tat     1248
   Pro Gly Pro Leu Leu Cys Val Leu Val His Val Leu Pro Gln Val Tyr
                   405                 410                 415
   gtg tac agt gga cca gaa gga cga caa ctc atc gct ccc cct gag tta     1296
   Val Tyr Ser Gly Pro Glu Gly Arg Gln Leu Ile Ala Pro Pro Glu Leu
               420                 425                 430
   cat ccc agg ttg cac caa gct gtc cca ctt ctg gtt ccc cta ttg gct     1344
   His Pro Arg Leu His Gln Ala Val Pro Leu Leu Val Pro Leu Leu Ala
           435                 440                 445
   ggt ctt agc ata gct gga tca gca gcc att ggt acg gct gcc ctg gtt     1392
   Gly Leu Ser Ile Ala Gly Ser Ala Ala Ile Gly Thr Ala Ala Leu Val
       450                 455                 460
   caa gga gaa act gga cta ata tcc ctg tct caa cag gtg gat gct gat      1440
   Gln Gly Glu Thr Gly Leu Ile Ser Leu Ser Gln Gln Val Asp Ala Asp
   465                 470                 475                 480
   ttt agt aac ctc cag tct gcc ata gat ata cta cat tcc cag gta gag      1488
   Phe Ser Asn Leu Gln Ser Ala Ile Asp Ile Leu His Ser Gln Val Glu
                   485                 490                 495
   tct ctg gct gaa gta gtt ctt caa aac tgc cga tgc tta gat ctg cta      1536
   Ser Leu Ala Glu Val Val Leu Gln Asn Cys Arg Cys Leu Asp Leu Leu
               500                 505                 510
   ttc ctc tct caa gga ggt tta tgt gca gct cta gga gaa agt tgt tgc      1584
   Phe Leu Ser Gln Gly Gly Leu Cys Ala Ala Leu Gly Glu Ser Cys Cys
           515                 520                 525
   ttc tat gcc aat caa tct gga gtc ata aaa ggt aca gta aaa aaa gtt      1632
   Phe Tyr Ala Asn Gln Ser Gly Val Ile Lys Gly Thr Val Lys Lys Val
       530                 535                 540
   cga gaa aat cta gat agg cac caa caa gaa cga gaa aat aac atc ccc      1680
   Arg Glu Asn Leu Asp Arg His Gln Gln Glu Arg Glu Asn Asn Ile Pro
   545                 550                 555                 560
   tgg tat caa agc atg ttt aac tgg aac cca tgg cta act act tta atc      1728
   Trp Tyr Gln Ser Met Phe Asn Trp Asn Pro Trp Leu Thr Thr Leu Ile
                   565                 570                 575
   act ggg tta gct gga cct ctc ctc atc cta cta tta agt tta att ttt      1776
   Thr Gly Leu Ala Gly Pro Leu Leu Ile Leu Leu Leu Ser Leu Ile Phe
               580                 585                 590
   ggg cct tgt ata tta aat tcg ttt ctt aat ttt ata aaa caa cgc ata      1824
   Gly Pro Cys Ile Leu Asn Ser Phe Leu Asn Phe Ile Lys Gln Arg Ile
           595                 600                 605
   gct tct gtc aaa ctt acg tat ctt aag act caa tat gac acc ctt gtt      1872
   Ala Ser Val Lys Leu Thr Tyr Leu Lys Thr Gln Tyr Asp Thr Leu Val
       610                 615                 620
   aat aac tga                                                          1881
   Asn Asn
   625
<210> 51
   <211> 626
   <212> PRT
   <213> Homo sapiens
<400> 51
   Met Gly Pro Glu Ala Trp Val Arg Pro Leu Lys Thr Ala Pro Lys Pro
   1               5                   10                  15
   Gly Glu Ala Ile Arg Leu Ile Leu Phe Ile Tyr Leu Ser Cys Phe Phe
               20                  25                  30
   Leu Pro Val Met Ser Ser Glu Pro Ser Tyr Ser Phe Leu Leu Thr Ser
           35                  40                  45
   Phe Thr Thr Gly Arg Val Phe Ala Asn Thr Thr Trp Arg Ala Gly Thr
   50 55 60
   Ser Lys Glu Val Ser Phe Ala Val Asp Leu Cys Val Leu Phe Pro Glu
   65 70 75 80
   Pro Ala Arg Thr His Glu Glu Gln His Asn Leu Pro Val Ile Gly Ala
   85 90 95
   Gly Ser Val Asp Leu Ala Ala Gly Phe Gly His Ser Gly Ser Gln Thr
   100 105 110
   Gly Cys Gly Ser Ser Lys Gly Ala Glu Lys Gly Leu Gln Asn Val Asp
   115 120 125
   Phe Tyr Leu Cys Pro Gly Asn His Pro Asp Ala Ser Cys Arg Asp Thr
   130 135 140
   Tyr Gln Phe Phe Cys Pro Asp Trp Thr Cys Val Thr Leu Ala Thr Tyr
   145 150 155 160
   Ser Gly Gly Ser Thr Arg Ser Ser Thr Leu Ser Ile Ser Arg Val Pro
   165 170 175
   His Pro Lys Leu Cys Thr Arg Lys Asn Cys Asn Pro Leu Thr Ile Thr
   180 185 190
   Val His Asp Pro Asn Ala Ala Gln Trp Tyr Tyr Gly Met Ser Trp Gly
   195 200 205
   Leu Arg Leu Tyr Ile Pro Gly Phe Asp Val Gly Thr Met Phe Thr Ile
   210 215 220
   Gln Lys Lys Ile Leu Val Ser Trp Ser Ser Pro Lys Pro Ile Gly Pro
   225 230 235 240
   Leu Thr Asp Leu Gly Asp Pro Ile Phe Gln Lys His Pro Asp Lys Val
   245 250 255
   Asp Leu Thr Val Pro Leu Pro Phe Leu Val Pro Arg Pro Gln Leu Gln
   260 265 270
   Gln Gln His Leu Gln Pro Ser Leu Met Ser Ile Leu Gly Gly Val His
   275 280 285
   His Leu Leu Asn Leu Thr Gln Pro Lys Leu Ala Gln Asp Cys Trp Leu
   290 295 300
   Cys Leu Lys Ala Lys Pro Pro Tyr Tyr Val Gly Leu Gly Val Glu Ala
   305 310 315 320
   Thr Leu Lys Arg Gly Pro Leu Ser Cys His Thr Arg Pro Arg Ala Leu
   325 330 335
   Thr Ile Gly Asp Val Ser Gly Asn Ala Ser Cys Leu Ile Ser Thr Gly
   340 345 350
   Tyr Asn Leu Ser Ala Ser Pro Phe Gln Ala Thr Cys Asn Gln Ser Leu
   355 360 365
   Leu Thr Ser Ile Ser Thr Ser Val Ser Tyr Gln Ala Pro Asn Asn Thr
   370 375 380
   Trp Leu Ala Cys Thr Ser Gly Leu Thr Arg Cys Ile Asn Gly Thr Glu
   385 390 395 400
   Pro Gly Pro Leu Leu Cys Val Leu Val His Val Leu Pro Gln Val Tyr
   405 410 415
   Val Tyr Ser Gly Pro Glu Gly Arg Gln Leu Ile Ala Pro Pro Glu Leu
   420 425 430
   His Pro Arg Leu His Gln Ala Val Pro Leu Leu Val Pro Leu Leu Ala
   435 440 445
   Gly Leu Ser Ile Ala Gly Ser Ala Ala Ile Gly Thr Ala Ala Leu Val
   450 455 460
   Gln Gly Glu Thr Gly Leu Ile Ser Leu Ser Gln Gln Val Asp Ala Asp
   465 470 475 480
   Phe Ser Asn Leu Gln Ser Ala Ile Asp Ile Leu His Ser Gln Val Glu
   485 490 495
   Ser Leu Ala Glu Val Val Leu Gln Asn Cys Arg Cys Leu Asp Leu Leu
   500 505 510
   Phe Leu Ser Gln Gly Gly Leu Cys Ala Ala Leu Gly Glu Ser Cys Cys
   515 520 525
   Phe Tyr Ala Asn Gln Ser Gly Val Ile Lys Gly Thr Val Lys Lys Val
   530 535 540
   Arg Glu Asn Leu Asp Arg His Gln Gln Glu Arg Glu Asn Asn Ile Pro
   545 550 555 560
   Trp Tyr Gln Ser Met Phe Asn Trp Asn Pro Trp Leu Thr Thr Leu Ile
   565 570 575
   Thr Gly Leu Ala Gly Pro Leu Leu Ile Leu Leu Leu Ser Leu Ile Phe
   580 585 590
   Gly Pro Cys Ile Leu Asn Ser Phe Leu Asn Phe Ile Lys Gln Arg Ile
   595 600 605
   Ala Ser Val Lys Leu Thr Tyr Leu Lys Thr Gln Tyr Asp Thr Leu Val
   610 615 620
   Asn Asn
   625
<210> 52
   <211> 1545
   <212> DNA
   <213> Homo sapiens
<220>
   <221> CDS
   <222> (1) .. (1545)
   <223>
<400> 52
   atg gat cca cta cac acg att gaa aaa gtt cct gca aga aga aac atc        48
   Met Asp Pro Leu His Thr Ile Glu Lys Val Pro Ala Arg Arg Asn Ile
   1               5                   10                  15
   cac gac aga gga cac caa ggc cac cga atg gga gat gga acc cct gga        96
   His Asp Arg Gly His Gln Gly His Arg Met Gly Asp Gly Thr Pro Gly
               20                  25                  30
   agg cct aag att tct gtt caa caa atg aca aga ttt tcc ctt ata ata       144
   Arg Pro Lys Ile Ser Val Gln Gln Met Thr Arg Phe Ser Leu Ile Ile
           35                  40                  45
   ttt ttc ctt tct gct cct ttt gtt gtt aat gcc tct acc tct aac gtt       192
   Phe Phe Leu Ser Ala Pro Phe Val Val Asn Ala Ser Thr Ser Asn Val
       50                  55                  60
   ttc cta caa tgg gca cac agt tat gca gat ggc tta caa caa gga gac       240
   Phe Leu Gln Trp Ala His Ser Tyr Ala Asp Gly Leu Gln Gln Gly Asp
   65                  70                  75                  80
   cct tgc tgg gtc tgt ggt tcg tta ccc gtc act aac acc atg gag cta       288
   Pro Cys Trp Val Cys Gly Ser Leu Pro Val Thr Asn Thr Met Glu Leu
                   85                  90                  95
   cct tgg tgg gtc tcc ccg cta caa ggg aaa gac tgg gtt ttt ttt caa       336
   Pro Trp Trp Val Ser Pro Leu Gln Gly Lys Asp Trp Val Phe Phe Gln
               100                 105                 110
   agc ttt ata ggg gat ctt aaa caa tgg aca ggg gca cag atg act ggg       384
   Ser Phe Ile Gly Asp Leu Lys Gln Trp Thr Gly Ala Gln Met Thr Gly
           115                 120                 125
   gta act aga aaa aac att tca gaa tgg cct ata aat aaa act tta aat       432
   Val Thr Arg Lys Asn Ile Ser Glu Trp Pro Ile Asn Lys Thr Leu Asn
       130                 135                 140
   gag cca ggg cat gat aaa cca ttc tca gta aat gag aca agg gat aaa      480
   Glu Pro Gly His Asp Lys Pro Phe Ser Val Asn Glu Thr Arg Asp Lys
   145                 150                 155                 160
   gta ata gcc ttt gcc atc ccc ttg ttg gat acc aag gtg ttt gtc cag      528
   Val Ile Ala Phe Ala Ile Pro Leu Leu Asp Thr Lys Val Phe Val Gln
                   165                 170                 175
   act tcc aga cct cag aac act caa tat aga aat ggg ttt ctc cag ata      576
   Thr Ser Arg Pro Gln Asn Thr Gln Tyr Arg Asn Gly Phe Leu Gln Ile
               180                 185                 190
   tgg gac ggg ttc att tgg ctg aca gcc act aag gga cac tta agc cag      624
   Trp Asp Gly Phe Ile Trp Leu Thr Ala Thr Lys Gly His Leu Ser Gln
           195                 200                 205
   ata gct ccc tta tgc tgg gag caa aga aat cac tcc ctt gat aac tgg      672
   Ile Ala Pro Leu Cys Trp Glu Gln Arg Asn His Ser Leu Asp Asn Trp
       210                 215                 220
   cca aac aca act cgt gtt atg gga tgg att cca cct gga cag tgc cga      720
   Pro Asn Thr Thr Arg Val Met Gly Trp Ile Pro Pro Gly Gln Cys Arg
   225                 230                 235                 240
   cat act ata ctg tta caa cag agg gac cta ttt gcc aca gac tgg tct      768
   His Thr Ile Leu Leu Gln Gln Arg Asp Leu Phe Ala Thr Asp Trp Ser
                   245                 250                 255
   cag caa cct ggc ttg aat tgg tat gct ccc aac gga acc cag tgg ctc      816
   Gln Gln Pro Gly Leu Asn Trp Tyr Ala Pro Asn Gly Thr Gln Trp Leu
               260                 265                 270
   tgc agc cca aac tta tgg cct tgg ctt ccc tca ggt tgg tta gga tgc      864
   Cys Ser Pro Asn Leu Trp Pro Trp Leu Pro Ser Gly Trp Leu Gly Cys
           275                 280                 285
   tgc act cta ggt att ccc tgg gca caa gga cgc tgg gta aaa acc atg      912
   Cys Thr Leu Gly Ile Pro Trp Ala Gln Gly Arg Trp Val Lys Thr Met
       290                 295                 300
   gaa gtc tat cct tat ctt cca cat gtg gtt aac caa ggg act agg gcc      960
   Glu Val Tyr Pro Tyr Leu Pro His Val Val Asn Gln Gly Thr Arg Ala
   305                 310                 315                 320
   att gtt cac agg aat gat cac cta ccc aca atc ttt atg ccc tca gta     1008
   Ile Val His Arg Asn Asp His Leu Pro Thr Ile Phe Met Pro Ser Val
                   325                 330                 335
   ggt tta gga act gta ata cag cac ata gag gct cta gcc aat ttt acc     1056
   Gly Leu Gly Thr Val Ile Gln His Ile Glu Ala Leu Ala Asn Phe Thr
               340                 345                 350
   caa cgg gcc cta aat gac agc ctc caa agt att tct ctc atg aat gct     1104
   Gln Arg Ala Leu Asn Asp Ser Leu Gln Ser Ile Ser Leu Met Asn Ala
           355                 360                 365
   gaa gtg tat tat atg cac gag gac atc tta caa aac cga atg gcc cta     1152
   Glu Val Tyr Tyr Met His Glu Asp Ile Leu Gln Asn Arg Met Ala Leu
       370                 375                 380
   gat att tta act gcg gct gaa gga gga acc tgt gcc ctc atc aaa act     1200
   Asp Ile Leu Thr Ala Ala Glu Gly Gly Thr Cys Ala Leu Ile Lys Thr
   385                 390                 395                 400
   gaa tgt tgt gtg tat att ccc aat aac tct aga aac att tcc ttg gcc     1248
   Glu Cys Cys Val Tyr Ile Pro Asn Asn Ser Arg Asn Ile Ser Leu Ala
                   405                 410                 415
   tta gag gat aca tgt cgg caa atc caa gtc atc tcc agc tct gca ctg      1296
   Leu Glu Asp Thr Cys Arg Gln Ile Gln Val Ile Ser Ser Ser Ala Leu
               420                 425                 430
   tca ctc cat gac tgg ata gca tct cag ttt agt gga aga cct tcc tgg      1344
   Ser Leu His Asp Trp Ile Ala Ser Gln Phe Ser Gly Arg Pro Ser Trp
           435                 440                 445
   tgg cag aaa atc ctc att gtc ctt gcc acc ctc tgg agc gta ggc ata      1392
   Trp Gln Lys Ile Leu Ile Val Leu Ala Thr Leu Trp Ser Val Gly Ile
       450                 455                 460
   gca ctg tgt tgt gga ctg tat ttt tgt cgc atg ttt tcc caa cac att      1440
   Ala Leu Cys Cys Gly Leu Tyr Phe Cys Arg Met Phe Ser Gln His Ile
   465                 470                 475                 480
   ccc caa act cat tcg att ata ttt caa cag gaa ctt ccc ttg agc ccc      1488
   Pro Gln Thr His Ser Ile Ile Phe Gln Gln Glu Leu Pro Leu Ser Pro
                   485                 490                 495
   cca agt cag gag cat tac cag agc caa aga gac atc ttc cac tct aac      1536
   Pro Ser Gln Glu His Tyr Gln Ser Gln Arg Asp Ile Phe His Ser Asn
               500                 505                 510
   gcc ccc tga                                                          1545
   Ala Pro
<210> 53
   <211> 514
   <212> PRT
   <213> Homo sapiens
<400> 53
   Met Asp Pro Leu His Thr Ile Glu Lys Val Pro Ala Arg Arg Asn Ile
   1               5                   10                  15
   His Asp Arg Gly His Gln Gly His Arg Met Gly Asp Gly Thr Pro Gly
               20                  25                  30
   Arg Pro Lys Ile Ser Val Gln Gln Met Thr Arg Phe Ser Leu Ile Ile
           35                  40                  45
   Phe Phe Leu Ser Ala Pro Phe Val Val Asn Ala Ser Thr Ser Asn Val
       50                  55                  60
   Phe Leu Gln Trp Ala His Ser Tyr Ala Asp Gly Leu Gln Gln Gly Asp
   65                  70                  75                  80
   Pro Cys Trp Val Cys Gly Ser Leu Pro Val Thr Asn Thr Met Glu Leu
                   85                  90                  95
   Pro Trp Trp Val Ser Pro Leu Gln Gly Lys Asp Trp Val Phe Phe Gln
               100                 105                 110
   Ser Phe Ile Gly Asp Leu Lys Gln Trp Thr Gly Ala Gln Met Thr Gly
   115 120 125
   Val Thr Arg Lys Asn Ile Ser Glu Trp Pro Ile Asn Lys Thr Leu Asn
   130 135 140
   Glu Pro Gly His Asp Lys Pro Phe Ser Val Asn Glu Thr Arg Asp Lys
   145 150 155 160
   Val Ile Ala Phe Ala Ile Pro Leu Leu Asp Thr Lys Val Phe Val Gln
   165 170 175
   Thr Ser Arg Pro Gln Asn Thr Gln Tyr Arg Asn Gly Phe Leu Gln Ile
   180 185 190
   Trp Asp Gly Phe Ile Trp Leu Thr Ala Thr Lys Gly His Leu Ser Gln
   195 200 205
   Ile Ala Pro Leu Cys Trp Glu Gln Arg Asn His Ser Leu Asp Asn Trp
   210 215 220
   Pro Asn Thr Thr Arg Val Met Gly Trp Ile Pro Pro Gly Gln Cys Arg
   225 230 235 240
   His Thr Ile Leu Leu Gln Gln Arg Asp Leu Phe Ala Thr Asp Trp Ser
   245 250 255
   Gln Gln Pro Gly Leu Asn Trp Tyr Ala Pro Asn Gly Thr Gln Trp Leu
   260 265 270
   Cys Ser Pro Asn Leu Trp Pro Trp Leu Pro Ser Gly Trp Leu Gly Cys
   275 280 285
   Cys Thr Leu Gly Ile Pro Trp Ala Gln Gly Arg Trp Val Lys Thr Met
   290 295 300
   Glu Val Tyr Pro Tyr Leu Pro His Val Val Asn Gln Gly Thr Arg Ala
   305 310 315 320
   Ile Val His Arg Asn Asp His Leu Pro Thr Ile Phe Met Pro Ser Val
   325 330 335
   Gly Leu Gly Thr Val Ile Gln His Ile Glu Ala Leu Ala Asn Phe Thr
   340 345 350
   Gln Arg Ala Leu Asn Asp Ser Leu Gln Ser Ile Ser Leu Met Asn Ala
   355 360 365
   Glu Val Tyr Tyr Met His Glu Asp Ile Leu Gln Asn Arg Met Ala Leu
   370 375 380
   Asp Ile Leu Thr Ala Ala Glu Gly Gly Thr Cys Ala Leu Ile Lys Thr
   385 390 395 400
   Glu Cys Cys Val Tyr Ile Pro Asn Asn Ser Arg Asn Ile Ser Leu Ala
   405 410 415
   Leu Glu Asp Thr Cys Arg Gln Ile Gln Val Ile Ser Ser Ser Ala Leu
   420 425 430
   Ser Leu His Asp Trp Ile Ala Ser Gln Phe Ser Gly Arg Pro Ser Trp
   435 440 445
   Trp Gln Lys Ile Leu Ile Val Leu Ala Thr Leu Trp Ser Val Gly Ile
   450 455 460
   Ala Leu Cys Cys Gly Leu Tyr Phe Cys Arg Met Phe Ser Gln His Ile
   465 470 475 480
   Pro Gln Thr His Ser Ile Ile Phe Gln Gln Glu Leu Pro Leu Ser Pro
   485 490 495
   Pro Ser Gln Glu His Tyr Gln Ser Gln Arg Asp Ile Phe His Ser Asn
   500 505 510
   Ala Pro
<210> 54
   <211> 1770
   <212> DNA
   <213> Homo sapiens
<220>
   <221> CDS
   <222> (1) .. (1581)
   <223>
<400> 54
   atg aat tct cca tgt gac agg ctc caa caa ttt att cag gtt ctt ctc       48
   Met Asn Ser Pro Cys Asp Arg Leu Gln Gln Phe Ile Gln Val Leu Leu
   1               5                   10                  15
   gag gaa agc tgg tca ttc cct agt ttt gct aac acc ctt cac tgg cct       96
   Glu Glu Ser Trp Ser Phe Pro Ser Phe Ala Asn Thr Leu His Trp Pro
               20                  25                  30
   gaa aat ctg ttg tcc tat ata gac gaa ctg gta tgg caa ggc tcc ctc      144
   Glu Asn Leu Leu Ser Tyr Ile Asp Glu Leu Val Trp Gln Gly Ser Leu
           35                  40                  45
   cag aac ttt cac caa cat gaa gtt cgc ttt gac aag ccc cct ctc aga      192
   Gln Asn Phe His Gln His Glu Val Arg Phe Asp Lys Pro Pro Leu Arg
       50                  55                  60
   ctc cct ctc act ggg ttt tct tcc ctc act gag aat tgg agt tcc aga      240
   Leu Pro Leu Thr Gly Phe Ser Ser Leu Thr Glu Asn Trp Ser Ser Arg
   65                  70                  75                  80
   cag gca gtg tcc tct aga cta gta gcc acg gca gca tcc ccg cca gca      288
   Gln Ala Val Ser Ser Arg Leu Val Ala Thr Ala Ala Ser Pro Pro Ala
                   85                  90                  95
   ggg tgt cag gca ccc ata gct ttc cta ggt cta aaa ttc tct tcc cta      336
   Gly Cys Gln Ala Pro Ile Ala Phe Leu Gly Leu Lys Phe Ser Ser Leu
               100                 105                 110
   ggc ccg gct aga aaa aac cct gca ctt tgc ttc ctc tat gat caa agt      384
   Gly Pro Ala Arg Lys Asn Pro Ala Leu Cys Phe Leu Tyr Asp Gln Ser
           115                 120                 125
   aac tcc aaa tgc aat acc agc tgg gtc aaa gaa aat gta ggc tgt ccg      432
   Asn Ser Lys Cys Asn Thr Ser Trp Val Lys Glu Asn Val Gly Cys Pro
       130                 135                 140
   tgg cac tgg tgc aat atc cat gag gca tta att cgt act gaa aaa gga      480
   Trp His Trp Cys Asn Ile His Glu Ala Leu Ile Arg Thr Glu Lys Gly
   145                 150                 155                 160
   tct gac cca atg ttc tat gtc aat acc tcc act gga gga cgg gac ggc      528
   Ser Asp Pro Met Phe Tyr Val Asn Thr Ser Thr Gly Gly Arg Asp Gly
                   165                 170                 175
   ttt aac gga ttt aac ctc caa att tct gac cct tgg gac ccc cgc tgg      576
   Phe Asn Gly Phe Asn Leu Gln Ile Ser Asp Pro Trp Asp Pro Arg Trp
               180                 185                 190
   gcc tcc ggt gta gat gga gga cta tat gag cac aaa act ttt atg tat      624
   Ala Ser Gly Val Asp Gly Gly Leu Tyr Glu His Lys Thr Phe Met Tyr
           195                 200                 205
   cca gta gct aag atc cgc att gcc agg acc ctt aaa acc act gtc aca      672
   Pro Val Ala Lys Ile Arg Ile Ala Arg Thr Leu Lys Thr Thr Val Thr
       210                 215                 220
   ggg tta tcc gac tta gcc tcc tca atc cag tca gcc gag aaa gag ctt      720
   Gly Leu Ser Asp Leu Ala Ser Ser Ile Gln Ser Ala Glu Lys Glu Leu
   225                 230                 235                 240
   acc agc cag ctt caa ccg gca gct gac cag gcc aag tcc tcc cgc ttc      768
   Thr Ser Gln Leu Gln Pro Ala Ala Asp Gln Ala Lys Ser Ser Arg Phe
                   245                 250                 255
   tcg tgg tta act tta atc tca gaa ggt gca caa ttg ctc caa tcc aca      816
   Ser Trp Leu Thr Leu Ile Ser Glu Gly Ala Gln Leu Leu Gln Ser Thr
               260                 265                 270
   ggg gta caa aac ctc tcc cac tgc ttc ctc tgt gca gcc ctc aga aga      864
   Gly Val Gln Asn Leu Ser His Cys Phe Leu Cys Ala Ala Leu Arg Arg
           275                 280                 285
   cct cca tta gta gca gtt cct ctc cct acc ccc ttt aat tat act ata      912
   Pro Pro Leu Val Ala Val Pro Leu Pro Thr Pro Phe Asn Tyr Thr Ile
       290                 295                 300
   aat tca tca acc cct ata cca ccg gtc cca aaa gga cag gtc cca cta      960
   Asn Ser Ser Thr Pro Ile Pro Pro Val Pro Lys Gly Gln Val Pro Leu
   305                 310                 315                 320
   ttc tca gac cct ata aga cat aag ttc cca ttc tgt tac tct acc cca      1008
   Phe Ser Asp Pro Ile Arg His Lys Phe Pro Phe Cys Tyr Ser Thr Pro
                   325                 330                 335
   aat gcc tct tgg tgt aac cag act agg atg ctt acc agc acc ccg gca      1056
   Asn Ala Ser Trp Cys Asn Gln Thr Arg Met Leu Thr Ser Thr Pro Ala
               340                 345                 350
   ccg ccc aga ggc tac ttc tgg tgt aac tcc acg cta act aaa gtt ctt      1104
   Pro Pro Arg Gly Tyr Phe Trp Cys Asn Ser Thr Leu Thr Lys Val Leu
           355                 360                 365
   aac tca act ggt aat cac acc ttg tgc tta ccc atc tct ctc atc cct      1152
   Asn Ser Thr Gly Asn His Thr Leu Cys Leu Pro Ile Ser Leu Ile Pro
       370                 375                 380
   ggc ctg acc cta tat agt cag gat gaa ctt agc cat ctg cta gcc tgg      1200
   Gly Leu Thr Leu Tyr Ser Gln Asp Glu Leu Ser His Leu Leu Ala Trp
   385                 390                 395                 400
   acc gag cca agg cca caa aat aaa agc aaa tgg gct att ttc tta ccc      1248
   Thr Glu Pro Arg Pro Gln Asn Lys Ser Lys Trp Ala Ile Phe Leu Pro
                   405                 410                 415
   tta gta cta ggc atc tcc tta gcc tcc tcc tta gtg gca tca ggg cta      1296
   Leu Val Leu Gly Ile Ser Leu Ala Ser Ser Leu Val Ala Ser Gly Leu
               420                 425                 430
   gga aaa gga gcc ctc acc cac tca atc caa aca tct caa gat ctg tct      1344
   Gly Lys Gly Ala Leu Thr His Ser Ile Gln Thr Ser Gln Asp Leu Ser
           435                 440                 445
   act cac ctg cag ttg gcc att gag gca tcg gcc gag tcc ctg gac tcc      1392
   Thr His Leu Gln Leu Ala Ile Glu Ala Ser Ala Glu Ser Leu Asp Ser
       450                 455                 460
   cta cag cga cag atc act aca gta gca cag gtc gca gca cag aac agg      1440
   Leu Gln Arg Gln Ile Thr Thr Val Ala Gln Val Ala Ala Gln Asn Arg
   465                 470                 475                 480
   cag gcc tta gac ttg ctt atg gca gaa aaa gga agg aca tgt tta ttc      1488
   Gln Ala Leu Asp Leu Leu Met Ala Glu Lys Gly Arg Thr Cys Leu Phe
                   485                 490                 495
   tta cag gaa gaa tgc tgc tat tat ctc aat gag tca ggg gta gtt gag      1536
   Leu Gln Glu Glu Cys Cys Tyr Tyr Leu Asn Glu Ser Gly Val Val Glu
               500                 505                 510
   aac agt tta caa act tta aaa aaa aaa aaa agc tcc aag agg agt          1581
   Asn Ser Leu Gln Thr Leu Lys Lys Lys Lys Ser Ser Lys Arg Ser
           515                 520                 525
   taaaacactc ctacgaccct ccaccctggt ccttcttggt ggttttcaac cgtggctcaa    1641
   cagatgctcc ctttccttat cccaattata attctctgta taataatgtg ttttgcccca    1701
   atcctagcac agtttctttg ccagcggata caagaaatca ccagggttct tcaagtagaa    1761
   gaaaaataa                                                            1770
<210> 55
   <211> 527
   <212> PRT
   <213> Homo sapiens
<400> 55
   Met Asn Ser Pro Cys Asp Arg Leu Gln Gln Phe Ile Gln Val Leu Leu
   1               5                   10                  15
   Glu Glu Ser Trp Ser Phe Pro Ser Phe Ala Asn Thr Leu His Trp Pro
               20                  25                  30
   Glu Asn Leu Leu Ser Tyr Ile Asp Glu Leu Val Trp Gln Gly Ser Leu
           35                  40                  45
   Gln Asn Phe His Gln His Glu Val Arg Phe Asp Lys Pro Pro Leu Arg
       50                  55                  60
   Leu Pro Leu Thr Gly Phe Ser Ser Leu Thr Glu Asn Trp Ser Ser Arg
   65                  70                  75                  80
   Gln Ala Val Ser Ser Arg Leu Val Ala Thr Ala Ala Ser Pro Pro Ala
                   85                  90                  95
   Gly Cys Gln Ala Pro Ile Ala Phe Leu Gly Leu Lys Phe Ser Ser Leu
               100                 105                 110
   Gly Pro Ala Arg Lys Asn Pro Ala Leu Cys Phe Leu Tyr Asp Gln Ser
           115                 120                 125
   Asn Ser Lys Cys Asn Thr Ser Trp Val Lys Glu Asn Val Gly Cys Pro
       130                 135                 140
   Trp His Trp Cys Asn Ile His Glu Ala Leu Ile Arg Thr Glu Lys Gly
   145                 150                 155                 160
   Ser Asp Pro Met Phe Tyr Val Asn Thr Ser Thr Gly Gly Arg Asp Gly
                   165                 170                 175
   Phe Asn Gly Phe Asn Leu Gln Ile Ser Asp Pro Trp Asp Pro Arg Trp
               180                 185                 190
   Ala Ser Gly Val Asp Gly Gly Leu Tyr Glu His Lys Thr Phe Met Tyr
           195                 200                 205
   Pro Val Ala Lys Ile Arg Ile Ala Arg Thr Leu Lys Thr Thr Val Thr
       210                 215                 220
   Gly Leu Ser Asp Leu Ala Ser Ser Ile Gln Ser Ala Glu Lys Glu Leu
   225                 230                 235                 240
   Thr Ser Gln Leu Gln Pro Ala Ala Asp Gln Ala Lys Ser Ser Arg Phe
   245 250 255
   Ser Trp Leu Thr Leu Ile Ser Glu Gly Ala Gln Leu Leu Gln Ser Thr
   260 265 270
   Gly Val Gln Asn Leu Ser His Cys Phe Leu Cys Ala Ala Leu Arg Arg
   275 280 285
   Pro Pro Leu Val Ala Val Pro Leu Pro Thr Pro Phe Asn Tyr Thr Ile
   290 295 300
   Asn Ser Ser Thr Pro Ile Pro Pro Val Pro Lys Gly Gln Val Pro Leu
   305 310 315 320
   Phe Ser Asp Pro Ile Arg His Lys Phe Pro Phe Cys Tyr Ser Thr Pro
   325 330 335
   Asn Ala Ser Trp Cys Asn Gln Thr Arg Met Leu Thr Ser Thr Pro Ala
   340 345 350
   Pro Pro Arg Gly Tyr Phe Trp Cys Asn Ser Thr Leu Thr Lys Val Leu
   355 360 365
   Asn Ser Thr Gly Asn His Thr Leu Cys Leu Pro Ile Ser Leu Ile Pro
   370 375 380
   Gly Leu Thr Leu Tyr Ser Gln Asp Glu Leu Ser His Leu Leu Ala Trp
   385 390 395 400
   Thr Glu Pro Arg Pro Gln Asn Lys Ser Lys Trp Ala Ile Phe Leu Pro
   405 410 415
   Leu Val Leu Gly Ile Ser Leu Ala Ser Ser Leu Val Ala Ser Gly Leu
   420 425 430
   Gly Lys Gly Ala Leu Thr His Ser Ile Gln Thr Ser Gln Asp Leu Ser
   435 440 445
   Thr His Leu Gln Leu Ala Ile Glu Ala Ser Ala Glu Ser Leu Asp Ser
   450 455 460
   Leu Gln Arg Gln Ile Thr Thr Val Ala Gln Val Ala Ala Gln Asn Arg
   465 470 475 480
   Gln Ala Leu Asp Leu Leu Met Ala Glu Lys Gly Arg Thr Cys Leu Phe
   485 490 495
   Leu Gln Glu Glu Cys Cys Tyr Tyr Leu Asn Glu Ser Gly Val Val Glu
   500 505 510
   Asn Ser Leu Gln Thr Leu Lys Lys Lys Lys Ser Ser Lys Arg Ser
   515 520 525
<210> 56
   <211> 1755
   <212> DNA
   <213> Homo sapiens
<220>
   <221> CDS
   <222> (1) .. (1755)
   <223>
<400> 56
   atg gcc aga cct tcc cca cta tgc ctc cta ctc ctc ctg acc ctc cta      48
   Met Ala Arg Pro Ser Pro Leu Cys Leu Leu Leu Leu Leu Thr Leu Leu
   1               5                   10                  15
   acc ccc ata gtg ccc agt aac tcc ctc cta act gaa ccc ccg ttc cga      96
   Thr Pro Ile Val Pro Ser Asn Ser Leu Leu Thr Glu Pro Pro Phe Arg
               20                  25                  30
   tgg agg ttc tac ctg cat gag act tgg acc caa ggc aac cgg ctc tcc      144
   Trp Arg Phe Tyr Leu His Glu Thr Trp Thr Gln Gly Asn Arg Leu Ser
           35                  40                  45
   act gtc aca ctg gca acg gtg gac tgc caa cct cac ggt tgt cag gcc      192
   Thr Val Thr Leu Ala Thr Val Asp Cys Gln Pro His Gly Cys Gln Ala
       50                  55                  60
   caa gta act ttt aac ttc act tcc ttt aaa agt gtt ctg cgg ggc tgg      240
   Gln Val Thr Phe Asn Phe Thr Ser Phe Lys Ser Val Leu Arg Gly Trp
   65                  70                  75                  80
   tcc aat ccc acc atc tgc ttt gtc tat gat caa aca cac agc aac tgc      288
   Ser Asn Pro Thr Ile Cys Phe Val Tyr Asp Gln Thr His Ser Asn Cys
                   85                  90                  95
   cgc gac tat tgg gtg gac aca aac gga gga tgc ccc tat gcc tat tgt      336
   Arg Asp Tyr Trp Val Asp Thr Asn Gly Gly Cys Pro Tyr Ala Tyr Cys
               100                 105                 110
   cgt atg cat gtg acc cag ctc cat acc gcc aag aaa ctc caa cac acc      384
   Arg Met His Val Thr Gln Leu His Thr Ala Lys Lys Leu Gln His Thr
           115                 120                 125
   tat cgc ctg aca tct gat gga agg aca act tac ttc ctg acc atc cca      432
   Tyr Arg Leu Thr Ser Asp Gly Arg Thr Thr Tyr Phe Leu Thr Ile Pro
       130                 135                 140
   gac cca tgg gat tct cgg tgg gtc agt gga gtc act ggt cga ctg tac      480
   Asp Pro Trp Asp Ser Arg Trp Val Ser Gly Val Thr Gly Arg Leu Tyr
   145                 150                 155                 160
   cgg tgg ccc acc gac tcc tac cca gtt ggc aaa ctc cgg ata ttc ctg      528
   Arg Trp Pro Thr Asp Ser Tyr Pro Val Gly Lys Leu Arg Ile Phe Leu
                   165                 170                 175
   act tat ata cga gtt atc ccc cag gtt ttg tct aat tta aag gac caa      576
   Thr Tyr Ile Arg Val Ile Pro Gln Val Leu Ser Asn Leu Lys Asp Gln
               180                 185                 190
   gca gac aac att aag cat cag gaa gag gtc atc aat act ttg gtg cag     624
   Ala Asp Asn Ile Lys His Gln Glu Glu Val Ile Asn Thr Leu Val Gln
           195                 200                 205
   tcc cat ccg aag gct gac atg gtc acc tat gat gac aag gct gag gca     672
   Ser His Pro Lys Ala Asp Met Val Thr Tyr Asp Asp Lys Ala Glu Ala
       210                 215                 220
   gga ccg ttt tca tgg ata acc cta gtc cgc cac ggg gct cgc ctt gtt     720
   Gly Pro Phe Ser Trp Ile Thr Leu Val Arg His Gly Ala Arg Leu Val
   225                 230                 235                 240
   aat atg gca ggc cta gtt aat ctc tcc cac tgt ttc ctt tgc acc gcc     768
   Asn Met Ala Gly Leu Val Asn Leu Ser His Cys Phe Leu Cys Thr Ala
                   245                 250                 255
   ctc agc caa cca cca cta gta gct gta ccc cta ccc cag gct ttt aac     816
   Leu Ser Gln Pro Pro Leu Val Ala Val Pro Leu Pro Gln Ala Phe Asn
               260                 265                 270
   acc tct ggt aac cac act gcc cac cct tcc ggc gtc ttc tct gag cag     864
   Thr Ser Gly Asn His Thr Ala His Pro Ser Gly Val Phe Ser Glu Gln
           275                 280                 285
   gtc cct ctt ttc cga gac ccc ctc cag ccc cag ttc ccc ttc tgc tac     912
   Val Pro Leu Phe Arg Asp Pro Leu Gln Pro Gln Phe Pro Phe Cys Tyr
       290                 295                 300
   acc act cct aac tca tcc tgg tgc aac cag acc tat tct ggc tcc cta     960
   Thr Thr Pro Asn Ser Ser Trp Cys Asn Gln Thr Tyr Ser Gly Ser Leu
   305                 310                 315                 320
   tct aac ctc tct gca ccg gca ggt ggc tac ttc tgg tgt aac ttc acc    1008
   Ser Asn Leu Ser Ala Pro Ala Gly Gly Tyr Phe Trp Cys Asn Phe Thr
                   325                 330                 335
   ctt aca aaa cat ctt aat att tcc tct aac aat acc ctt tct aga aac    1056
   Leu Thr Lys His Leu Asn Ile Ser Ser Asn Asn Thr Leu Ser Arg Asn
               340                 345                 350
   tta tgc ctc ccc atc tct ctg gtg cct cga ctc act ctg tac agc gag    1104
   Leu Cys Leu Pro Ile Ser Leu Val Pro Arg Leu Thr Leu Tyr Ser Glu
           355                 360                 365
   gct gaa ctc tct tcc ctt gtc aac ccg cct atg cgt cag aag cgg gcc    1152
   Ala Glu Leu Ser Ser Leu Val Asn Pro Pro Met Arg Gln Lys Arg Ala
       370                 375                 380
   gtt ttc cca ccg ctg gta ata ggt gtc tcc ttg acc tcc tca ctt gtt    1200
   Val Phe Pro Pro Leu Val Ile Gly Val Ser Leu Thr Ser Ser Leu Val
   385                 390                 395                 400
   gcc tcc ggg ctg ggc aca ggt gct att gta cat ttc ata agc tct tcc    1248
   Ala Ser Gly Leu Gly Thr Gly Ala Ile Val His Phe Ile Ser Ser Ser
                   405                 410                 415
   caa gat ctc tct att aag ctc cag atg gcc atc gaa gcc tca gcc gaa    1296
   Gln Asp Leu Ser Ile Lys Leu Gln Met Ala Ile Glu Ala Ser Ala Glu
               420                 425                 430
   tcc tta gcc tct cta cag aga cag att acg tct gtg gcc aag gtg gcc    1344
   Ser Leu Ala Ser Leu Gln Arg Gln Ile Thr Ser Val Ala Lys Val Ala
           435                 440                 445
   atg cag aac cgg aga gcc cta gat ctc ctc aca gcc gac aaa ggc gga      1392
   Met Gln Asn Arg Arg Ala Leu Asp Leu Leu Thr Ala Asp Lys Gly Gly
       450                 455                 460
   acc tgc atg ttt ctc ggg gaa gag tgc tgt tat tac atc aat gaa tca      1440
   Thr Cys Met Phe Leu Gly Glu Glu Cys Cys Tyr Tyr Ile Asn Glu Ser
   465                 470                 475                 480
   ggc tta gta gaa acc agc ctc ctc acc ctt gat aaa atc cgg gac ggt      1488
   Gly Leu Val Glu Thr Ser Leu Leu Thr Leu Asp Lys Ile Arg Asp Gly
                   485                 490                 495
   ctc cat cga ccc tcc tca act ccc aac tat gga gga ggg tgg tgg caa      1536
   Leu His Arg Pro Ser Ser Thr Pro Asn Tyr Gly Gly Gly Trp Trp Gln
               500                 505                 510
   tcc cct tta acc act tgg att atc cct ttc ata agc ccc atc cta atc      1584
   Ser Pro Leu Thr Thr Trp Ile Ile Pro Phe Ile Ser Pro Ile Leu Ile
           515                 520                 525
   att tgc ctt tta ctt ctc ata gcc ccc tgt gtc ctc aag ttc atc aaa      1632
   Ile Cys Leu Leu Leu Leu Ile Ala Pro Cys Val Leu Lys Phe Ile Lys
       530                 535                 540
   aac cgc atc agc gaa gtc tcc cgg gtg acg gtc aac caa atg tta cta      1680
   Asn Arg Ile Ser Glu Val Ser Arg Val Thr Val Asn Gln Met Leu Leu
   545                 550                 555                 560
   cac cct tac tcc cgt ctt ccg acc tcc gaa gac cac tat gac gac gcc      1728
   His Pro Tyr Ser Arg Leu Pro Thr Ser Glu Asp His Tyr Asp Asp Ala
                   565                 570                 575
   ctc act cag cag gaa gca gcc aga tga                                  1755
   Leu Thr Gln Gln Glu Ala Ala Arg
               580
<210> 57
   <211> 584
   <212> PRT
   <213> Homo sapiens
<400> 57
<210> 58
   <211> 43
   <212> DNA
   <213> Artificial sequence
<220>
   <223> PCR primer
<400> 58
   atcacctcga gcaccatggg cctgctcctg ctggttctca ttc 43
<210> 59
   <211> 30
   <212> DNA
   <213> Artificial sequence
<220>
   <223> PCR primer
<400> 59
   atcacctcga ggcttcagta caggtggata 30
<210> 60
   <211> 54
   <212> DNA
   <213> Artificial sequence
<220>
   <223> PCR primer
<400> 60
   gctaatacga ctcactatag gaacagacca ccatgggtct gctcctgctg cttc 54
<210> 61
   <211> 53
   <212> DNA
   <213> Artificial sequence
<220>
   <223> PCR primer
<400> 61
   gctaatacga ctcactatgg aacagaccac catgctcctg ctggttctca ttc 53
<210> 62
   <211> 30
   <212> DNA
   <213> Artificial sequence
<220>
   <223> PCR primer
<400> 62
   atcacctcga ggcttcagta caggtggata 30
<210> 63
   <211> 20
   <212> DNA
   <213> Artificial sequence
<220>
   <223> PCR primer
<400> 63
   tttgagcaag ggtgattcat 20
<210> 64
   <211> 521
   <212> PRT
   <213> Artificial sequence
<220>
   <223> Deletion mutant of Syncitin 2
<400> 64
   Met Gly Leu Leu Leu Leu Val Leu Ile Leu Thr Pro Ser Leu Ala Ala
   1               5                   10                  15
   Tyr Arg His Pro Asp Phe Pro Leu Leu Glu Lys Ala Gln Gln Leu Leu
               20                  25                  30
   Gln Ser Thr Gly Ser Pro Tyr Ser Thr Asn Cys Trp Leu Cys Thr Ser
           35                  40                  45
   Ser Ser Thr Glu Thr Pro Gly Thr Ala Tyr Pro Ala Ser Pro Arg Glu
       50                  55                  60
   Trp Thr Ser Ile Glu Ala Glu Leu His Ile Ser Tyr Arg Trp Asp Pro
   65                  70                  75                  80
   Asn Leu Lys Gly Leu Met Arg Pro Ala Asn Ser Leu Leu Ser Thr Val
                   85                  90                  95
   Lys Gln Asp Phe Pro Asp Ile Arg Gln Lys Pro Pro Ile Phe Gly Pro
               100                 105                 110
   Ile Phe Thr Asn Ile Asn Leu Met Gly Ile Ala Pro Ile Cys Val Met
           115                 120                 125
   Ala Lys Arg Lys Asn Gly Thr Asn Val Gly Thr Leu Pro Ser Thr Val
       130                 135                 140
   Cys Asn Val Thr Phe Thr Val Asp Ser Asn Gln Gln Thr Tyr Gln Thr
   145                 150                 155                 160
   Tyr Thr His Asn Gln Phe Arg His Gln Pro Arg Phe Pro Lys Pro Pro
                   165                 170                 175
   Asn Ile Thr Phe Pro Gln Gly Thr Leu Leu Asp Lys Ser Ser Arg Phe
               180                 185                 190
   Cys Gln Gly Arg Pro Ser Ser Cys Ser Thr Arg Asn Phe Trp Phe Arg
           195                 200                 205
   Pro Ala Asp Tyr Asn Gln Cys Leu Gln Ile Ser Asn Leu Ser Ser Thr
   210 215 220
   Ala Glu Trp Val Leu Leu Asp Gln Thr Arg Asn Ser Leu Phe Trp Glu
   225 230 235 240
   Asn Lys Thr Lys Gly Ala Asn Gln Ser Gln Thr Pro Cys Val Gln Val
   245 250 255
   Leu Ala Gly Met Thr Ile Ala Thr Ser Tyr Leu Gly Ile Ser Ala Val
   260 265 270
   Ser Glu Phe Phe Gly Thr Ser Leu Thr Pro Leu Phe His Phe His Ile
   275 280 285
   Ser Thr Cys Leu Lys Thr Gln Gly Ala Phe Tyr Ile Cys Gly Gln Ser
   290 295 300
   Ile His Gln Cys Leu Pro Ser Asn Trp Thr Gly Thr Cys Thr Ile Gly
   305 310 315 320
   Tyr Val Thr Pro Asp Ile Phe Ile Ala Pro Gly Asn Leu Ser Leu Pro
   325 330 335
   Ile Pro Ile Tyr Gly Asn Ser Pro Leu Pro Arg Val Arg Arg Ala Ile
   340 345 350
   His Phe Ile Pro Leu Leu Ala Gly Leu Gly Ile Leu Ala Gly Thr Gly
   355 360 365
   Thr Gly Ile Ala Gly Ile Thr Lys Ala Ser Leu Thr Tyr Ser Gln Leu
   370 375 380
   Ser Lys Glu Ile Ala Asn Asn Ile Asp Thr Met Ala Lys Ala Leu Thr
   385 390 395 400
   Thr Met Gln Glu Gln Ile Asp Ser Leu Ala Ala Val Val Cys Leu Ala
   405 410 415
   Leu Asp Glu Lys Cys Cys Phe Trp Val Asn Gln Ser Gly Lys Val Gln
   420 425 430
   Asp Asn Ile Arg Gln Leu Leu Asn Gln Ala Ser Ser Leu Arg Glu Arg
   435 440 445
   Ala Thr Gln Gly Trp Leu Asn Trp Glu Gly Thr Trp Lys Trp Phe Ser
   450 455 460
   Trp Val Leu Pro Leu Thr Gly Pro Leu Val Ser Leu Leu Leu Leu Leu
   465 470 475 480
   Leu Phe Gly Pro Cys Leu Leu Asn Leu Ile Thr Gln Phe Val Ser Ser
   485 490 495
   Arg Leu Gln Ala Ile Lys Leu Gln Thr Asn Leu Ser Ala Gly Arg His
   500 505 510
   Pro Arg Asn Ile Gln Glu Ser Pro Phe
   515 520
<210> 65
   <211> 17
   <212> PRT
   <213> Artificial sequence
<220>
   <223> Immunosuppressive sequence of Syncitin 2
<400> 65
   Leu Gln Asn Arg Arg Gly Leu Asp Met Leu Thr Ala Ala Gln Gly Gly
   1 5 10 15
   Ile
<210> 66
   <211> 24
   <212> DNA
   <213> Artificial sequence
<220>
   <223> PCR primer
<400> 66
   tcgagctctt aggtcaccat gggc 24
<210> 67
   <211> 25
   <212> DNA
   <213> Artificial sequence
<220>
   <223> PCR primer
<400> 67
   aacggacgcg tctctgtcgt gttcc 25
<210> 68
   <211> 21
   <212> DNA
   <213> Artificial sequence
<220>
   <223> PCR primer
<400> 68
   cgtcttgtct gctgcagcat c 21
<210> 69
   <211> 83
   <212> DNA
   <213> Artificial sequence
<220>
   <223> PCR primer
<400> 69
   catactcgag tcaacgcgta gaatcgagac cgaggagagg gttagggata ggcttaccgt 60
   acggctcgta ctctataggc ttc 83

## Description

The present invention relates to a new protein with fusogenic properties, nucleic acid sequences encoding for said protein, and pharmaceutical compositions comprising them.

The human genome contains a large fraction (ca. 8%) of elements of retroviral origin, with thousands of sequences close to the integrated proviral form of infectious retroviruses, with two long terminal repeats (LTR) bordering internal regions homologous to the *gag, pol* and *env* genes (Löwer, R., Lower, J. & Kurth, R. (1996) Proc. Natl. Acad. Sci. USA 93, 5177-5184; Boeke, J. D. & Stoye, J. P. (1997) in Retroviruses, ed28s. Coffin, J. M., Hughes, S. H. & Varmus, H. E. (Cold Spring Harbor Laboratory Press, New York), pp. 343-436; Lander, E. S., Linton, L. M., Birren, B., Nusbaum, C., Zody, M. C., Baldwin, J., Devon, K., Dewar, K., Doyle, M., FitzHugh, W. & al. (2001) Nature 409, 860-921). These elements, named Human Endogenous Retro Viruses (HERV), are most probably the proviral remnants of ancestral germline infections by active retroviruses, which have thereafter been transmitted in a mendelian manner. HERV can be grouped according to sequence homologies into approximately 100 distinct families, each containing a few to several hundreds elements.

Strong similarities between HERV and present-day retroviruses can be inferred from phylogenetic analyses on the Reverse Transcriptase domain of the *pol* gene or the TransMembrane moiety (TM) of the *env* gene, which disclose interspersion of both classes of elements and suggest a common history and shared ancestors (Tristem, M. (2000) J. Virol. 74, 3715-3730; Benit, L., Dessen, P. & Heidmann, T. (2001) J. Virol. 75, 11709-11719). Similarities are also observed at the functional level.

Thus, the envelope protein of a HERV-W family member, Syncitin (hereafter named Syncitin 1), has retained the ability to interact with the receptor of the D-type retroviruses (Blond, J. L., Lavillette, D., Cheynet, V., Bouton, O., Oriol, G., Chapel-Fernandès, S., Mandrand, B., Mallet, F. & Cosset, F.-L. (2000) J. Virol. 74, 3321-3329, Lavillette, D., Marin, M., Ruggieri, A., Mallet, F., Cosset, F. L. & Kabat, D. (2002) J. Virol. 76, 6442-6452) and can confer infectivity on pseudotyped retroviral particles (Lavillette, D., Marin, M., Ruggieri, A., Mallet, F., Cosset, F. L. & Kabat, D. (2002) J. Virol. 76, 6442-6452, An, D. S., Xie, Y.-M. &' Chen, I. S. Y. (2001) J. Virol. 75, 3488-3489).

As a consequence of the close relationship between HERVs and infectious retroviruses, and despite the fact that most HERVs have accumulated mutations, deletions and/or truncations, it remains plausible that some elements still possess functions of infectious retroviruses, that may have been diverted by the host to its benefit. Along this line, it has been proposed (reviewed in ref. Venables, S., Brookes, M., Fan, W., Larsson, E., Maini, R. N. & Boyd, M. T. (1995) Virology 211, 589-592, Harris, J. R. (1998) BioEssays 20, 307-316) that the HERV envelopes could play a role in several processes including i) protection against infection by present-day retroviruses through receptor interference (Best, S., Le Tissier, P. R. & Stoye, J. P. (1997) Trends Microbiol 5, 313-318), ii) protection of the fetus against the maternal immune system via an immunosuppressive domain located in the envelope TM subunit (Cianciolo, G. J., Copeland, T., Orozlan, S. & Snyderman, R. (1985) Science 230, 453-455; Mangeney, M. & Heidmann, T. (1998) Proc. Natl. Acad. Sci. USA 95, 14920-14925), and iii) placenta morphogenesis through fusogenic effects allowing differentiation of cytotrophoblast cells into the syncytiotrophoblast (Blond, J. L., Lavillette, D., Cheynet, V., Bouton, O., Oriol, G., Chapel-Fernandes, S., Mandrand, B., Mallet, F. & Cosset, F.-L. (2000) J. Virol. 74, 3321-3329; Mi, S., Lee, X., Li, X., Veldman, G. M., Finnerty, H., Racie, L., LaVallie, E., Tang, X. Y., Edouard, P., Howes, S., Keith, J. C. J. & McCoy, J. M. (2000) Nature 403, 785-788).

In accordance with a symbiotic role for HERVs, WO 01 16171 discloses that the HERV-W envelope gene product (Syncitin 1) is a highly fusogenic glycoprotein which is specifically expressed in the placenta and can mediate cell-cell fusion *ex vivo*. This HERV-W envelope could be used in cancer treatment by promoting the formation a syncitia by cancerous cells, thus leading to their death. Disorders in the expression of the HERV-W envelope could impair placental morphogenesis.

However, the HERV envelope can not mediate the fusion of all cell types, therefore it can not be used for the treatment of all cancer types. Besides, disorders in the expression-of the HERV-W envelope do not account for all placental morphogenesis disorders and other HERV envelopes might be implicated.

Therefore, an object of the present invention is to provide a new fusogenic HERV envelope, with a cell-specificity different from that of HERV-W envelope and which accounts for all the placental morphogenesis disorders arising from disorders in the expression of a HERV envelope Other than the HERV-W envelope.

Another object of the present invention is to provide a new HERV envelope which presents an immunosuppressive activity in addition to a fusogenic activity, said new HERV envelope representing the sole example of a HERV envelope with both fusogenic activity and immunosuppressive activity.

**The inventors have isolated** a protein or a polypeptide, characterized in that it corresponds to an amino acid sequence, comprising or being constituted by:
● a sequence corresponding to SEQ ID NO: 2 (Syncitin 2), or
● a sequence derived from SEQ ID NO: 2 by insertion, deletion or substitution of at least one amino acid, provided that the protein or the polypeptide corresponding to said derived sequence presents a fusogenic activity similar to that of (Syncitin 2 and/or an immunosuppressive activity similar to that of Syncitin 2 and/or an antigenic activity similar to that of Syncitin 2, or
● a homologous sequence presenting at least 85% amino acid identity with SEQ ID NO: 2, provided that the protein or the polypeptide corresponding to said homologous sequence presents a fusogenic activity similar to that of Syncitin 2, and/or an immunosuppressive activity similar to that of Syncitin 2 and/or, an antigenic activity similar to that of Syncitin 2.

**The inventors have also isolated** a protein or a polypeptide as defined above, characterized in that it corresponds to an amino acid sequence, comprising or being constituted by:
- a sequence corresponding to SEQ ID NO: 2 (Syncitin 2), or
- a sequence derived from SEQ ID NO: 2 by insertion, deletion or substitution of at least one amino acid, provided that the protein or the polypeptide corresponding to said derived sequence presents a fusogenic activity similar to that of Syncitin 2 and/or an antigenic activity similar to that of Syncitin 2, or
- a homologous sequence presenting at least 85% amino acid identity with SEQ ID NO: 2, provided that the protein or the polypeptide corresponding to said homologous sequence presents a fusogenic activity similar to that of Syncitin 2, and/or an antigenic activity similar to that of Syncitin 2.

**The inventors have also isolated** a protein or a polypeptide as defined above, characterized in that it corresponds to an amino acid sequence, comprising or being constituted by:
- a sequence corresponding to SEQ ID NO: 2 (Syncitin 2), or
- a sequence derived from SEQ ID NO: 2 by insertion, deletion or substitution of at least one amino acid, provided that the protein or the polypeptide corresponding to said derived sequence presents an immunosuppressive activity similar to that of Syncitin 2 and/or an antigenic activity similar to that of Syncitin 2, or
- a homologous sequence presenting at least 85% amino acid identity with SEQ ID NO: 2, provided that the protein or the polypeptide corresponding to said homologous sequence presents an immunosuppressive activity similar to that of Syncitin 2, and/or an antigenic activity similar to that of Syncitin 2.

**The inventors have also isolated** a protein or a polypeptide as defined above, characterized in that it corresponds to an amino acid sequence, comprising or being constituted by:
- a sequence corresponding to SEQ ID NO: 2 (Syncitin 2), or
- a sequence derived from SEQ ID NO: 2 by insertion, deletion or substitution of at least one amino acid, provided that the protein or the polypeptide corresponding to said derived sequence presents a fusogenic activity similar to that of Syncitin 2, or
- a homologous sequence presenting at least 85% amino acid identity with SEQ ID NO: 2, provided that the protein or the polypeptide corresponding to said homologous sequence presents a fusogenic activity similar to that of Syncitin 2.

The expression "fusogenic activity" relates to the ability of said protein or polypeptide to promote the fusion, *i.e.* the coalescence, of biological membranes, in particular the fusion of cellular membranes.

Membrane fusions between eukaryotic cells lead to the formation of a syncitium. A syncitium is in particular characterized by the presence of several nuclei enclosed by a single cellular membrane.

Such a fusogenic activity can be detected according to the following procedure:
- an expression vector carrying the coding sequence of said polypeptide or protein under the control of a promoter is transformed into host cells, to obtain host cells producing said polypeptide or protein, the formation of a syncitium from said host cells being indicative of a fusogenic activity of said polypeptide or protein;
- alternatively, it is possible to contact said host cells with untransformed cells of various lineages, the formation of a syncitium arising from the fusion of transformed cells with untransformed cells being indicative of a fusogenic activity of said polypeptide or protein and of the presence of a receptor of said polypeptides or protein in said untransformed cells.

Syncitin 2 is a HERV envelope presenting a fusogenic activity. Syncitin 2 is also refered to as HERV-FRD in the examples.

The expression "presents a fusogenic activity similar to that of Syncitin 2" means that the protein or the polypeptide to which it relates has the same cell-specificity than Syncitin 2 and/or a fusogenic activity which represents at least 50% of the fusogenic activity of Syncitin 2 in the same conditions.

Avantageously, Syncitin 2 and the HERV-W envelope (Syncitin 1) are the only two HERV envelopes with fusogenic activity normally expressed in the human body.

Equally advantageous, Syncitin 2 presents a fusogenic cell-specificity different from that of the HERV-W envelope. That is, the panel of cells which can be brought to form syncitia by Syncitin 2 is different from the panel which can be brought to form syncitia by Syncitin 1. The cells which are liable to specifically fusion with cells expressing respectively Syncitin 1 or 2, express membrane receptors respectively specific to Syncitin 1 or 2.

The expression "immunosuppressive activity" relates to the ability of said protein or polypeptide to enable the cells carrying it to escape immune control.

Such an activity can be evidenced and measured following the procedure described in Mangeney & Heidmann, PNAS 1998, and Mangeney et al., J.Gen.Virol. 2001, and as described in Example 4. Advantageously Syncitin 2 presents an immunosuppressive activity.

The expression "presents an immunosuppressive activity similar to that of Syncitin 2" means that the protein or the polypeptide to which it relates has an immunosuppressive activity which represents at least 50% of the immunosuppressive activity of Syncitin 2 in the same conditions.

The expression "antigenic activity" relates to the ability of said protein or polypeptide to activate the immune system and to elicit an immune response directed against said protein or polypeptide.

The expression "presents an antigenic activity similar to that of Syncitin 2" means that the protein or the polypeptide to which it relates is liable to elicit an immune response directed against Syncitin 2.

Syncitin 2 is essentially expressed in the placenta, and impaired expression of Syncitin 2 may result in placental morphogenesis disorders, in particular syncitiotrophoblast disorders. Such disorders may arise either from an expression of Syncitin 2 higher or lower than normal. Higher than normal expression of Syncitin 2 leads to the formation and/or the development of unwanted syncitia, such as trophoblastic invasion which occurs in choriocarcinoma.
Conversely, lower than normal expression of Syncitin 2 leads to the absence or to the underdevelopment of normally occurring syncitia, such as trophoblast underdevelopment, which may be a cause of unfertility.

According to **the above,** fragments of the Syncitin 2 protein **have been isolated,** said fragments being constituted by a sequence deriving from SEQ ID NO: 2 by deletion of at least one amino acid and containing at least 20 contiguous amino acids.

According to another particular embodiment, a protein or a polypeptide is characterized in that it corresponds to an amino acid sequence comprising or being constituted by a sequence derived from SEQ ID NO: 2 by deletion of at least the 17 contiguous amino acids from position 414 to position 430 of SEQ ID NO: 2.

The 17 contiguous amino acids from position 414 to position 430 of SEQ ID NO: 2 correspond to SEQ ID NO: 65. This sequence has a major role in the immunosuppressive activity of Syncitin 2. Proteins or polypeptides devoid of SEQ ID NO: will not generally have any immunosuppressive activity. Such proteins or polypeptides devoid of SEQ ID NO: 65 encompass in particular fragments comprising at least 20 amino acids selected from contiguous amino acids from position 1 to position 413 or from position 431 to 538 of SEQ ID NO: 2.

A protein or a polypeptide **disclosed above** is characterized in that it corresponds to SEQ ID NO: 64.

SEQ ID NO: 64 corresponds to SEQ ID NO: 2 from which SEQ ID NO: 65 has been deleted. Advantageously, a protein or a polypeptide corresponding to such a sequence presents a fusogenic activity and an antigenic activity similar to that of Syncitin 2 but is devoid of any immunosuppressive activity. Such a protein or polypeptide presents a better antigenic activity than Syncitin 2'as it is devoid of immunosuppressive activity, which makes it a good candidate for the manufacture of a vaccine composition.

**The inventors have also isolated** a nucleic acid, characterized in that it corresponds to a sequence encoding a protein or a polypeptide as defined above, and/or to its complementary sequence.

**The inventors have also isolated** a nucleic acid, characterized in that it corresponds to a nucleic acid sequence comprising or being constituted by:
- a sequence, corresponding to SEQ ID NO: 1, or
- a sequence derive from SEQ ID NO: 1 by insertion, deletion or substitution of at least one nucleotide, provided that said derived sequence encodes a protein or a polypeptide presenting a fusogenic activity similar to that of Syncitin 2, or
- a homologous sequence presenting at least 66% nucleotide identity with SEQ ID NO: 1, provided that said homologous sequence encodes a protein or a polypeptide presenting a fusogenic activity similar to that of Syncitin 2, or
- fragments of said sequences, such as SEQ ID NO: 3 or 4,
and/or to its complementary sequence.

**It is also made reference to** a nucleic acid, characterized in that it hybridizes with a nucleic acid as defined above under stringent conditions.

Such stringent conditions are defined in particular in Church, G. M. & Gilbert, W. (1984) Proc. Natl. Acad. Sci. USA 81, 1991-1995 at a temperature of 65°C.

**The inventors have also isolated** a prokaryotic or eukaryotic expression vector, characterized in that it comprises a nucleic acid as defined above and the elements required for its expression in a host cell.

An eukaryotic expression vector is particularly useful for gene therapy, in particular to compensate for disorders resulting in an absence of expression or a lower than normal expression of fusogenic proteins, such as Syncitin 2. Such vectors can also be used to transfect tumoral cells to induce the formation of a syncitium resulting from the fusion of transfected tumoral cells with transfected or untransfected tumoral cells. Such vectors may be advantageously derived from vectors classically used in gene therapy protocols, such as retroviral or lentiviral vectors.

**The inventors have also isolated** to a prokaryotic or eukaryotic cell, characterized in that it comprises vector as defined above.

Advantageously, such cells express fusogenic polypeptide or protein and have the capacity of forming syncitia.

Advantageously, such cells can be administered to an individual suffering from cancer at a tumor site, to promote the formation of a syncitium resulting from the fusion of cells with tumoral cells. Alternatively, such cells can also be administered to an individual suffering from a placental morphogenesis disorders caused by the absence of formation or the underdevelopment of functional syncitia, such as the syncitiontrophoblast, to promote the formation and/or the development of syncitia, such as the syncitiotrophoplast.

**Patients** suffering from cancers can be treated as follows:
- healthy cells, in particular healthy cells originating from a tissue similar to the tissue which carries tumoral cells, are taken from the patient,
- said healthy cells are transfected with a vector **mentioned above**,
- transfected cells are administrated to the patient, in particular at a site close to the targeted tumor,
- the tumoral cells are induced to form syncitia by the transfected cells, and die.

The killing of tumor cells by syncitia formation promoted by fusogenic proteins is notably described in Bateman et al., (2002) Cancer Research 15: 6566-6578.

**The inventors have also isolated** an antibody, characterized in that it is directed against a protein or a polypeptide as defined above.

The expression "antibody" as intended herein relates to monoclonal or polyclonal antibodies, as well as to antibody fragments.

Antibodies **mentioned above** can be obtained by methods well known to the man skilled in the art. For instance monoclonal antibodies can be produced according to Köhler and Milstein (1975) Nature 256:495-497 or Galfre et al. (1977) Nature 266:522-550 and polyclonal antibodies can be produced according to Roda and Bolelli (1980) Journal of Steroid Biochemistry, 13:449-454.

Antibody fragments **mentioned above** relate more particularly to Fab, F(ab')₂, or scFv fragments. Fab and F(ab')₂ fragments are particularly well known to the man skilled in the art, and can be obtained respectively by papain and pepsin proteolytic cleavage of antibodies, scFv fragments are in particular described in Blazar et al. (1997) Journal of Immunology 159-5821-5833 or Bird et al. (1988) Science 242:423-426, advantageously scFv fragments are recombinant.

**The inventors have also** purified **a** natural human protein, characterized in that:
it is recognized by an antibody as defined above and,
it presents a fusogenic activity.

**A** pharmaceutical or a vaccinal composition, **is also disclosed, said composition** comprising as active substance at least one substances selected from the group comprising:
a protein or a polypeptide as defined above,
a nucleic acid as defined above,
a vector as defined above, or
a cell as defined above;
in association with a pharmaceutically acceptable vehicle.

A protein or a polypeptide **as mentioned above** can be used for the treatment of abnormal syncitium formation and/or development, *i.e*. syncitium formation and/or development which does not occur under normal conditions, mediated by an above normal expression of Syncitin 2, by competing for Syncitin 2 receptor sites on cells. A protein or a polypeptide **mentioned above** could also be used to target compounds, in particular therapeutic compounds to cells which carry receptors for Syncitin 2.

Furthermore, a protein or a polypeptide **mentioned above** can be used for the immunosuppressive treatment of patients, such as patients afflicted with allergy, autoimmune disease or graft rejection.

Possibly, a protein **mentioned above** has anti-cancerous prophylactic properties and can be used for the manufacture of a vaccinal composition. As HERV-FRD is liable to be involved in cancer development due to its immunosuppressive activity which can induce immune tumor escape and due to its expression in tumors, administration of a protein **mentioned above** to a patient, could help preventing the development of tumors expressing this protein thanks to immunological reactions elicited in response to said administration.

In particular, proteins or polypeptides devoid of immunosuppressive activity **mentioned above**, such as the protein corresponding to SEQ ID NO: 65, are particularly useful since they have an antigenic activity similar to that of Syncitin 2 without the drawback of its immunosuppressive activity, they can therefore easily elicit an immune response against tumors expressing HERV-FRD. Vaccinal compositions comprising such proteins or polypeptides devoid of immunosuppressive activity are therefore particularly useful in anti-tumor prevention and/or therapy.

A nucleic acid **mentioned above** and a vector **mentioned above** can be used in gene therapy to compensate for a lower than normal expression of a fusogenic protein, in particular of Syncitin 2, notably in the placenta. Alternatively, such nucleic acids and vectors can be used to transfect tumor cells so as to induce tumor cell fusion and consequently tumor destruction.

A cell **mentioned above** can be used to cure tissues which should undergo cell fusion but for which this function is impaired, or to promote the formation of syncitia in tumors.

**It is also disclosed a** vaccinal composition as defined above, comprising as active substance a protein or a polypeptide as defined above, in association with a pharmaceutically acceptable vehicle.

**It is also disclosed** a pharmaceutical composition, comprising as active substance at least one substance selected from the group comprising:
an antibody as defined above,
an antisense sequence hybridizing to SEQ ID NO: 1,
a RNAi comprising a sequence hybridizing to SEQ ID NO: 1, or
an aptamer directed against a protein corresponding to SEQ ID NO: 2,
in association with a pharmaceutically acceptable vehicle.

An antibody **mentioned above** can be used to prevent the binding of Syncitin 2 to its receptor on cells, thus preventing syncitium formation and/or development. Likely, an antisens sequence **mentioned above** is meant to impair the translation a Syncitin 2 encoding mRNA thus impairing its expression and as a consequence preventing syncitium formation and/or development.

Similarly, a RNAi (interference RNA) **mentioned above** prevents the expression of Syncitin 2, and as a consequence prevents syncitium formation and/or development.

An aptamer **mentioned above** is a nucleic acid, in particular a ribonucleic acid, the folding of which allows its binding to the Syncitin 2 protein so as to prevent the Syncitin 2 protein from promoting the formation and/or the develomment of syncitia.

The present invention relates to the use of at least one substance selected from the group comprising:
a protein or a polypeptide as defined above,
a nucleic acid as defined above,
for the manufacture of a drug useful for the prevention and/or the treatment: a placental morphogenesis disorder

The invention relates to the use of a protein or a polypeptide, being constituted by:
- a sequence SEO ID NO: 2 (Syncitin 2), or
- a homologous sequence derived from SEQ ID NO: 2 presenting at least 85% amino acid identity with SEO ID NO: 2, provided that said homologous sequence presents a fusogenic activity similar to that of Syncitin 2, and an immunosuppressive activity similar to that of Syncitin 2 and an antigenic activity similar to that of Syncitin 2,
for the manufacture of a drug useful for the prevention and/or the treatment of a placental morphogenesis disorder.
Moreover, the invention also relates to the use of a nucleic acid, characterized in that it encodes a protein or a polypeptide as defined above, in particular said nucleic acid comprising or being constituted by sequence SEQ ID NO: 1, for the manufacture of a drug useful for the Prevention and/or the treatment of a placental morphogenesis disorder.

Drugs mentioned above are useful for treating cancers by promoting the formation of syncitia by cancerous cells, which leads to cellular death. As mentioned before, the killing of tumor cells by syncitia formation promoted by fusogenic proteins is notably described in Bateman et al., (2002) Cancer Research 15: 6566-6578.

Drugs according to the invention can also be used to compensate for the absence of a functional Syncitin 2 in tissues in which it is normally present, such absence leading to a fusogenicity disorder or a syncitium morphogenesis disorder, in which the physiologic formation, of a syncitium is impaired or prevented. In the case of placental morphogenesis, an impaired or deficient syncitium formation results in infertility.

Finally, drugs mentioned above could be used as anti-tumor vaccines, whereby the administration of said drugs to an individual could lead to an immunological reaction directed against said drugs, thus controlling and/or destroying tumor which, express said the Syncitin 2 protein.

It is also disclosed a method for detecting the Syncitin 2 protein or for measuring the concentration of the Syncitin 2 protein in a biological sample, characterized in that it comprises a step of contacting said biological sample with an antibody as defined above.

Such methods are useful for the diagnosis of pathologies in which the expression of Syncitin 2 is modified. Such methods are also useful for assessing transfection efficiency in gene therapy assays in which nucleic acids mentioned above are introduced into cells.

Prefereably, the biological sample is a placental sample.

It is also disclosed a method for detecting the Syncitin 2 mRNA or for measuring the concentration of the expression of the Syncitin 2 mRNA in a biological sample, characterized in that it comprises a step of contacting said biological sample with at least one nucleic acid as defined above.

The Syncitin 2 mRNA expression detection or measure methods which can be used with nucleic acids mentioned above notably comprise Reverse Transcriptase Polymerase Chain Reaction (RT-PCR), Nuceic Acid Based Sequence Amplification (NASBA), *in situ* hybridization and Northern blot. Such methods are well known to the man skilled in the art.

Such methods are useful for the diagnosis of pathologies in which the expression of Syncitin 2 is modified. Such methods are also useful for assessing transfection efficiency in gene therapy assays in which nucleic acids mentioned above are introduced into cells.

Preferably, the biological sample is a placental sample.

Preferably, in the above defined detection method, the nucleic acids correspond to SEQ ID NO: 3 and SEQ ID NO: 4.

Advantageously, nucleic acids corresponding to SEQ ID NO: 3 and 4 can be used as primers for the detection or for the assessment of the concentration of the Syncitin 2 mRNA by RT-PCR, in particular by real-time quantitative RT-PCR using the following procedure:

RNA is first extracted from biological samples in which the Syncitin 2 mRNA concentration is to be assessed, according to methods well known to the man skilled in the art. RNA concentration is then quantified spectrophotometrically. Five micrograms of each RNA sample are submitted to DNAse treatment to eliminate DNA contaminants. One microgram of RNA from each sample is reverse transcribed using a virus Reverse Transcriptase, notably in presence of a ribonuclease inhibitor, deoxynucleotides, MgCl₂, KCl, and random hexamers. The complementary DNAs thus obtained are then diluted in nuclease-free water. Real-Time quantitative PCR is achieved using a complementary DNA equivalent of 20 ng of total RNA notably in presence of a thermostable polymerase, the primers mentioned above , deoxynucleotides and MgCl₂. The amplification is performed using a 2 min step at 50°C, a 10 min denaturation step at 95°C, followed by 40 cycles of 15 s denaturation at 95°C, 1 min primer annealing and polymerization step at 60°C. To normalize for differences in the amount of total RNA added to the reaction, amplification of 18S total ribosomal RNA was performed as an endogenous control. The relative expression in each sample is calculated with respect to a standard calibration curve (e.g, dilution series of genomic DNA).

A method for the in vitro diagnosis of pathologies linked to the level of expression of Syncitin 2 in a tissue, comprising the following steps:
- taking a tissue sample from an individual,
- measuring, in said sample, the concentration Syncitin 2 mRNA, or the concentration of the Syncitin 2 protein.

Advantageously, measure and detection methods as described above can be used in the *in vitro* diagnosis method mentioned above .

It is also disclosed a method for the screening of drugs liable to modify the fusogenic activity of a polypeptide or a protein as defined above, characterize in that a drug to screen is contacted with cells expressing said polypeptide or protein, and the presence or the absence of formation of a syncitium by said cells is observed.

Drugs which lead to the absence of formation of a syncitium can be used to block abnormal syncitium formation and/or development, such as in trophoblast invasion which occurs in choriocarcinoma.

Drugs which lead to the formation of a syncitium can be used to promote the formation and/or the development of syncitium, for instance in cases where there is an absence of formation and/or an underdevelopment of functional syncitia.

### Description of the figures

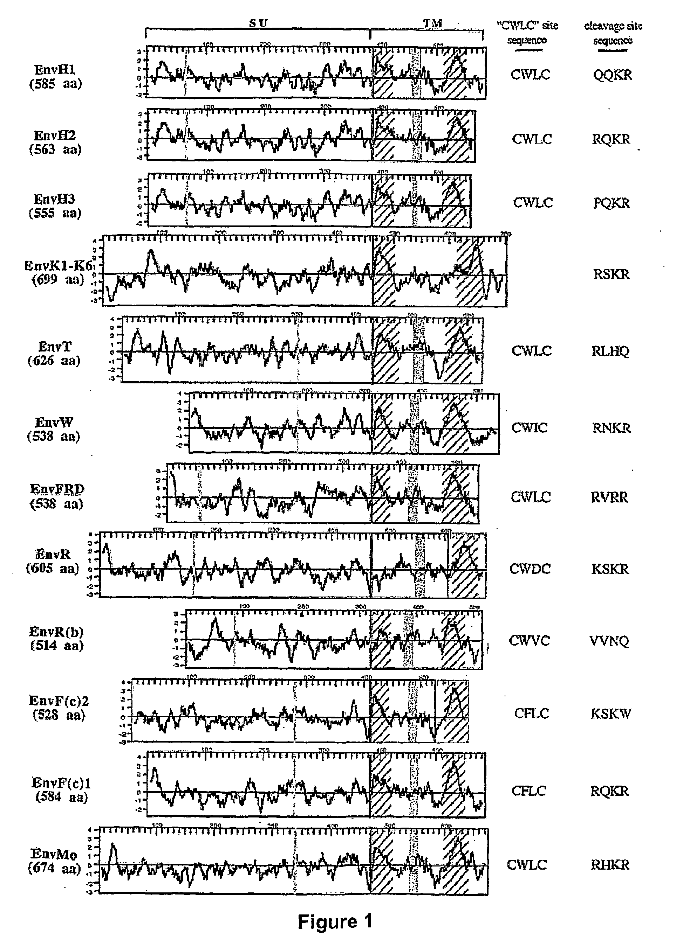

Figure 1. Structural organization and characteristic features of the 16 identified HERV envelopes. Hydrophobicity profiles and characteristic domains of the 16 HERV envelope proteins (the 6 HERV-K HML2 envelopes being almost identical are represented by a unique protein) and of the Moloney murine leukemia virus envelope. The black rectangles delineate the open reading frames, and the gray rectangles at the end of R and Fc2 envelope represent the short reading frames present downstream of the stop codon. Stripped boxes correspond to hydrophobic regions associated with the fusion peptide and the transmembrane region, respectively; the « CWLC » motifs (consensus C-X-X-C) and the proteolytic cleavage sites (consensus R/K-X-R/K-R) are represented with pale and dark gray vertical bars respectively, and their sequence are indicated in the right part of the figure; the putative immunosuppressive domain is represented with a gray box.

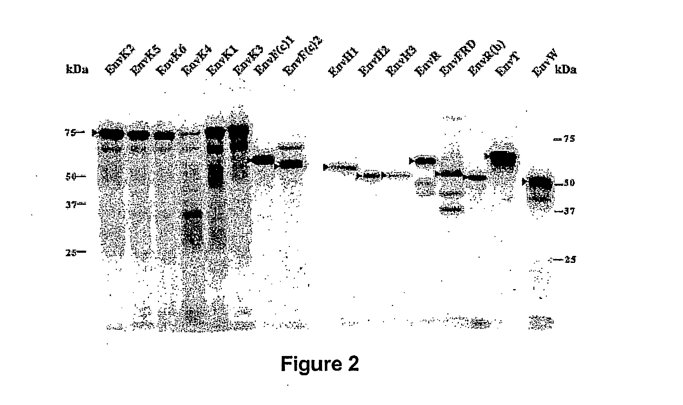

**Figure 2****.** SDS-PAGE analysis of the radiolabelled proteins produced upon direct *in vitro* transcription / translation assays of the amplification products of the coding retroviral envelope genes obtained from the corresponding BAC clones.

Arrowheads point to the translation products of the expected size for each envelope gene. Bands of higher molecular weight are observed for envF(c)2, most probably associated with the presence of a frameshift signal at the env gene 3' end.

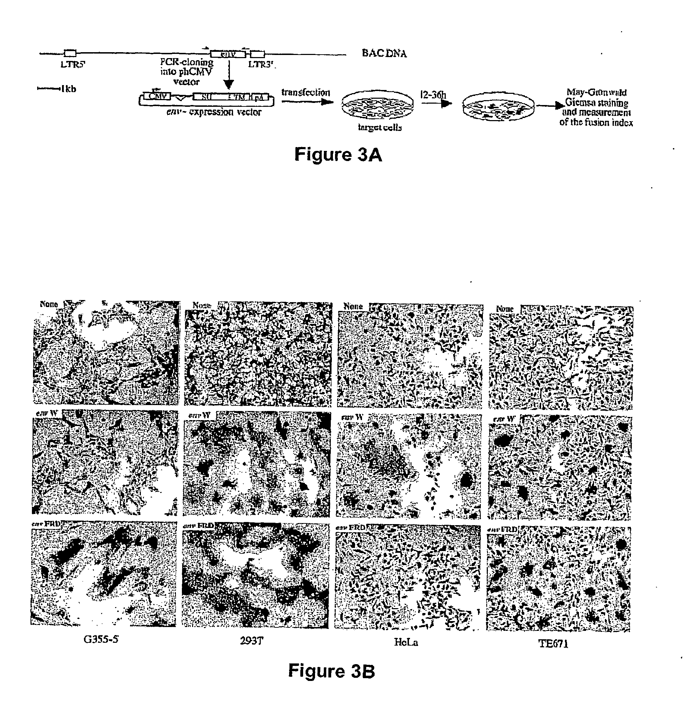

**Figure 3A and Figure 3****B.** Envelope-mediated cell-cell fusion. (Figure 3A) Construction of envelope-expressing vectors and rationale of the fusion assay. Each of the 16 envelope genes was PCR-amplified from BAC DNA and cloned into the phCMV expression vector. The *env* genes inserted in-between the ß-globin intron and pA sequences are schematized with the putative cleavage site between the surface (SU) and the transmembrane (TM) envelope subdomains. Cells were transfected with the env-expressing vectors and stained with May-Grunwald and Giemsa solutions 12 h (for the G355-5 cells) or 36 h (for the other cell types) after transfection. Fusion indices were calculated as indicated in Materials and Methods. (Figure 3B) Syncytia formation by HERV envelope glycoproteins in various cell types. Cells were transfected with vectors expressing the HERV-FRD envelope (envFRD), the HERV-W envelope (envW), or an "empty' vector (none).

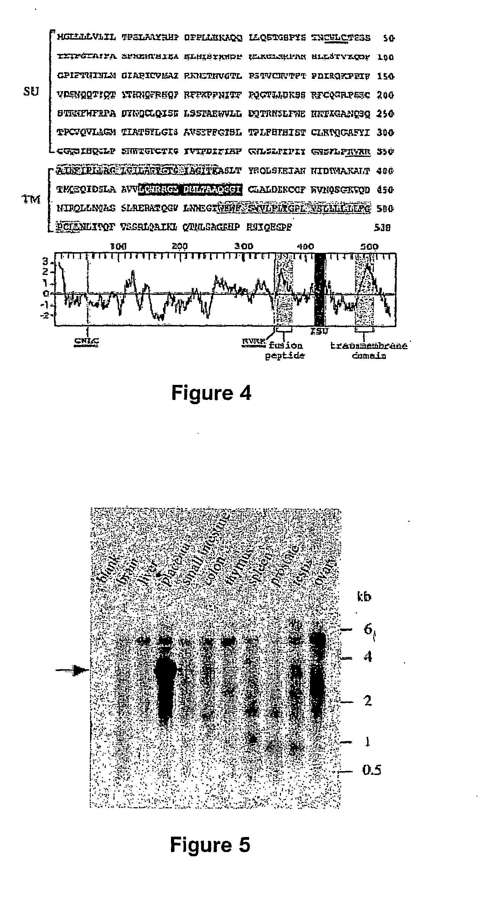

**Figure 4****.** Primary sequence; hydrophobicity profile, and predicted features of the HERV-FRD envelope. The SU and TM moities of the envelope are delineated, with the canonical RVRR cleavage site between the two subunits underlined (consensus: R/K-X-R/K-R); in the TM subunit, the hydrophobic fusion peptide and transmembrane domains are shaded in light gray, and the putative immunosuppressive domain (ISU) in dark grey; in the SU subunit, the canonical CWLC domain involved in SU-TM interaction, is underlined.

**Figure 5****.** Northero blot analysis of HERV-FRD env expression in human tissues. Poly(A)⁺ RNAs (Ambion, Human Northern Blot 2) were probed with an antisense-strand HERV-FRD env riboprobe. Exposure time 12 h.

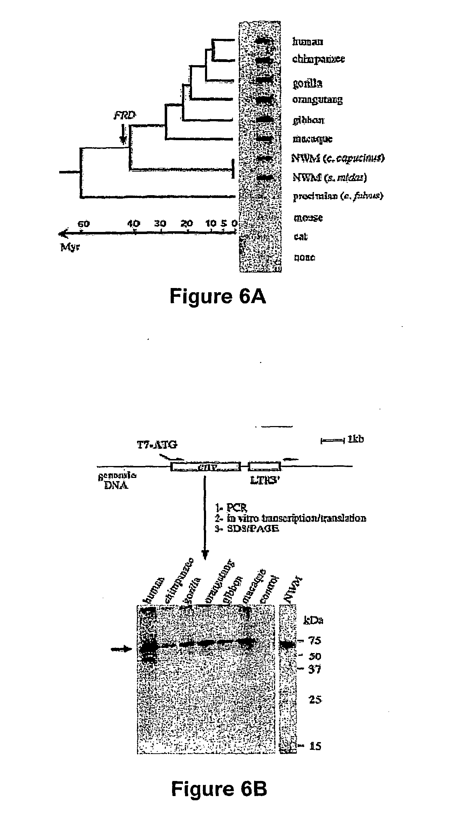

**Figure 6A and Figure 6****B.** Conservation of the HERV-FRD env locus and open reading frame upon primate evolution. (Figure 6A) Slot-blot of genomic DNA from simian, prosimian, cat and mouse species, probed with a full-length HERV-FRD env fragment. The inferred date of insertion of HERV-FRD is indicated with an arrow on the primate phylogenetic tree on the left. NWM: New World Monkey. (Figure 6B) *In vitro* transcription-translation assay of the human HERV-FRD env gene and of the orthologous genes from the indicated simian species. Env genes were PCR-amplified as schematized, submitted to *in vitro* transcription/translation ('control' is without DNA template), run on a polyacrylamide gel and autoradiographed. The arrow points the bands of the expected size.

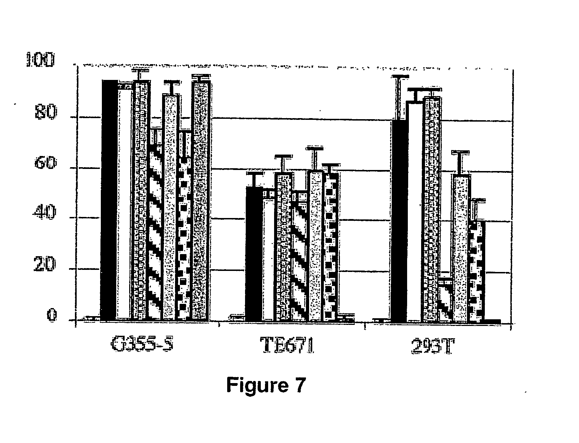

**Figure 7****.** Fusion activity of the orthologous primate ERV-FRD envelope glycoproteins. The indicated target cells were transfected as indicated in Figure 3A with vectors expressing the human or simian orthologous ERV-FRD envelopes, and fusion indices were determined as indicated in Table 2 (means± standard deviations; n = 5).

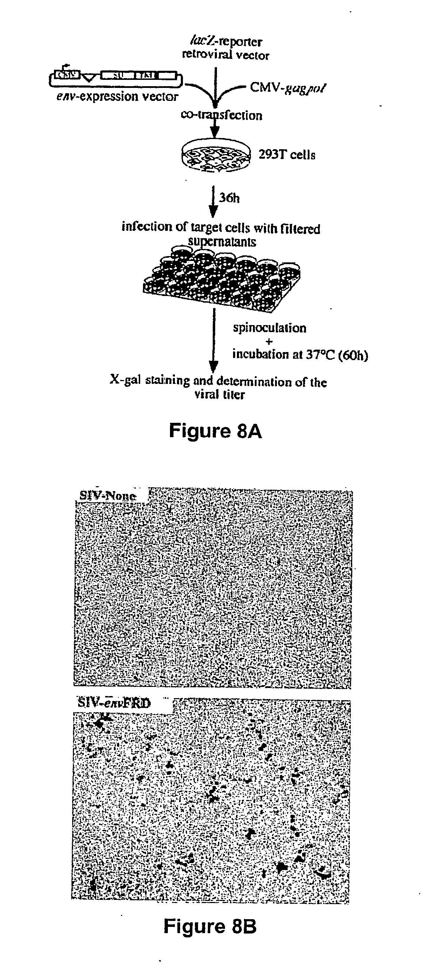

**Figure 8A and Figure 8****B.** Infection assays using retroviral particles pseudotyped with the HERV-FRD and control envelopes. (Figure 8A) Rationale of the assay. To determine whether the HERV-FRD envelope can confer infectivity on MLV, HIV or SIV Env-defective pseudoparticles, corresponding pseudotype viruses were produced by cotransfecting 7.5 x 10⁵ 293T cells with 0.55 µg of the phCMV-HERV-FRD vector expressing the HERV-FRD envelope (or pcDNA3 for the negative -none- control, and phCMVampho for the amphotropic MLV envelope positive control), 1.75 µg of a vector encoding the retroviral proteins (except the envelope) of MLV (phCMVintron) HIV (pCMVΔR8.91) or STV (pSIV3+), and 1.75 µg of the corresponding defective retroviral vectors marked with a ß-galactosidase-reporter gene (pMFGsnls*lacZ*; pHR'CMV*lacZ*; R9SA), using calcium phosphate precipitation (Invitrogen). 36 h post-transfection, viral supernatants were collected and filtered through 0.45-µm-pore-size membranes. Target cells (murine 3T3, feline G355-5, or human TE671, HeLa and 293T cells) were seeded in 24-well plates at a density of 10⁴ cells per well and incubated overnight at 37°C. 5-500 µl of virus samples containing 4 µg of Polybrene per ml were added to the cells and centrifuged for spinoculation at 1,200 x g for 2 h 30 min at 25°C (10). After removal of the supernatants, the cells were incubated in regular medium for 60 h at 37°C. Viral titers were then measured upon by X-Gal staining of the cells and expressed as *lac*Z Colony Forming Unit (CFU) per milliliter of viral supernatant. (Figure 8B) Infectivity of SIV-based retroviral particles pseudotyped with the HERV-FRD envelope (or with no protein in the 'SIV-none' control), as assayed using human 293T cells as target cells and X-gal staining, following the protocole described in (Figure 8A).

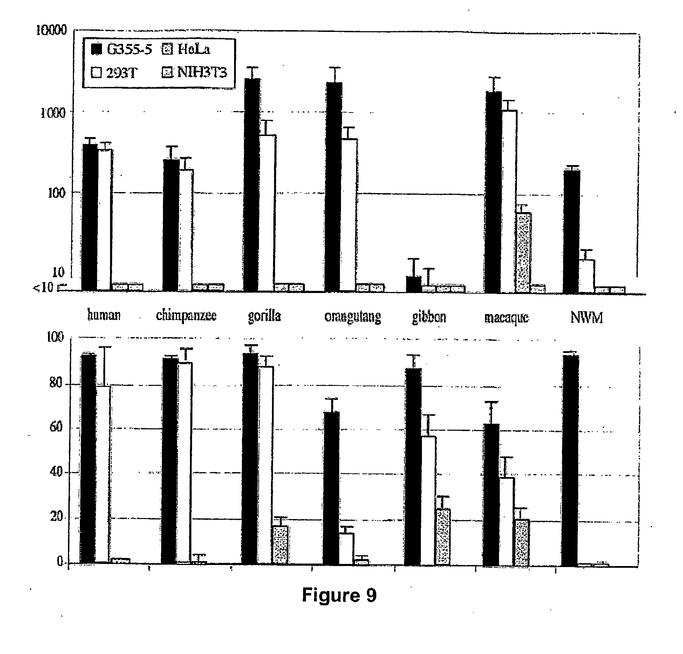

**Figure 9****.** Infection / cell-cell fusion activities and host range of the HERV-FRD and simian orthologous envelopes. Upper panel: Infection of feline (G355.5), human (293T and HeLa) and murine (3T3) cells by SIV-based particles pseudotyped with the indicated ERV-FRD envelopes. Same experimental conditions as in Figure 8A, with negative and positive control values as in Table 1. NWM: New World Monkey *(Callithrix jacchus* marmoset). Lower panel: Cell-cell fusion assay for the ERV-FRD envelopes and cells as in the upper panel. The G355-5 and HeLa cells were transfected using Lipofectamine (Invitrogen, 2 µg of DNA for 5x10⁵ cells), and the 293T and TE671 cells using calcium phosphate precipitation (Invitrogen, 5 µg of DNA for 5x10⁵ cells). Fusion activities were measured 12 to 36 h post-transfection of the corresponding expression vectors. To visualize syncytia, cells were fixed in methanol and stained by adding May-Grünwald and Giemsa solutions (Sigma) according to the manufacturer's instructions. The fusion index, which represents the percentage of fusion events in a cell population is defined as [(N-S)/T] x 100, where N is the number of nuclei in the syncytia, S is the number of syncytia, and T is the total number of nuclei counted.

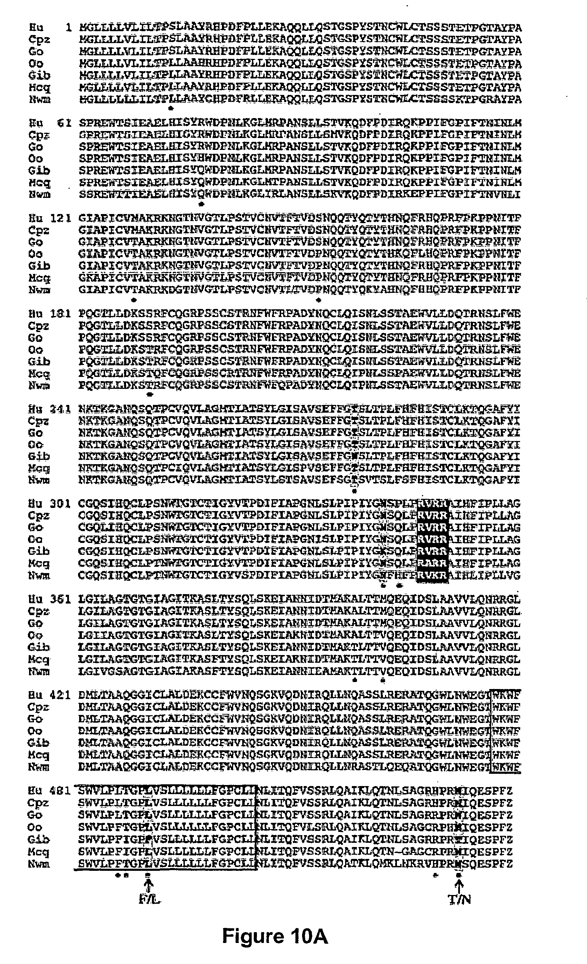

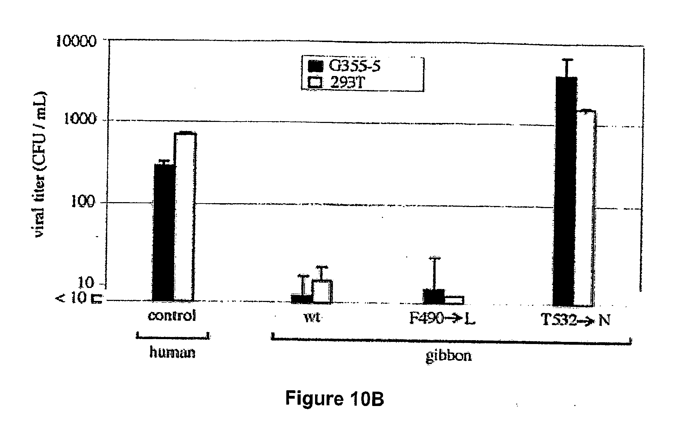

**Figure 10A** **and** **Figure 10****B.** Comparison of the simian ERV-FRD envelopes and identification of a unique point mutation responsible for the gibbon envelope loss-of-function. (Figure 10A) Aligned primary sequences of the HERV-FRD and simian orthologous envelopes. Abbrevations are: Hu:homo sapiens; Cpz: chimpanzee; Go: gorilla; Oo; orangutan; Gib: gibbon; Mcq: macaque; Nwm: New World Monkey. Accession numbers are AL136139 for the human gene, and AJ577595 to AJ577600 for the simian genes. The RVRR canonical motif for SU-TM cleavage is darkened, and the predicted transmembrane hydrophobic domain is boxed. Aminoacids differing between the human and gibbon proteins are indicated with an asterisk, among which those unique to the gibbon sequence are grayed. The two aminoacids in the TM moiety of the gibbon protein that were mutated and assayed for reversion to the infectious phenotype are indicated with an arrow. (Figure 10B) Infection of feline (G355-5) and human (293T) cells by SIV-based particles pseudotyped with the wild type (wt) and mutant (F490->L or T532->N) gibbon FRD envelopes, with the human FRD envelope as a control. The indicated aminoacid changes in the gibbon envelope were performed by a three-fragment ligation, using PCR fragments generated with oligonucleotides containing the gibbon-to-human single-base mutations (TTT→CTT and AAT→ACT for aminoacid positions 490 and 532, respectively), and subsequent sequencing of the resulting constructs. Infection assays were performed as described in Figure 8A. The viral titers are average from two independent experiments.

### Examples

### Example 1

### Survey of human genes of retroviral origin: identification and transcriptome of the genes with coding capacity for complete envelope proteins

### Materials and Methods

### Computer sequence analyses

Horology searches were performed using the BLASTN program at the Rational Center for Biotechnology Information (http://www.ncbi.nlm.nih.gov/BLAST), screening the finished (nr) and unfinished (htgs) databases with consensus envelope genes for each family. Hydropathy of the envelope proteins was calculated by the Kyte-Doolittle method implemented by the DNA Strider program (Marck, C. 1988. DNA Strider': a'C' program for the fast analysis of DNA and protein sequences on the Apple Macintosh family of computers. Nucleic Acids Res. 16:1829-36). Alignment were performed with the CLUSTALW multialignment program at Infobiogen (http:/hvww.infobiogen.fr). Chromosomal localizations of the coding *env* genes were performed at the Ensembl web site (http://www.ensembl.org/Homo sapiens).

### In vitro transcription / translation assay

Amplification of the coding *env* genes for *in vitro* transcription / translation assays was performed with the Expand Long Template PCR System (Roche, Indianapolis, IN). PCRs were carried out for 35 cycles (1 min at 94°C, 30 s at 58°C, 2 min at 68°C), using, ng of BAC DNA. Primers were as follow: envIIT7.3 (GCTAATACGACTCACTATAGGAACAGACCACCATGCACCACAGTATCAACCTTA C) (SEQ ID NO: 5) and fIR2B1 (TTCTGTTTCAGCTACAACTCTGT) (SEQ ID NO: 6) for envH1; envHT7.3 and flRB2 (AGCAATAGTTTGTTAAATTC) (SEQ ID NO: 7) for cnvH2; envHT7.2 (GCTAATACGACTCACTATACTATAACAGACCACCATGAGGGCACCCTCCAATACTT C) (SEQ ID NO: 8) and flRB3 (ACCCCATGTTCTAGTCTTCC) (SEQ ID NO: 9) for envH3; envKT7 (GCTAATACGACTCACTATAGGAACAGACCACCATGGAGATGCAAAGAAAAGCA) (SEQ ID NO: 10) and LTRenvsK (GTGAACAAAGGTCTTTGCATCATAG) (SEQ ID NO: 11) for envK1, envK3, envK5 and envK6; envKT7 and 3'fl51C12 (GAATTAGGCTTTCGGGACTTGAA) (SEQ ID NO: 12) for envK2; envKT7 and KLTR3' (C/TTTAAC/G/AG/AAGCATGCTGC/AC) (SEQ ID NO: 13) for envK4, envTT7.2 (GCTAATACGACTCACTATAGGAACAGACCACCATGTTGGATTCATCACTCCCA) (SEQ ID NO: 14) and envTflanq2 (CTGAAGGGAGTTCCTCCTAGG) (SEQ ID NO: 15) for envT, envWT7 (GCTAATACGACTCACTATAGGAACAGACCACCATGGCCCTCCCTTATCAT) (SEQ ID NO: 16) and envW64flR2 (ACAGCCAAGCAGGTACAG) (SEQ ID NO: 17) for envW, envFRDT7.2 (GCTAATACGACTCACTATAGGAACAGACCACCATGCTCCTGCTGGTTCTCATTC) (SEQ ID NO: 18) and envFRDfl3' (CTGCAGCAGACTCCATCCTTG) (SEQ ID NO: 19) for envFRD, enverv3T7 (GCTAATACGACTCACTATAGGAACAGACCACCATGACTAAAACCCTGTTGTATC A) (SEQ ID NO: 20) and enven3AS (GTTAATACTTAGTTAGGGCC) (SEQ ID NO: 21) for envR, HS89F2T7 (GCTAATACGACTCACTATAGGAACAGACCACCATGGATCCACTACACACGATTG A) (SEQ ID NO: 22) and HS89R3fl (TGTTTTGGGACACCACGAAT) (SEQ ID NO: 23) for envR(b), envF(c)2T7 (GCTAATACGACTCACTATAGGAACAGACCACCATGAATTCTCCATGTGAC) (SEQ ID NO: 24) and envF(c)2flR3 (GACACTTAATAGTTCCGACA) (SEQ ID NO: 25) for envF(c)2, envF(c)1T7 (GCTAATACGACTCACTATAGGAACAGACCACCATGGCCAGACCTTCCCCACTAT GC) (SEQ ID NO: 26) and envF(c)1fl3' (GCCTTGGCAACTAAACCATTC) (SEQ ID NO: 27) for envF(c)1.

T7 promoter-containing PCR products were ethanol precipitated and 200 ng of the amplification products were used in the TNT Coupled Reticulocyte Lysate System (Promega Corp., Madison, WI) according to the manufacturer instructions, with (³H) methionine (ICN Biomedicals Inc., Irvine, CA) for protein labeling. After electrophoresis of the translation products, the SDS-polyacrylamide gels were impregnated in Amplify (Amersham Biosciences, Piscataway, NJ), rinsed with water, dried and autoradiographed.

### Tissue samples, RNA calibration and reverse transcription

Human BAC clones containing the identified coding envelope genes were purchased from BACPAC Resources (Oakland, CA). RNA from various human tissues were purchased from Clontech (Palo Alto, CA), Stratagene (La Jolla, CA) and Ambion (Austin, TX). Quality of the RNA was assessed on a RNA LabChip (Agilent 2100 Bioanalyzer), and RNA concentration was quantified spectrophotometrically. Five micrograms of each RNA sample were submitted to DNAse treatment (DNA-free, Ambion) to eliminate DNA contaminants. One microgram of RNA from each sample was reverse transcribed in a 20 µl volume reaction using 50U Moloney murine leukemia virus Reverse. Transcriptase, 20U ribouclease inhibitor (Applied Biosystems, Foster City, CA), 1 mmol/L dA/T/G/C (Amersham-Pharmacia Biotech, Upsala, Sweden), 5 mmol/L MgCl₂, 10 mmol/L Tris-HCl (pH8.3), 10 mmol/L KCl, and 50 pmol/L of random hexamers (Applied Biosystems). The complementary DNAs were then diluted 1/25 in nuclease-free H₂O (Promega Corp.).

### Real-Time quantitative PCR

Oligonucleotides were designed using the computer program Oligo (MedProbe, Oslo, Norway). The special requirements were a Tm of 60° and an amplicon length comprised between 80 and 350 bp. Oligonucleotides were purchased from MWG (Ebersberg, Germany). Real-Time quantitative PCR was achieved using a complementary DNA equivalent of 20 ng of total RNA. The reaction was performed in 25 µl using SYBR Green PCR Core Reagents (Applied Biosystems, Foster City, CA) according to the manufacturer's instructions. PCR was developed with the ABI PRISM 7000 Sequence Detection System (Applied Biosystems). Amplification was performed using a 2 min. step at 50°C then a 10 min. denaturation step at 95°C, followed by 40 cycles of 15 s denaturation at 95°C, 1 min. primer annealing and polymerization step at 60°C. To normalize for differences in the amount of total RNA added to the reaction, amplification of 185 total ribosomal RNA was performed as an endogenous control (less than a factor of 3.5 variation). Primers and probe from 18S RNA were purchased from Applied Biosystems. The relative expression in each sample was calculated with respect to a standard calibration curve (the dilution series of genomic DNA). Each sample was analyzed at least twice.

The control plasmid (pllenv) containing the 11 envelope amplicons obtained with the primers listed in **Table 3** was constructed by first cloning each amplicon into the pOEMT vector, and then by successive three-fragment ligations using fragments from these 11 plasmids and from construct intermediates. A control series of p11env plasmid dilutions ranging from 1 ng to 0.008 ng was amplified with each primer set to measure their relative yield (less than a factor of 2 variation). A control tube containing 1 ng of the p11env plasmid was included in each Real-Time PCR assay as a reference.

**Table 3 : Sequence of the primers used to detect the expression of the coding envelopes by quantitative RT-PCR analysis**

| Gene | Primers sequence (5'-3') | SEQ ID |
|---|---|---|
| *env*H1 | F:TTCACTCCATCCTTGGCTAT | SEQ ID NO: 28 |
| | R:CGTCGAGTATCTACGAGCAAT | SEQ ID NO: 29 |
| *env*H2 | F : ACTACACACATCACTGAAACAAA | SEQ ID NO : 30 |
| | R:GGATGGAGTGAAATACAGGAC | SEQ ID NO : 31 |
| *env*H3 | F : CCCTCCTCCACATTTATTTG | SEQ ID NO : 32 |
| | R : GTTGGGCTTTGGAGATGG | SEQ ID NO : 33 |
| *env*K | F : CACAACTAAAGAAGCTGACG | SEQ ID NO : 34 |
| | R : CATAGGCCCAGTTGGTATAG | SEQ ID NO : 35 |
| *env*T | F : CCAGGATTTGATGTTGGG | SEQ ID NO : 36 |
| | R : GGGGTGAGGTTAAGGAGATGG | SEQ ID NO : 37 |
| *env*W | F : CCCCATCGTATAGGAGTCTT | SEQ ID NO : 38 |
| | R : CCCCATCAGACATACCAGTT | SEQ ID NO : 39 |
| *env*FRD | F : GCCTGCAAATAGTCTTCTTT | SEQ ID NO : 3 |
| | R : ATAGGGGCTATTCCCATTAG | SEQ ID NO : 4 |
| *env*R | F : CCATGGGAAGCAAGGGAACT | SEQ ID NO: 40 |
| | R : CTTTCCCCAGCGAGCAATAC | SEQ ID NO : 41 |
| *env*R(b) | F : GGACAGTGCCGACATACTAT | SEQ ID NO: 42 |
| | R : TAGAGTGCAGCATCCTAACC | SEQ ID NO : 43 |
| *env*F(c)2 | F : ATGGAGGACTATATGAGCACAA | SEQ ID NO : 44 |
| | R : ATTAAAGTTAACCACGAGAAGC | SEQ ID NO : 45 |
| *env*F(c)1 | F : GGGCCACTAAGTTACTAGGTC | SEQ ID NO : 46 |
| | R : AGTTAGGAGGGAGTTACTGGG | SEQ ID NO : 47 |

### Screening of human databases for coding envelope genes

The rationale of the screening procedure to identify the coding envelopes in the human genome is summarized below.

Briefly, the Repbase database (final control search with Update 8.2.1, Oct. 2002) (Jurka, J. 2000. Repbase update, a database an an electronic journal of repetitive elements. Trends Genet. 16:418-420) in which each family is represented by a « consensus » element, built from an alignment of all the proviruses belonging to the same family (i.e. copies which cluster together in phylogenetic trees) was used. It was implemented by RT- and TM-based searches as in (refs. Benit, L., P. Dessen, and T. Heidmann. 2001. Identification, phylogeny, and evolution of retroviral elements based on their envelope genes. J. Virol. 75:11709-11719 and Tristem, M. 2000. Identification and characterization of novel human endogenous retrovirus families by phylogenetic screening of the human genome mapping project database. J. Virol. 74:3715-3730) which revealed two additional families (namely the F(c)1 and F(c)2 families).

Overall; approximately 100 HERV families were identified. For each family, the envelope gene was tentatively delineated, with the *pol* gene (which was easily positioned due to its high conservation among retroviral elements) at its 5' end, and with the retroviral LTR at its 3' end. When the distance between the *pol* gene and LTR was higher than 1.4 kb (the average length of an envelope gene being 1.9 kb), the envelope gene was considered as potentially complete and the HERV family retained. The resulting list of the 34 corresponding HERV families is given in **Table 1.**

**Table 1: HERV families containing full-length envelope genes**

| Family name¹ | Repbase identifier | Class | Total number of elements² | Number of elements with full-length *env* genes | Number of elements with coding *env* genes |
|---|---|---|---|---|---|
| HERV-H | HERVH | I | 1000 | 43 | 3 |
| HERV-IP | HERVIP10FH | I | 60 | 38 | 0 |
| HERV-9 | HERV9 | I | 1000 | 37 | 0 |
| HERV-K HML-2 | HERVK | II | 50 | 35 | 6 |
| HERV-K HML-8 | HERVK11 | II | 100 | 31 | 0 |
| HERV-K HML-5 | HERV22 | II | 15 | 25 | 0 |
| HERV-E | HERVE | I | 150 | 22 | 0 |
| HERV-T | HERVS71 | I | 100 | 19 | 1 |
| HERV-S | HERV18 | III | 60 | 19 | 0 |
| HERV-Z69907 | MER66 | I | 100 | 18 | 0 |
| HERV-ADP | HERVP71A | I | 50 | 17 | 0 |
| HERV-K HML-6 | HERVK3 | II | 100 | 17 | 0 |
| HERV-P | HUERS-P3 | I | 100 | 13 | 0 |
| HERV-W | HERV17 | I | 100 | 13 | 1 |
| HERV-F | HERVFH19 | I | 50 | 11 | 0 |
| HERV-U3 | HERV-L66 | III | 30 | 10 | 0 |
| HERV-K HML-7 | HERVK11D | II | 20 | 10 | 0 |
| | MER57(A) | I | 150 | 10 | 0 |
| RRHERV-I | HERV15 | I | 30 | 10 | 0 |
| HERV-U2 | PRIMA41 | III | 100 | 10 | 0 |
| HERV-I / FTD | HERVI | I | 50 | 8 | 0 |
| | PRIMA4 | I | 100 | 8 | 0 |
| HERV-FRD | MER50 | I | 100 | 8 | 1 |
| HERV-K HML-10 (C4) | HERVK14 | II | 20 | 7 | 0 |
| HERV-K HML-3 | HERVK9 | II | 150 | 6 | 0 |
| HERV-R / erv3 | HERV3 | I | 100 | 6 | 1 |
| HERV-R(b) | PABL B | I | 10 | 6 | 1 |
| | MER70 | III | 100 | 6 | 0 |
| HERV-XA34 | HERVFH21 | I | 30 | 5 | 0 |
| HERV-F(c)2 | - | 1 | 15 | 5 | 1 |
| HERV-U4 | MER83 | I | 10 | 4 | 0 |
| | HERV30 | I | 40 | 1 | 0 |
| HERV-KHML-4 | HERVK13 | II | 20 | 1 | 0 |
| HERV-F(c)1 | - | I | 2 | 1 | 1 |
| total | | | -4,000 | 476 | 16 |

| | | | | | |
|---|---|---|---|---|---|
| ¹refers to names given in original publications, most of the time following the PBS-related nomenclature (reviewed in Tristem, 2000 and Benit *et al.,* 2001). ²estimates based on the number of hits obtained in blast searches with the consensus provirus against human databases. | | | | | |

For each family, a BLAST query was then performed on human genome databases using the envelope gene as a probe. Overall, it yielded 476 potentially complete envelope genes (see **Table 1** for their distribution among the HERV families). The coding status of each identified *env* gene was finally assessed, and only those with an open reading frame beginning at the first Met codon of the consensus envelope gene and uninterrupted over >90% of this gene were retained. Among the 476 envelope genes that were individually analyzed, only 16 genes were found to potentially encode envelope proteins.

These 16 genes are listed and described in **Table 2** and **Figure 1****.**

**Table 2 : The 16 coding envelope genes of the human genome.**

| Gene name | Chrom. localization | Accession number | Position of the envelope in sequence entry^{a} | Bibliographic name | References |
|---|---|---|---|---|---|
| ***env*H1** | 2q24 3 | AJ289709 | (6313-8067) + | envH/p62, H19 | de Parseval *et al.,* 2001; Lindeskog *et al.,* 1999 |
| *env***H2** | 3q26 | AJ289710 | (5393-7084) + | envH/p60 | de Parseval *et al.,* 2001 |
| *env***H3** | 2q24.1 | AJ289711 | (5204-6871) + | envH/p59 | de Parseval *et al.,* 2001 |
| *env***K1** | 12q14.1 | AC074261 | (93508-95604) - | - | SEQ ID NO: 48 |
| *env***K2** | 7p22.1 | AC072054 | (30365-32464) - | HML-2.HOM, | Mayer *et al.,* 1999; |
| | | | (38869-40968) -^{b} | K(C7), | Tönjes *et al.,* 1999; |
| | | | | HERV-K108 | Barbulescu *et al.,* 1999 |
| *env***K3** | 19q12 | Y17833 | (5581-7680) + | HERV-K(C19) | Tönjes *et al*., 1999 |
| *env***K4** | 6q14.1 | AF164615 | (6412-8508) + | HERV-K109 | Barbulescu *et al*., 1999 |
| *env***K5** | 19p13.11 | AY037928 | (6451-8550) + | HERV-K113 | Turner *et al*., 2001 |
| *env***K6** | gp23.1 | AY037929 | (6442-8541) + | HERV-K115 | Turner *et al.,* 2001 |
| *env***T** | 19p13.11 | AC078899 | (154738-156618) + | | SEQ ID NO: 50 |
| *env***W** | 7q21.2 | AC000064 | (35879-37495) + | syncytin | Blond *et al.,* 1999 |
| *env***FRD** | 6p24.1 | AL136139 | (21355-22972) - | - | SEQ ID NO: 1 |
| *env***R** | 7q11.21 | AC073210 | (54963-56978) - | erv3 | Cohen *et al.,* 1985 |
| *env***R(b)** | 3p24.3 | AC093488 | (78681-80225) + | - | SEQ ID NO: 52 |
| *env***F(c)2** | 7q36.2 | AC016222 | (85216-86963) + | - | SEQ ID NO: 54 |
| *env***F(c)1** | Xq21.33 | AL354685 | (46744-48717) - | - | SEQ ID NO: 56 |

| | | | | | |
|---|---|---|---|---|---|
| ^{a}+ and - designate orientation in the sequence entry ^{b} the HML-2.HOM ! K(C7) / HERV-K108 locus is organized as a tandem repeat in some individuals, with 100% nt identity for two the envelope genes. | | | | | |

Ten of these genes had previously been identified (Barbulescu, M., G. Turner, M. I. Seaman, A. S. Deinard, K. K. Kidd, and J. Lenz. 1999. Many human endogenous retrovirus K (HERV-K) proviruses are unique to humans. Curr. Biol. 9:861-868, Blond, J.-L., F. Besème, L. Duret, O. Bouton, F. Bedin, H. Perfon, B. Mandrand, and P. Mallet. 1999. Molecular characterization and placental expression of HERV-W, a new human endogenous retrovirus family. J. Virol. 73:1175-1185, Cohen, M., M. Powers, C. O'Connell, and N. Kato. 1985. The nucleotide sequence of the env gene from the human provirus erv3 and isolation and characterization of an erv3-specific cDNA. Virology. 147:449-458, de Parseval, N., J. F. Casella, L. Gressin, and T. Heidmann. 2001. Characterization of the three HERV-H proviruses with an open envelope reading frame encompassing the immunosuppressive domain and evolutionary history in primates. Virology. 279:558-569, Lindeskog, M., D. Manger, and J. Blomberg. 1999. Isolation of a human endogenous retroviral HERV-H element with an open env reading frame. Virology. 258:441-450, Mayer, J., M. Sauter, A. Racz, D. Scherer, N. Mueller-Lantzsch, and E. Meese. 1999. An almost-intact human endogenous retrovirus K on human chromosome 7. Nature Genet. 21:257-258, Tönjes, R. R., F. Czaudema, and R. Kurth. 1999. Genome-wide screening, cloning, chromosomal assignment, and expression of full-length human endogenous retrovirus type K. J. Virol. 73:9187-9195, Turner, G., M. Barbulescu, M. Su, M. I. Jensen-Seaman, K. K. Kidd, and J. Lenz. 2001. Insertional polymorphisms of full-length endogenous retroviruses in humans. Curr. Biol. 11:1531-1535), and six new genes emerged from this screen, including genes from the FRD. T, R(b), F(c)1 and F(c)2 families, and one supplementary gene from the HERV-K (HML-2) family. The chromosomal localization was determined or confirmed using the Ensembl web site and the corresponding accession numbers. Noteworthy, one of these genes, namely *env*K2, can be found in duplicate in some individuals since the corresponding provirus is organized as a tandem repeat (Reus, K., J. Mayer, M. Sauter, D. Scherer, N. Muller-Lantzsch, and E. Meese. 2001. Genomic organization of the human endogenous retrovirus HERV-K(HML-2.HOM) (ERVK6) on chromosome 7. Genomics. 72:314-320). The two *env* sequences being 100% identical at the nucleotide level, both will be further referred to as the *env*K22 gene. As can be observed in **Figure 1** which describes the 16 putative envelopes in comparison with the Moloney leukemia virus envelope, the overall length of the proteins is variable, with a minimum of 514 aa for EnvR(b) and maximum of 699 aa for EnvK. This size variability is also found among exogenous retroviral envelopes since, For example, HTLV-1 and HIV-1 envelopes are 488 and 861 aa long, respectively.

To ascertain the existence of the open reading frames inferred from the nucleotide sequences, an *in vitro* transcription-translation assay was performed using human BAC clones containing the identified coding envelope genes. The 16 BACs were obtained from BACPAC Resources (Oakland, CA), except for the H1, H2 and H3-containing BACs that had been cloned previously (de Parseval, N., J. F. Casella, L. Gressin, and T. Heidmann. 2001. Characterization of the three HERV-H proviruses with an open envelope reading frame encompassing the immunosuppressive domain and evolutionary history in primates. Virology. 279:558-569). The *in vitro* transcription-translation assay was performed directly on the env gene PCR products (generated using a forward T7-containing primer at the *env* 5' end and a reverse primer downstream of the stop codon). In all cases, translation products of the size expected from the sequences in the database were obtained (indicated with arrowheads in **Figure 2**). For some envelopes, additional bands of lower molecular weight were observed, compatible with initiation at internal sites, and interestingly in one case (i.e. F(c)2) additional bands at higher molecular weight were detected, most probably associated with the presence at the gene 3' end of a frameshift signal (Benit, L., A. Calteau, and T. Heidmann in press. Characterization of the low copy HERV-Fc family: evidence for recent integrations in primates of elements with coding envelope genes. Virology).

The predicted hydrophobic profiles of the 16 proteins represented in **Figure 1** allow the identification of characteristic domains of these envelopes, namely the fusion peptide, located just downstream of the proteolytic cleavage site between the SUrface (SU) and TransMembrane (TM) moieties of the envelopes, and the transmembrane domain of the TM subunit which permits the anchorage of the envelope in the membrane. Noteworthy, this analysis revealed that one envelope. (EnvR) seems to be devoid of a fusion peptide, and that two envelopes (EnvR and EnvF(c)2) disclose a premature stop codon just upstream of their transmembrane hydrophobic domain. Two other domains are delineated in **Figure 1****,** which are characteristic features of the envelopes belonging to the C-type and D-type retroviruses : the CWLC domain, involved in the interaction between the SU and the TM moieties (Pinter, A., R. Kopelman, Z. Li, S. C. Kayman, and D. A. Sanders. 1997. Localization of the labile disulfide bond between SU and TM of the murine leukemia virus envelope protein complex to a highly conserved CWLC motif in SU that resembles the active-site sequence of thiol-disulfide exchange enzymes. J. Virol. 71:8073-8077), and the CKS17-like immunosuppressive domain (Cianciolo, G. J., T. Copeland, S. Orozlan, and R. Snyderman. 1985. Inhibition of lymphocyte proliferation by a synthetic peptide homologous to retroviral envelope proteins. Science. 230:453-455). Noteworthy, as B-type, lenti-, and spuma-retroviruses envelopes, the 6 EnvK lack these two latter domains.

### Transcriptome of the coding envelope genes : strategy

To get insight into the expression profile of these genes in a quantitative manner, the Inventors devised a Real-Time RT-PCR strategy which uses specific primers designed in such a way that only envelope genes with an open reading frame should be amplified among all the envelope genes of a given family. To do so, for each HERV family comprising a member with a coding elements, *env* nucleotide sequences were aligned with the CLUSTALW program and primers were designed within domains of maximal divergence between the coding copy and the other copies. Primers for Sybr Green amplification were devised with their 3'-enda forced at nucleotide positions with, again, maximum divergence between the coding sequence and the others. For the HERV-K (HML-2) family which contains six coding *env* genes, this strategy could not be applied due to the too high sequence conservation between copies. In this case, specific primers were devised that matched all six coding genes, tentatively excluding most other HERV-K (HML-2) envelope genes. The complete list of devised primers is given in **Table 3**.

To determine whether the resulting primers fulfilled the requirements for both efficiency and specificity, a first series of assays was performed by PCR amplification of human genomic DNA. As expected, in all cases a single band was observed, thus excluding non-specific amplifications or amplifications of elements of unusual size (data not shown). Then, PCR products for each couple of primers were cloned into a pGEM-T vector and at least 6 clones per amplicon were sequenced for each envelope (26 clones for the HERV-K (HML-2) family, see below). In all cases, the 6 clones for a given envelope were identical and unambiguously corresponded to the coding sequence, being different from all the other aligned sequences within each HERV family. For the HERV-K (HML-2) family, 26 clones were sequenced, among which 21 had an identical sequence corresponding to the sequence of the 6 coding envelopes, and 5 which corresponded to two other HERV-K (HML-2) envelope genes. Despite this parasitic amplification of non coding envelopes, the HERV-K (HML-2) coding envelope primer set was retained since it allowed the amplification of at least all the coding envelopes.

A second series of assays was then performed to determine the yield of each couple of primers to normalize *env* expression levels. To do so, a single plasmid (see Materials and Methods) which contained the complete set of 11 amplicons (the 6 *env*K (HML-2) being amplified by the same primer set) was constructed. This plasmid, used as a « control » matrix for each couple of primers, actually allowed a refined normalization for possible differences in the yield of the various primers, and was used in each Real-Time amplification below.

### Transcriptome of the coding Envelope genes : survey of human tissues

A systematic screening of the expression level of the 16 coding *env* genes present in the human genome was achieved with the primers listed above, on a series of 19 healthy human tissues (**Table 4**).

**Table 4: Transcript levels of the 16 coding retroviral envelope genes of the human genome in a panel of 19 healthy human tissues.**

| | *env* H1 | *env* H2 | *env* H3 | *env* K | *env*T | *env*W | *env*FRD | *env*R | *env*R(b) | *env*F(c)2 | *env*F(c)1 |
|---|---|---|---|---|---|---|---|---|---|---|---|
| adrenal | | | | 9.0±0.4 | 1.9±0.1 | 8.6±0.1 | 1.7±0.2x10¹ | 1.1±0.1x10³ | | | |
| bone marrow | | | | 3.9±0.8 | 1.0±0.2 | 7.8±2.0 | 6.4=0.7 | 2.0±0.2x10² | | | |
| brain | | | | 2.1±0.3 | 1.1±0.8 | 7.0±0.1 | 4.8±0.3 | 1.3±0.2x10² | | | |
| breast | | | | 1.1±0.1x10¹ | 8.9±4.1 | 1.5±0.1x10¹ | 8.8±1.0 | 3.8±0.1x10² | | | |
| colon | | | | 2.7±0.1x10¹ | | 1.0±0.1x10¹ | 1.10±0.2x10¹ | 1.6±0.2x10² | | | |
| heart | | | | | | | | 4.4±0.5 | | | |
| kidney | | | | 6.9±0.8x10¹ | 1.3±0.1x10¹ | 1.2±0.1x10¹ | 1.3±0.3x10¹ | 1.4±0.1x10² | | | |
| liver | | | | | | | | 5.7±1.8 | | | |
| lung | | 1.4±0.5 | | | | | 3.7±0.1 | 1.6±0.4x10² | | | |
| ovary | | | | 5.4±0.5 | 2.7±0.5 | 1.0±0.3x10¹ | 8.5±1.3 | 4.4±0.2x10² | | | |
| PBL | | | | 1.4±0.9 | | | 5.8±1.2 | 5.2±0.1x10¹ | | | |
| placenta | | | | 1.1±0.2x10¹ | 2.0±0.8 | 2.7±0. 1x10³ | 1.0±0.4x10³ | 4.3±0.3x10³ | 1.8±0.1 | | |
| prostate | | | | 1.5±0.1x10¹ | 7.9±0.9x10¹ | 6.5±0.1 | 6.8±3.1 | 38±0.6x10² | | | |
| skin | 1.1±0.3 | | | 9.3±2.2 | 4.4±0.2 | 1.3±0.1x10¹ | 2.9±0.3x10¹ | 4.0±0.4x10² | | 5.4±0.4 | 2.5±0.9 |
| spleen | | | | 4.5±0.9 | | 1.0±0.4x10 | 7.0±0.4 | 2.1±0.1x10² | | | |
| testis | 3.5±1.5 | 7.7±3.3 | 3.5±1.1 | 6.5±0.4x10¹ | 2.6±0.3 | 8.2±0.6x10¹ | 3.9±0.9x10¹ | 4.5±0.2x10² | 1.0±0.1 | 2.5±0.9 | 7.2±0.1x10¹ |
| thymus | | | | 4.5±0.7 | | 5.9±0.2 | 2.0±0.1x10¹ | 2.2±0.6x10² | | | |
| thyroid | | | | 5.2±0.2 | 1.0±0.1x10³ | 1.0±0.1x10¹ | 4.3±1.0 | 2.8+0.4x10² | | | |
| trachea | | | | 1.1±0.1x10¹ | 4.4±1.3 | 8.9±2.1 | 7.9±1.3 | 1.2±0.1x10² | | | 2.0±0.4 |

| | | | | | | | | | | | |
|---|---|---|---|---|---|---|---|---|---|---|---|
| The values are determined by real-time quantitative PCR. Each value is expressed as the mean standard deviation relative to the value for a reference control plasmid assayed in each real-time PCR experiment after correction for total RNA content in each tissue extract (using the 18S transcript as an internal control). PBL, peripheral blood lymphocytes. | | | | | | | | | | | |

Briefly, RNA were submitted to DNAse treatment and reverse transcribed using MLV RT, and a first Real-Time quantitative PCR was performed using primers for rRNA 18S to normalize for differences in the amount of total RNA added to the reaction (less than a 3.5-fold variation among samples). Real-Time PCR was then performed for each couple of specific primer. Control PCRs performed on RNA without the RT step never resulted in any amplification, as expected. The expression levels represented in **Table 4** disclose several interesting features.

Firstly, there is an enormous variation in the level of expression (up to 4 log) among the different tissues for a given gene as well as among the different *env* genes. Secondly, and at a more refined level, it appears that all genes are transcribed at a significant level in the testis, still with variations (over a 100-fold) depending on the gene tested. Thirdly, the placenta is the organ where maximal expression can be observed, for the three R, W, and FRD genes, but the other *env* genes are very poorly or even not expressed in this organ. Fourthly, thyroid discloses a specific and high level expression of the *env*T gene, not observed for the other *env* genes in this organ. Fifthly, there is no expression of coding *env* genes in heart and liver, except for *env*R (the lowest level of expression for this gene).

Finally, two groups of coding envelope genes can be inferred from these transcriptional data: the envelope genes which disclose a severe tissue-specificity together with an overall low expression level, namely the 3 *env*H, *env*R(b), *env*F(c)1 and *env*F(c)2, and the envelope genes which are transcribed in the majority of the tissues, namely the 6 *env*K*, env*T, *env*W, *env*FRD and *env*R. This latter gene is singular as its level of expression is extremely high in all tissues tested, with actually the highest value among all coding *env* genes (with the exception of the thyroid, for *env*T)*.*

### A limited number of retroviral coding envelope genes

The extensive survey of the human genome performed here reveals that among the >10,000 retroviral elements clustered into approximately 100 families, only 16 possess a coding retroviral envelope gene (**Table 1** and **Figure 1**). The most important contributor to the coding envelope genes is the HERV-K (HML-2) family, since it comprises 6 coding envelopes out of the 35 full-length envelope copies.

The status of the HERV-K (HML-2) family is unique among the coding env-containing families in several respects. Although elements of this family first entered the primate genome more than 30 million years ago (36), new proviral copies have been generated in the recent past. Actually, the entry date in the human genome of 4 out of the 6 HERV-K (HML-2) proviruses with a coding *env* gene has been estimated to be less than 5 million years ago (ref. 2 for K2, K3, K4, and data not shown for K1). In addition, the latter two proviruses are polymorphic in humans with allele frequencies of 0.04 and 0.19 (ref. 41 for K5 and K6). Furthermore, the 6 HERV-K (HML-2) proviruses with coding *env* have open reading frames in other genes (all 6 have ORFs in their *gag* genes, 4 in their *pro* genes and 4 in their *pol* genes, resulting in three completely coding HERV-K (HML-2) proviruses), and among the 29 other *env* gene-containing proviruses of this family, 10 additional retroviral coding genes can be found.

In comparison with the HERV-K (HML-2) family, the other *env* gene-containing families can be considered as « old families », with no human-specific integrations. The entry date in the primate genome of the coding env gene-containing proviruses vary from 10 million years (for the HERV-Hp62 provirus, (15) to more than 45 million years (for the the coding *env-*containing HERV-R(b) provirus, data not shown). Furthermore, no entirely coding gene besides the env genes have been found so far among those families.

### Transcriptional status of the coding envelope genes

The Inventors have devised an efficient method to detect the expression of specific genes belonging to large multigenic families with high homology between their members. It allowed the quantitative and specific monitoring of the expression level of these endogenous retroviral genes, a task which would be impossible using the Northern blot or classical RT-PCR methods which detect the overall expression of the complete set of elements among each family.

The first important outcome in the observed transcriptional pattern is that all genes are transcribed, at least in the testis. Despite the low (although unambiguous) transcriptional level of some of the *env* genes in this organ (e.g. *env*R(b), envF(c)2 and the three *env*H genes), it indicates that all promoters are active. The fact that testis is an organ in which all coding envelopes are transcribed is not a totally unexpected result since germline expression is a common feature of transposable elements in other species, including mice and drosophila (refs. 17 and 39 for the mouse, reviewed in refs. 11 and 18 for drosophila). In these species, expression in the germ line is associated with a high transpositional activity which may result in stably inherited mutations and most probably plays a role in the generation of genetic diversity in the course of evolution.

For the placenta, expression of the coding env genes is much more heterogeneous than in the testis, with an extremely high expression level detected in this organ for three envelope genes, i. e. for the newly identified *env*FRD gene, and for *env*R and *env*W (for the latter two, high level expression had previously been observed at the protein level, using antibodies, refs. 8 and 42). Conversely, for five coding envelope genes (namely the three *env*H, *env*F(c)2 and *env*F(c)1) expression is undetectable. Several studies on HERV transcription have been performed by Northern blot analyses (reviewed in ref. 25) which disclosed a preferential expression of all HERV families in the placenta (except for the HERV-K family). It was put forward that a release of retroelements expression might take place in this organ, without deleterious consequences for the individual due to the « provisional » status of this organ. The above mentionned results of the invention suggest that such a « generalized » expression is likely not to stem from all HERV members among each family but rather results from the activation of a limited number of proviral copies (possibly non coding), for instance via position effects (e. g. ref. 17).

Finally, the high level expression of the newly identified *env*T detected in the thyroid is intriguing because it is one of the rare coding *env* gene highly expressed in a « permanent » healthy tissue and it is highly expressed only in this organ. This expression might be relevant to the hormone-producing status of the thyroid gland (and this interpretation might similarly hold for *env*R in the adrenal gland) and to specific sequences in the HERV LTR or in the vicinity of the proviral insertion site.

### Biological relevance of env expression in human tissues

Detection of an active transcription of the coding envelope genes in healthy tissues together with the probable positive selective pressure responsible for the conservation of open reading frames throughout million years raises the question of a putative role of these genes in normal cell physiology.

The envelope proteins of exogenous counterparts of HERVs possess several functions besides their primary role as key molecules for viral entry: most of them elicit immunosuppressive effects and some of them have the capacity to create cell-cell fusion. Consequently, the high level expression of three endogenous coding *env* genes observed in the placenta (namely *env*R, *env*W and the newly identified *env*FRD) could be implicated in two major physiological processes of this organ: fusion of the cytotrophoblast cells to form the syncytiotrophoblast layer, and local immunosuppression at the materno-fcetatl barrier.

Along the first line, it has been shown that the *env*W gene product is a highly fusogenic protein (Blond) J. L., D. Lavillette, V. Cheynet, O. Bouton, G. Oriol, S. Chapel-Fernandes, B. Mandrand, F. Mallet, and F.-L. Cosset. 2000. An envelope glycoprotein of the human endogenous retrovirus HERV-W is expressed in the human placenta and fuses cells expressing the type D mammalian retrovirus receptor. J. Virol. 74:3321-3329). Furthermore, inhibition of *env*W expression in primary cultures of human villous cytotrophoblast leads to a decrease in trophoblast fusion and differentiation, suggesting a role in syncytiotrophoblast layer formation (Frendo, J. L., D. Olivier, V. Cheynet, J. L. Blond, O. Bouton, M. Vidaud, M. Rabreau, D. Evain-Brion, and F. Mallet. 2003. Direct Involvement of HERV-W Env Glycoprotein in Human Trophoblast Cell Fusion and Differentiation. Mol. Cell. Biol. 23:3566-3574). Interestingly, the protein encoded by *env*FRD (also highly expressed in the placenta) might have a similar role, as it can as well generate cell-cell fusion (**Example 2**).

In the case of *env*R, on the contrary, it had been previously ruled out its possible implication in fundamental processes of placental formation by the discovery of a premature stop mutation present in 16% of caucasians in the heterozygous state, and in 1% in the homozygous state (de Parseval, N., and T. Heidmann. 1998. Physiological knock-out of the envelope gene of the single copy ERV-3 human endogenous retrovirus in a fraction of the caucasian population. J. Virol. 72:3442-3445).

Another property of retroviral envelopes is to inhibit infection by retroviruses sharing the same receptor - i. e. receptor interference, reviewed in refs. Best, S., P. R. Le Tissier, and J. P. Stoye. 1997. Endogenous retroviruses and the evolution of resistance to retroviral infection. Trends Microbiol. 5:313-318 and Rosenberg, N., and P. Jolicoeur 1997. Retroviral Pathogenesis, p. 475-586. In J. M. Coffin, Hughes, C. L., Varmus, H. E. (ed.), Retroviruses. Cold Spring Harbor Laboratory Press, New York -. It is therefore plausible, though again difficult to demonstrate, that some of the identified endogenous envelopes, as demonstrated in the mouse for the Fv4 locus (Ikeda, H., and H. Sugimura. 1989. Fv-4 resistance gene: a truncated endogenous murine leukemia virus with ecotropic interference properties. J. Virol. 63:5405-5412), protect cells against infections by exogenous retroviruses. Interestingly, it has been demonstrated that EnvW interacts with the type-D mammalian retrovirus receptor (Blond, J. L., D. Lavillette, V. Cheynet, O. Bouton, G. Oriol, S. Chapel-Fernandes, B. Mandrand, F. Mallet, and F.-L. Cosset. 2000. An envelope glycoprotein of the human endogenous retrovirus HERV-W is expressed in the human placenta and fuses cells expressing the type D mammalian retrovirus receptor. J. Virol. 74:3321-3329), and this envelope could therefore play a protective role at the placental barrier.

HBRV-encoded proteins have also been tentatively involved in several human pathologies including cancer, immune disorders and neurological diseases (reviewed in refs. Löwer, R., J. Löwer, and R. Kurth. 1996. The viruses in all of us: characteristics and biological significance of human endogenous retrovirus sequences. Proc. Natl. Acad. Sci. USA- 93:5177-5184 and Nakagawa, K., and L C. Harrison. 1996. The potential roles of endogenous retroviruses in autoimmunity. Immun. Rev. 152:193-236). By analogy with animal models, it is particularly tempting to implicate endogenous retroviral envelopes in tumorigenesis as a result of a transforming (Alberti, A., C. Murgia, S. L. Liu, M. Mura, C. Cousens, M. Sharp, A. D. Miller, and M. Palmarini. 2002. Envelope-induced cell transformation by ovine betaretroviruses. J Virol. 76:5387-94, Palmarini, M., and H. Fan . 2001. Retrovirus-induced ovine pulmonary adenocarcinoma, an animal model for lung cancer. J. Nat. Cancer Inst. 93:1603-1614) or immunosuppressive (Blaise, S., M. Mangeney, and T. Heidmann. 2001. The envelope of Mason-Pfizer monkey virus has immunosuppressive properties. J. Gen. Virol. 82:1597-600, Mangeney, M., N. de Parseval, G. Thomas, and T. Heidmann. 2001. The full-length envelope of an HERV-H human endogenous retrovirus has immunosuppressive properties. J. Gen. Viol. 82:2515-2518, Mangeney, M., and T. Heidmann. 1998. Tumor cells expressing a retroviral envelope escape immune rejection in vivo. Proc. Natl. Acad. Sci. USA. 95:14920-14925) effect. Numerous studies report on expressed endogenous retroviral sequences in pathological tissues, but in most cases the techniques used (Northern blot or RT-PCR using degenerate primers) did not allow the specific detection of coding sequences. Accordingly, the significance of these expressions remains elusive, and an association with diseases is only speculative. With the help of the primers devised in this study, a systematic and quantitative evaluation of the transcription levels of coding envelopes in pathological versus healthy tissues should now be possible.

In Conclusion, the present survey of coding env genes of the human genome provides a comprehensive list of candidate genes for the possible involvement of HERVs in human physiology and physiopathology. Furthermore, it shown herein that all of the identified genes can be expressed at least in some tissues, and the invention provide tools to specifically evaluate their transcriptional status in physiological and pathological conditions. The identified genes should allow further studies of their function via classical genetic approaches (identification of susceptibility loci, search for polymorphisms among the human population as previously performed for the HERV-R locus, ref. de Parseval, N., and T. Heidmann. 1998. Physiological knock-out of the envelope gene of the single copy ERV-3 human endogenous retrovirus in a fraction of the caucasian population. J. Virol. 72:3442-3445), whereas the limited number of unraveled coding sequences downsizes an a priori insoluble multigenic analysis to a simpler one.

### Example 2

### Genome-wide screening for Human Endogenous Retrovirus envelopes with fusogenic properties identifies syncytin-2, a new gene with conserved function upon primate evolution

### Materials and Methods

**Cell lines**. The human TE671 rhabdomyosarcoma cells (ATCC CRL8805), 293T embryonal kidney cells (ATCC CRL11268) and HeLa epithelioid carcinoma cells (ATCC CCL2), the Cos-7 African green monkey kidney cells (ECACC 87021302), the G355-5 feline astrocyte cells (ATCC CRL2033), and the NIH3T3 mouse fibroblasts were grown in Dulbeco's modified Eagle's medium (DMEM) supplemented with 10 % fetal calf serum; the CHO chinese hamster ovary cells (ATCC-CCL61) were grown in F12K nutrient medium (Gibco) supplemented with 7 % fetal calf serum. All cell culture media were supplemented with streptomycin (100 µg / mL) and penicillin (100 U / mL).

**DNAs**. Human BACs were obtained from BAC PAC Resources (Oakland) and from the UK HGMP Resource Centre (Cambridge, UK). The sources of the genomic DNAs are given in (de Parseval, N., Casella, J. F., Gressin, L. & Heidmann, T. (2001) Virology 279, 558-569) and (Benit, L., Lallemand, J.-B., Casella, J.-F., Philippe, H. & Heidmann, T. (1999) J. Virol. 73, 3301-3308) for Human, Chimpanzee (*Pan troglodytes*), Gorilla (*Gorilla gorilla*), Orangutan (*Pongo pygmaeus*), Gibbon (*Hylobates Lar Moloch*), Rhesus macaque (*Macaca cynomolgus*), New world monkeys (*Saguinus midas* and *Cebus capucinus*) and prosimian (*Eulemur fulvus*). The Marmoset new world monkey (*Callithrix jacchus*) DNA was from ECACC (85011419). Mouse (*Mus musculus*) and Cat (*Felis cattus*) DNAs were extracted from spleen tissue samples.

**Envelope expression vectors**. The FBA-RlessSALF expression vector (Lavillette, D., Maurice, M., Roche, C., Russell, S. J., Sitbon, M. & Cosset, F.-L. (1998) J. Virol. 72, 9955-9965) encoding the A-Rless hyperfusogenic mutant amphotropic MLV envelope glycoprotein and the phCMV-G expression plasmid (GenBank accession number AJ318514) were gifts from F-L Cosset (ENS, Lyon, France). The HERV-W envelope expression vector (phCMV-EnvpH74) was a gift from F. Mallet (ENS, Lyon, France). The fifteen other endogenous envelope expression vectors were constructed as follows. Full-length envelope of each provirus was PCR-amplified from the corresponding BAC DNA using a proofreading DNA polymerase and appropriate primers (sequences available upon request). PCR were carried out for only 15 cycles (1 min at 94 °C, 1 min at 60 °C, 4 min at 72 °C), in 50 µl, using 100 ng of BAC DNA, 48 pmol of each primer, 200 µM of each dNTP, 2.5 µl PfuTurbo Hotstart polymerase and 1X *Pfu* reaction buffer (Stratagene). Each PCR product was then cloned into the phCMV-G vector opened with *Eco*RI and blunt-ended by Kleenow treatment except for the HERV- Fc2 envelope, which was cloned into phCMV-G restricted with *Eco*RI and *Bsu*36I and blunt-ended.

**Cell-cell fusion assay**. Cells were transfected using Lipofectamine (Invitrogen, 2 µg of DNA, for 5x10⁵ cells), except for 293T and TE671 cells which were transfected using calcium phosphate precipitation (Invitrogen, 5 µg of DNA for 5x10⁵ cells). Fusion activity of envelope glycoproteins was measured 12 to 36 h after transfection of the corresponding expression vectors. To visualize syncytia, cells were fixed in methanol and stained by adding May-Grünwald and Giemsa solutions (Sigma) according to the manufacturer's instructions. The fusion index, which represents the percentage of fusion events in a cell population is defined as [(N-S)/T] x 100, where N is the number of nuclei in the syncytia, S is the number of syncytia, and T is the total number of nuclei counted.

**Cloning of the HERV-FRD envelope gene from simians**. The envelope genes orthologous to the human HERV-FRD gene were PCR-amplified from simian (and human as a control) genomic DNAs. PCR were carried out for 25 cycles (10 sec at 93 °C, 30 sec at 56 °C, 4 min at 68 °C), in 50 µl, using 100 ng of genomic DNA, 48 pmol of each primer, 350 µM of each dNTP, 0.75 µl Expand long template enzyme mix and 1X reaction buffer (Roche). *Xho*I-containing primers were ATCACCTCGAGCACCATGGGCCTGCTCCTGCTGGTTCTCATTC SEQ ID NO: 58) as forward primer and ATCACCTCGAGGCTTCAGTACAGGTGGATA (SEQ ID NO: 59) as reverse primer. Each PCR product was then *Xho*I restricted and cloned into the phCMV-G vector opened with *Xho*I. Sequencing of the simian envelope genes was performed on the bulk of the PCR products before cloning (MWG-Biotech). Sequences have been deposited in the EMBL nucleotide sequence database under accession numbers AJ577595 to AJ577600 for the chimpanzee, gorilla, orangutang, gibbon, macaque and marmoset envelope, respectively.

***In vitro* transcription-translation.** *In vitro* transcription-translation assays were performed using the Promega TNT coupled reticulocyte lysate system, following the manufacturer's instructions. DNA templates were obtained by PCR with a forward primer designed with a T7 promoter (GCTAATACGACTCACTATAGGAACAGACCACCATGGGTCTGCTCCTGCTG CTTC (SEQ ID NO: 60) for Marmoset and GCTAATACGACTCACTATGGAACAC3ACCACCATGCTCCTG CTGGTTCTCATTC (SEQ ID NO: 61) for all other species) and a reverse primer (ATCACCTCGAGG CTTCAGTACAGGTGGATA (SEQ ID NO: 62) for Marmoset and TTTGAGCAAGGGTGATTCAT (SEQ ID NO: 63) for all other species). [³⁵S] methionine was from ICN. Posttranslational analyses were performed by SDS-polyacrylamide gel electrophoresis, with the gels exposed to X-ray films (Fuji) for 6 h.

**DNA slot-blot**. DNAs (3 µg) from each species were loaded on Hybond N+ membranes (Amersham) using a slot blot apparatus (Hoefer). Blots were hybridized overnight at 65 °C under standard conditions (24), using a ³²P-labelled full-length FRD *env* gene (1679 bp PCR-fragment) as a probe. Membranes were then washed at 65 °C, once with 2X SSC-0.1 % SDS for 15 min and once with 1X SSC-0.1 % SDS for 15 min. Labelling was detected using a PhosphorImager (FLA-3000 scanner).

**Northern-blot analysis and RNA probe**. The full-length FRD *env* gene obtained by PCR as described above was cloned in the antisense orientation under the T7 promoter into the pBlueScript (Stratagene) vector restricted with *Eco*RV. The construct was linearized by *Xho*I digestion and an antisense riboprobe was synthesized using the Strip-EZ RNA probe system from Ambion. A human northern blot (first choice poly(A)⁺ Human northern blot 2, Ambion) was used according to the manufacturer instructions, with prehybridazation at 68 °C in the UltraHyb buffer for 1 h, riboprobe addition and overnight hybridization at 68 °C. The membrane was washed at 68 °C, twice with the NorthernMax low stringency buffer and once with the NorthernMax high stringency buffer (Ambion), and then exposed to X-ray film (Kodak) at -80 °C for 12h.

### Rationale of the assay for human endogenous retrovirus envelopes with fusogenic properties

An assay for fusogenicity of the sixteen cloned envelopes genes identified in **Example 1** was performed. This assay relies on the transfection of cells expected to carry a receptor for the corresponding envelope, with the expression vectors for the envelope proteins. Accordingly, fusion assays were performed for all sixteen envelope genes upon transfection of a large series of cells, including feline, murine, simian and human cells. In the latter case, different cell lines were used, namely Hela cells, 293T embryonic kidney cells, and TE671 cells which have been previously used as they possess the receptors for several retrovirus groups (Porter, C. D., Collins, M. K., Tailor, C. S., Parkar, M. H., Cosset, F. -L., Weiss, R. A. & Takeuchi, Y. (1996) Hum. Gene. Ther. 7, 913-919). In a standard assay 5x10⁵ cells were transfected with 2-5 µg of the Env-expression vector, and fusion was tested by screening the transfected cell culture for multinucleated giant cells -or syneytia- 12-36 h after transfection. Controls included the highly fusogenic A-Rless envelope from the amphotropic MLV (Rein, A., Mirro, J., Haynes, J. G., Ernst, S. M. & Nagashima, K. (1994) J. Virol. 68, 1773-1781), and an 'empty' vector.

### Identification of a new fusogenic HERV envelope

As illustrated in **Figure 3** and **Table 5**, among all the coding env genes, only two induced syncytia formation, namely the HERV-W and the HERV-FRD genes, the other genes resulting in no effect. The result obtained with HERV-W is confirmatory, as it had been previously demonstrated in (Blond, J. L., Lavillette, D., Cheynet, V., Bouton, O., Oriol, G., Chapel-Fernandes, S., Mandrand, B., Mullet, F. & Cosset, F.-L. (2000) J. Virol. 74, 3321-3329, Mi, S.. Lee, X., Li, X., Veldman, G. M., Finnerty, H., Racie, L., Lavallie, E., Tang.,X. Y., Edouard, P., Howes, S., Keith, J. C. J. & McCoy, J. M. (2000) Nature 403, 785-788). Yet, an original outcome of the present invetion is the sorting out of a new envelope gene with fusogenic properties -the HERV-FRD env gene- and the evidence that these two genes most probably constitute the sole reservoir of fusogenic human endogenous retroviral envelopes.

**Table 5. Assay for fusogenicity of the 16 coding endogenous envelopes genes of the human genome.**

| Gene name | Chrom. localization | Accession number | fusion assay^{a.} |
|---|---|---|---|
| **envH1** | 2q24.3 | AJ289709 | - |
| **envH2** | 3q26 | AJ289710 | - |
| **envH3** | 2q24.1 | AJ289711 | - |
| **envK1** | 12q14.1 | AC074261 | - |
| **envK2** | 7p22.1 | AF164614 | - |
| **envK3** | 19q12 | Y17833 | - |
| **envK4** | 6q14.1 | AF164615 | - |
| **envK5** | 19p13.11 | AY037928 | - |
| **envK6** | 8p23.1 | AY037929 | - |
| **envT** | 19p13.11 | AC078899 | - |
| **envW** | 7q21.2 | AC000064 | + |
| **envFRD** | 6p24.1 | AL136139 | + |
| **envR** | 7q11.21 | AC073210 | - |
| **envR(b)** | 3p24.3 | AC018389 | - |
| **envF(c)2** | 7q36.2 | AC016222 | - |
| **envF(c)1** | Xq21.33 | AL354685 | - |

| | | | |
|---|---|---|---|
| ^{a} Fusion assay was performed with the following target cells: NIH3T3, CHO, G355-5, Cos-7, TE671, 293T and HeLa. (-) indicates a negative result in all cell types ; (+) indicates a positive result in at least one cell type. | | | |

The properties of the HERV-FRD *env* gene was analyzed further and compared to that of HERV-W.

Firstly, as illustrated in **Table 6**, it can be observed that the syncytia-forming activity of both envelopes, although of quite related extent when using the syncytia forming index defined in Materials and Methods, do not coincide for a given cell-type: for instance, the HERV-FRD Env is highly fusogenic in feline cells where that of HERV-W has no activity, and within human cells where the HERV-W Env is fusogenic, that of HERV-FRD is not fusogenic in all cell types, with only limited effect in Hela cells. Such differences can be simply accounted for by taking into consideration that retroviral envelope-mediated cell fusion is dependent on the presence of an appropriate receptor on the cell surface, which may be different for different envelope proteins, as currently observed for most exogenous infectious animal retroviruses (reviewed in ref. Hunter, E. (1997) in Retroviruses, eds. Coffin, J. M., Hughes, S. H. & Varmus, H. E. (Cold Spring Harbor Laboratory Press, New York), pp. 71-119 and Overbaugh, J., Miller, A. D. & Eiden, M. V. (2001) Microbiol Mol. Biol. Rev. 65, 371-389). In this respect, it has been previously demonstrated that the receptor for the HERV-W envelope protein is the receptor for the D-type retroviruses (Blond, J. L., Lavillette, D., Cheynet, V., Bouton, O., Oriol, G., Chapel-Feimandes, S., Mandrand, B., Mallet, F. & Cosset, F.-L. (2000) J. Virol. 74, 3321-3329; Lavillette, D., Marin, M., Ruggieri, A., Mallet, F., Cosset, F. L. & Kabat, D. (2002) J. Virol. 76, 6442-6452). Clearly, the receptor for the HERV-FRD envelope is distinct, as the cell specificity for fusion for both envelopes is different. This was further confirmed by performing an interference assay using the panel of cells derived in (Blond, J. L., Lavillette, D., Cheynet, V., Bouton, O., Oriol, G., Chapel-Fernandes, S., Mandrand, B., Mallet, F. & Cosset, F.-L. (2000) J. Virol. 74, 3321-3329), where no decrease in the fusogenic activity of the HERV-FRD Env could be detected, under conditions where the cells had been stably transfected with an expression vector for the envelope of a type-D retrovirus (RD114, not shown).

**Table 6. Fusion host range of the HERV-FRD envelope**

| Species | Target cells | Fusion index ^{a} | | | |
|---|---|---|---|---|---|
| | | HERV-FRD | HERV-W | A-Rless | none |
| Mouse | NIH3T3 | 1.0 ± 0.9 | 0.2 ± 0.5 | 41 ± 17 | 0.4 ± 0.3 |
| Hamster | CHO | 1.8 ± 0.6 | 1.3 ± 0.8 | 1.8 ± 0.4 | 1.0 ± 0.5 |
| Cat | G355-5 | 90 ± 2 | 2.2 ± 3.2 | 83 ± 3 | 0.7 ± 0.8 |
| Monkey | Cos-7 | 20 ± 4 | 62 ± 3 | 19 ± 4 | 13 ± 3 |
| Human | TE671 | 66 ± 6 | 56 ± 10 | 39 ± 8 | 3.1 ± 1.8 |
| | 293T | 81 ± 7 | 90 ± 4 | 9 ± 3 | 0.3 ± 0.6 |
| | HeLa | 1.8 ± 0.7 | 65 ± 9 | 23 ± 6 | 1.0 ± 0.4 |

| | | | | | |
|---|---|---|---|---|---|
| ^{a}Target cells were transfected with expression vectors for the HERV-FRD envelopes, the HERV-W envelope, the hyperfusogenic mutant amphotropic MLV envelope (A-Rless) or no protein (none) as a negative control, and fusion indices were determined as indicated in Materials and Methods (means ± standard deviations; n=5). | | | | | |

Consistent with its fusogenic property, analysis of the aminoacid sequence of the FRD envelope together with its hydrophobic profile (**Figure 4**) discloses the characteristic features of retroviral envelopes (reviewed in Hunter, E. (1997) in Retroviruses, eds. Coffin, J. M., Hughes, S. H. & Varmus, H. E. (Cold Spring Harbor Laboratory Press, New York), pp. 71-119), with a canonical cleavage site (consensus: R/K-X-R/K-R; (Coffin, J. M. (1986) Cell 46, 1-4) between the SUrface (SU) and TransMembrane (TM) moieties of the protein, and the presence of hydrophobic domains corresponding to the fusion peptide and the TM domain. A `CWLC' domain, involved in the interaction between the SU and TM moities in retroviral envelopes (Pinter, A., Kopelman, R., Li, Z., Kayman, S. C. & Sanders, D. A. (1997) J. Virol. 71, 8073-8077), and an 'immunosuppressive' domain (Cianciolo, G. J., Copeland, T., Orozlan, S. & Snyderman, R. (1985) Science 230, 453-455) can also be identified in the SU and TM moieties, respectively.

Finally, a Northern blot analysis was performed with a membrane containing polyA⁺ RNA from a panel of human tissues that were hybridized with a riboprobe for the HERV-FRD envelope. As can be observed in **Figure 5****,** a strong band is observed in the placenta, and actually almost exclusively in this organ, at the expected position for a spliced subgenomic retroviral transcript. A real-time RT-PCR analysis of the HERV-FRD *env* gene transcripts further disclosed high level expression in cytotrophoblast cells isolated from the placenta, not observed in the corresponding fibroblast cells.

### Conservation of the FRD locus and fusogenicity upon primate evolution

The FRD family of endogenous retroviruses consists of approximately 50 provirus copies per haploid genome (Tristem, M. (2000) J. Virol. 74, 3715-3730; Seifarth, W., Skladny, H., Krieg-Schneider, F., Reichert, A., Hehlmann, R. & Leib-Mosch, C. (1995) J. Viro/. 69, 6408-6416). This family is most probably a very ancient family whose members entered the primate genome after the divergence between prosimians and simians: this is demonstrated in the zoo-slot in **Figure 6A**, where hybridization can be detected using an envelope probe and genomic DNAs from humans to New World Monkeys, with no signal for prosimians nor rodents (nor other mammals, not shown). Taking into consideration that conservation of the HERV-FRD envelope gene with an open reading frame might not be fortuitous but rather be the result of positive selection for a still unknown function, the status of the orthologous locus in the course of primate evolution was analyzed using a PCR. approach with a 'flanking' primer located 3' to the proviral insertion, and an 'internal' proviral primer 5' to the *env* gene. Consistent with the zoo-blot analysis, PCR amplification was found positive for all simians, resulting in DNA fragments of the expected size.

The identified putative envelope genes were characterized further, along three lines: firstly, the PCR products were sequenced for an unambiguous identification of the *env* genes, and the determination of their level of divergence in the course of primate evolution; secondly, an *in vitro* transcription/translation assay was performed to determine whether the genes are fully coding; and thirdly a fusion assay was performed as above, to determine if *env* gene function has been reserved.

As illustrated in **Table 7,** sequencing of the PCR products disclosed very high sequence conservation among the primate ERV-FRD *env* genes, with values in the 95-100 % range from human to macaque, and still 88 % identity between human and New World Monkey, which have diverged at least 40 millions years ago. No stop codon could be found in the sequences, a result consistent with the *in vitro* transcription/translation assay performed directly on the PCR products (**Figure 6B**), which demonstrates full-length products for all genes.

**Table 7. Percentage identity of amino-acid sequences of envFRD in simians^{a}**

| | Human | Chimpanzee | Gorilla | Orangutang | Gibbon | Macaque | NWM |
|---|---|---|---|---|---|---|---|
| Human | _ | | | | | | |
| Chimpanzee | 99.6 | _ | | | | | |
| Gorilla | 99.1 | 99.1 | - | | | | |
| Orangutang | 96.7 | 96.7 | 96.8 | - | | | |
| Gibbon | 97.2 | 97.2 | 97.4 | 97.0 | - | | |
| Macaque | 95.2 | 95.2 | 95.4 | 95.7 | 96.5 | _ | |
| NWM | 88.1 | 88.1 | 88.0 | 87.9 | 88.5 | 87.9 | - |

| | | | | | | | |
|---|---|---|---|---|---|---|---|
| ^{a}Orthologous *env*FRD genes were PCR-amplitied and sequenced. Alignments were performed with the LALIGN program. Sequences are deposited in the EMBL nucleotide sequence database (see Materials and Methods). | | | | | | | |

To assay for the possible conservation of the fusogenic property of the identified genes, the PCR *env* fragments were cloned into the phCMV expression vector and tested in the syncytia-forming assay, as above. For each *env* gene, at least six clones were assayed to circumvent possible mutations introduced by the PCR amplification procedure (a feature with no consequence on both the sequencing and the transcription/translation assay above, directly performed on the bulk of the PCR product), using both the highly fusogenic feline G355.5 cells, and human cells (see **Figure 7**). Remarkably, as illustrated in the figure, fusion of the transduced feline cells could be observed for all *env* genes (with in each case at least four out of six cloned PCR-fragments positive, a result consistent with the expected rate of mutation of the DNA polymerase used for the corresponding genomic amplifications), with no significant difference in the extent of fusogenicity from human to New World Monkey.

A similar conclusion holds using human cells for the fusion assay, as expected, but with an exception for the most distantly related *env* gene (the New World Monkey gene), which is not positive in human cells although clearly fusogenic when assayed in feline cells.

This unexpected result is most probably relevant to subtle differences in the envelope aminoacid sequence which, together with expected differences among the human and feline receptors, result in the impairment of a productive interaction with the former. Such differences might be of interest for a future identification of the aminoacids involved in the fusion process.

The present investigation has unraveled a new fusogenic protein of retroviral origin that is encoded in primate genomes. This envelope protein promotes cell-cell fusion in a specific manner as it depends on the cell type used, with positive effects in both feline and human cells. Interestingly, analysis of the cell types prone to fusion discloses differences with the other fusogenic envelope identified so far, i.e. the HERV-W envelope (Blond, J. L., Lavillette, D., Cheynet, V., Bouton, O. Oriol, G., Chapel-Fernandes, S., Mandrand, B., Mallet, F. & Cosset, F.-L. (2000) J. Virol. 74, 3321-3329; Mi, S., Lee, X., Li, X., Veldman, G. M., Finnerty, H., Racic, L., LaVallie, E., Tang, X. Y., Edouard, P., Howes, S., Keith, J. C. J. & McCoy, J. M. (2000) Nature 403, 785-788), indicating differences in receptor usage. In the case of the HERV-W envelope, interference assays had led to the identification of the corresponding receptor as being that for D-type retroviruses (Blond, J. L., Lavillette, D., Cheynet, V., Bouton, O., Oriol, G., Chapel-Fernandes, S., Mandrand, B., Mallet, F. & Cosset, F.-L. (2000) J. Virol. 74, 3321-3329; Lavillette, D., Marlin, M., Ruggieri, A., Mallet, F., Cosset, F. L. & Kabat, D. (2002) J. Virol. 76, 6442-6452). Similar assays demonstrate that this receptor is not that for the HERV-FRD envelope, and preliminary experiments suggest that it is none of the identified receptors for the present-day infectious retroviruses. Specific search for the HERV-FRD receptor will therefore be necessary to identify the cell membrane protein involved in the observed cell-cell fusion. Following the proposed nomenclature for the HERV-W envelope gene, that was named syncytin in relation with its ability to make syncytia via cell-cell fusion (Mi, S., Lee, X., Li, X., Veldman, G. M., Finnerty, H., Racie, L., LaVallie, E., Tang, X. Y., Edouard, P., Howes, S., Keith, J. C. J. & McCoy, J. M. (2000) Nature 403, 785-788), it seems appropriate to name the fully coding HERV-FRD envelope gene the syncytih-2 gene, and accordingly to rename the syncytin gene as syncytin-1 in future studies.

An important issue of the present investigation is the discovery that the identified envelope gene has been conserved in primate evolution in a functional state over >40 millions years, as the corresponding locus is present from New World Monkeys to humans as a full-length coding gene, for which the Inventors further demonstrate that the fusogenic function is conserved in all primates tested. Conservation of the fusogenic function is a strong hint for a functional role of the gene in the physiology of the host, and suggests a selective process by which the retroviral gene function has been diverted, by the host to its own benefit. Similar diversions have already been described for 'parasitic' genes, with examples of retroviral promoters which confer novel tissue specificities to nearby cellular genes (Wilkinson, D. A., Mager, D. L. & Leong, J.-A. C. (1994) in The Retroviridae, ed. Levy, J. A. (Plenum Press, New York), Vol. 3, pp. 465-535; Samuelson, L. C., Philips, R. S. & Swanberg, L. J. (1996) Mol. Biol. Evol. 13, 767-779; Schulte, A. M., Lai, S., Kurtz, A., Czubayko, F. & Regal, A. T. (1996) Proc. Natl. Acad. Sci. USA 93, 14759-14764), and examples of coding sequences of retroviral origin which, in the mouse, confer resistance to infections by exogenous retroviruses (reviewed in ref. Best, S., Le Tissier, P. R. & Stoye, J. P. (1997) Trends Microbiol 5, 313-318).

Among the numerous functions that have been suggested for a possible physiological role of HERV envelopes, the fusogenic function has been frequently hypothesized as being plausibly involved in placenta formation, via the fusion of the cytotrophoblast cells into the syncytiotrophoblast (Blond, J. L., Lavillette, D., Cheynet, V., Bouton, O., Oriol, G., Chapel-Fernandes, S., Mandrand, B., Mallet, F. & Cosset, F.-L. (2000) J. Virol. 74, 3321-3329; Mi, S., Lee, X., Li, X., Veldman, G. M., Finnerty, H., Racie, L., LaVallie, E., Tang, X. Y., Edouard, P., Howes, S., Keith, J. C. J. & McCoy, J. M. (2000) Nature 403, 785-788; Frendo. J. -L., Olivier, D., Cheynet, V., Blond, J. -L., Bouton, O., Vidaud, M., Rabreau, M., Evain-brion, D. & Mallet, F. (2003) Mol. Cell. Biol. 23, 3566-3574). This hypothesis is Consistent with the identified fusogenic activities of the HERV-W and -FRD envelope genes and their observed expression in the placenta -and cytotrophoblast cells. A possible approach to assay the model in humans would be a genetic one, with the finding of possible 'natural' mutants among the human population that could be associated with a pathological situation

Indeed, it had previously been shown, for the first envelope gene that had been unambiguously demonstrated to be highly expressed in the placenta - namely the HERV-R env gene- that it could not be involved in placenta formation, due to the occurrence of a severe polymorphism (a premature stop codon) found in 10 % of caucasians in the heterozygote state, and in 1% in the homozygote state, and not resulting in any placenta-related phenotype (de Parseval, N. & Heidmann, T. (1998) J. Virol. 72, 3442-3445). It was thereafter argued that this defect could be complemented by other genes with the same function, an interpretation which indeed could not be rejected. In fact, as a consequence of its exhaustivity, the present invention now provides the required genetic tools to unambiguously address the issue: there are two identified HERV envelope genes with fusogenic properties in the human genome, and these two genes are expressed in the placenta. A concomitant search for polymorphisms of these two genes among the human population, and in a more refined manner among groups disclosing abnormalities in placenta formation such as pre-eclampsia, Down's syndrome, or uncontrolled trophoblast invasion as observed in choriocarcinoma (Fox, H. (1997) in Pathology of the placenta, ed. Fox, H. (W. B. Saunders, Philadelphia), pp. 151-175; Frendo, J. L., Vidaud, M., Guibourdenche, J., Luton, D., Muller, F., Bellet, D., Giovagrandi, Y., Tarrade, A., Porquet, D., Blot, P. & Evain-Brion, D. (2000) J. Clin. Endocrinol. Metab. 85, 3700-3707; Baergen, R. N. (1997) Gen. Diagn. Pathol. 143, 127-141) should now provide hints for the possible involvement of these genes in placenta physiology.

Finally, it is noteworthy that the identified HERV-FRD envelope gene carries a domain -the immunosuppressive domain- which could play a physiological role in protecting the fetus against the maternal immune system, and as such have also participated in the 'positive selection' of the syncytin-2 gene in the course of primate evolution.

### Example 3

### Identification on an envelope protein from the FRD family of Human Endogenous Retroviruses (HERV-FRD) conferring infectivity and functional conservation among simians

It had previously been demonstrated that the HERV-W envelope gene is functional, since pseudotypes generated with HIV-1 virions are infectious (An, D. S., Y.-M. Xie, and I. S. Y. Chen. 2001. Envelope gene of the human endogenous retrovirus HERV-W encodes a functional retrovirus envelope. J. Virol. 75:3488-3489; Lavillette, D., M. Marin, A. Ruggieri. F. Mallet, F. L. Cosset, and D. Kabat. 2002. The envelope glycoprotein of human endogenous retrovirus type W uses a divergent family of amino acid transporters/cell surface receptors. J. Virol. 76:6442-6452).

In this Example, it is shown that HERV-FRD envelope can also confer infectivity to pseudotypes generated with lentiviral virions.

To do so, the HERV-FRD envelope gene was cloned into a hCMV promoter-driven expression vector to be used in a previously described infection assay (Lavillette, D., M. Marin, A. Ruggieri, F. Mallet, of L. Cosset, and D. Kabat. 2002. The envelope glycoprotein of human endogenous retrovirus type W uses a divergent family of amino acid transporters/cell surface receptors. J. Virol. 76:6442-6452), as schematized in **Figure 8A**.

Briefly, human 293T cells are co-transfected with an expression vector for the retroviral proteins -except the envelope- from type-C (MLV) or lenti (HIV-1 and SIV) viruses, a corresponding *lacZ* gene-marked defective retroviral vector, and an expression vector for the *env* gene to be tested (or an empty vector as a negative control and a vector for the amphotropic MLV envelope as a positive control). Then, the pseudotype virions are assayed for infectivity after recovery of the transfected cell supernatant 36 h post-transfection, transfer of the supernatant onto target-test cells and centrifugation of the plates (i.e. spinoculation, see Ref. Lavillette, D., M. Marin, A. Rugeieri, F. Mallet, F. L. Cosset, and D. Kabat. 2002. The envelope glycoprotein of human endogenous retrovirus type W uses a divergent family of amino acid transporters/cell surface receptors. J. Virol. 76:6442-6452) and, after an additional 60 h period, counting the *lacZ* positive test cell colonies by an *in situ* histochemical staining for ß-galactosidase activity. The results of such an assay are given in **Figure 8B** and **Table 8**, for the various viral cores (MLV, SIV and HIV-1) and target cells tested, which included murine, feline, and human cells. It can be clearly observed that the HERV-FRD envelope, when expressed on lentiviral particles, confers infectivity to the virions, with evidence for viral titers on feline (G355-5) and human (TE671 and 293T) cells in the 100-500 cfu per ml range. Interestingly, the murine 3T3 cells and the human HeLa cells are refractory to infection (although they are positive for the control amphotropic MLV envelope), a result consistent with the absence of any detectable syncytia formation in a fusion assay carried out with these cells (see below). It can also be noted that the MLV core is much less efficient -if any- than the lentiviral cores, as similarly observed for the HERV-W envelope (An, D. S., Y.-M. Xie, and I. S. Y. Chen. 2001. Envelope gene of the human endogenous retrovirus HERV-W encodes a functional retrovirus envelope. J. Virol. 75:3488-3489; Blond. J.-L., D. Lavillette, V. Cheynet, O. Bouton, G. Oriol, S. Chapel-Fernandes, B. Mandrand, F. Mallet, and F.-L. Cosset. 2000. An envelope glycoprotein of the human endogenous retrovirus HERV-W is expressed in the human placenta and fuses cells expressing the type D mammalian retrovirus receptor. J. Virol. 74:3321-3329; Lavillette, D., M. Marin, A. Ruggieri, F. Mallet, F. L. Cosset, and D. Kabat. 2002. The envelope glycoprotein of human endogenous retrovirus type W uses a divergent family of amino acid transporters/cell surface receptors. J. Virol. 76:6442-6452). Finally, since some envelopes are sensitive to shearing, viral titers were also measured in the absence of the spinoculation step: in all cases, its omission (not shown) resulted in a 5-20 fold reduction of the titers in **Table 8** (and similarly for the simian FRD envelopes assayed in **Figure 9**).

In view of the results of **Examples 1 and 2**, the Inventors assayed whether the simian orthologous env genes also confer infectivity to SIV-pseudotype virions. The results of an experiment similar to that carried out in **Figure 8A** for the human gene are given in **Figure 9**, which discloses the following features: i) all simian ERV-FRD envelopes (with one exception, see below) confer infectivity to the pseudotype virions, with closely related viral titers (up to 2000 cfu/ml) and target cell specificity, thus demonstrating conservation over >40 millions years of the ERV-FRD envelope function, but ii) there is one exception to this rule for the gibbon envelope which is negative for all the target cells tested. Interestingly, this negative result is not due to a severe defect in the gibbon envelope, since the protein is still capable of promoting cell-cell fusion, as illustrated in the lower panel of the Figure by an experiment carried out in parallel using the same expression vectors and cells : the results show that for all simian envelopes (except the gibbon envelope) there is a good correlation, as expected, between infectivity and cell-cell fusion, and that the gibbon envelope is clearly positive for cell-cell fusion, with the same cell specificity as the other simian envelopes. These features, which *de facto* uncouple the processes of cell-cell fusion and viral entry, could be accounted for by either intrinsic differences in the molecular requirements for the two processes, for by differences, possibly, in
the envelope viral load and/or stability (see discussion in Refs. Aguilar, H. C., W. F. Anderson, and P. M. Cannon. 2003. Cytoplasmic tail of Moloney murine leukemia virus envelope protein influences the conformation of the extracellular domain: implications for mechanism of action of the R peptide. J. Virol. 77:1281-1291; Lavillette, D., M. Marin, A. Ruggieri, F. Mallet, F, L. Cosset, and D. Kabat. 2002. The envelope glycoprotein of human endogenous retrovirus type W uses a divergent family of amino acid transporters/cell surface receptors. J. Virol. 76:6442-6452; Lavillette, D., M. Maurice, C. Roche, S. J. Russell, M. Sitbon, and F.-L. Cosset. 1998. A proline-rich motif downstream of the receptor binding domain modulates conformation and fusogenicity of murine retroviral envelopes. J. Virol. 72:9955-9965). At the sequence level, it is noteworthy that the gibbon envelope discloses strong conservation with the other simian FRD envelopes, as it is 97 % identical to the human sequence, i.e. it is closer than the infection-positive marmoset New World Monkey envelope which is only 88 % identical to the human sequence (**Figure 10A**). Actually, comparison of the gibbon and human sequences reveals only four mutations which are gibbon-specific, two within the SU and two within the TM subunits (grayed in **Figure 10A**). Preliminary experiments using a chimeric construct with the human SU domain fused to the gibbon TM moiety strongly suggested that mutations in the TM subunit were responsible for the loss of infectivity of the gibbon envelope, as infectivity of the chimeric envelope remained low (not shown). Two constructs with reversion of the identified TM point mutations were therefore tested for infectivity, one with the F to L reversion at position 490 and one with the T to N reversion at position 532. As illustrated in **Figure 10B**, the T to N reversion fully restored infectivity to the gibbon envelope, with no effect of the F to L mutation -located in the hydrophobic transmembrane domain of the TM subunit. Due to its specific location in the cytoplasmic tail of the envelope protein, it is likely that the N to T mutation alters some of the functions and interactions associated, in infectious retroviruses, with this specific domain and involved in the loading, stability, and conformational transition of the Env proteins (e.g. Brody, B. A., S. S. Rhee, and E. Hunter. 1994. Postassembly cleavage of a retroviral glycoprotein cytoplasmic domain removes a necessary incorporation signal and activates fusion activity. J. Virol. 68:4620-4627, Rein, A., J. Mirro, J. G. Haynes, S. M. Ernst, and K. Nagashima. 1994. Function of the cytoplasmic domain of a retroviral transmembrane protein: p15E-p2E cleavage activates the membrane fusion capability of the murine leukemia virus Env protein. J. Virol. 68:1773-1181, Yu, X., X. Yuan, M. F. McLane, T. H. Lee, and M. Essex. 1993. Mutations in the cytoplasmic domain of human immunodeficiency virus type 1 transmembrane protein impair the incorporation of Env proteins into mature virions. J. Virol. 67:213-221; references in Ref. Aguilar, H. C., W. F. Anderson, and P. M. Cannon. 2003. Cytoplasmic tail of Moloney murine leukemia virus envelope protein influences the conformation of the extracellular domain: implications for mechanism of action of the R Peptide. J. Virol. 77:1281-1291). In this respect, the gibbon envelope together with the other presently characterized simian envelopes can be considered as 'natural mutants' and be of use to study the refine molecular processes associated with envelope function and virus entry.

Whatever the underlying molecular events, the present functional characterization of the ERV-FRD envelopes is strongly suggestive of an 'ancestral' infection event by a functional retrovirus and its subsequent 'endogenization', with some of the envelope functions retained in evolution. Close examination of the primate FRD envelope sequences indeed reveals a prevalence of synonymous versus nonsynonymous substitutions (ratios for all pairwise comparisons >3, using the SNAP.pl program at the www.hiv-web.lanl.gov site), thus suggesting positive selection for a physiological function -which remains to be unraveled.

**Table 8. Infection assay of the HERV-FRD envelope ^{a}**

| Core | Env | Viral titer (CFU/mL) | | | | |
|---|---|---|---|---|---|---|
| | | NlH3T3 | G355-5 | TF671 | HeLa | 293T |
| MLV | FRD | 0 | 0 | 1.9x10¹ | 0 | 2.0 x 10¹ |
| | None | 0 | 0 | 0 | 0 | 6 |
| | Ampho | 4 x 10⁵ | 2 x 10⁶ | 4 x 10⁶ | 1 x 10⁶ | 1.1 x 10⁶ |
| | | | | | | |
| HIV | FRD | 1 | 1 x 10² | 2.1 x 10¹ | 0 | 8.8 x 10¹ |
| | None | 0 | 0 | 0 | 0 | 6 |
| | Ampho | 2 x 10⁵ | 1.4 x 10⁶ | 1 x 10³ | 1.2 x 10⁴ | 6 x 10⁴ |
| | | | | | | |
| SIV | FRD | 0 | 3.6 x 10² | 2.3 x 10² | 4 | 3.4 x 10² |
| | None | 0 | 5 | 0 | 0 | 6 |
| | Ampho | 5 x 10⁵ | 1.2 x 10⁶ | 6.8 x 10⁵ | 4.3 x 10⁵ | 8 x 10⁵ |

| | | | | | | |
|---|---|---|---|---|---|---|
| ^{a} experimental conditions as in Figure 8A; the retroviral particles (core) pseudotyped with the HERV-FRD envelope (FRD), no protein (none) or the amphotropic MLV envelope (Ampho), and the target test cells arc indicated : viral titers are each the means from two independent experiments. | | | | | | |

### Example 4

### Characterization of the immunosuppressive activity of the HERV-FRD envelope (Syncitin 2) and of the HERV-W envelope (Syncitin 1)

The assay for measuring the immunosuppressive activity was performed following the general procedure described in Mangeney & Heidmann, PNAS 1998 and Mangeney et al., J. Gen. Virol. 2001.

### Materials and Methods

### Mice and Cell Lines

The cell line used in the assay was MCA205(methylcholanthrene-induced murine fibrosarcoma cells (Shu and Rosemberg, 1985). Cells were cultured in DMEM supplemented with 10% fetal calf serum, streptomycin (100µg/ml) and penicillin (100units/ml).

In order to test the immunosuppressive effect of the envelope proteins, C57BL/6 and BALB/c mice, 8-12 wk-old, obtained from Janvier (Laval, France) were used.

### Constructions

pDFG-envHERV-FRD expression vector was constructed as follows. The full-length HERV-FRD envelope gene was PCR-amplified from the corresponding BAC DNA (accession number RP4761I2) obtained from BAC PAC resources (Oakland), using a proof reading DNA polymerase and with the 5' phosphate modified oligonucleotide TCGAGCTCTTAGGTCACATATGGGC (SEQ ID NO: 66) as forward primer and the *Mlu*I-containing oligonucleotide AACGGACGCGTCTCTGTCGTGTTCCC ACTA (SEQ ID NO: 67) as reverse primer. PCR was carried out for only 15 cycles (1 min at 94°C, 1 min at 60°C, 4 min at 72 °C), in 50 µl, using 100 ng of BAC DNA, 48 pmol of each primer, 200 µM of each dNTP, 2.5 µl PfuTurbo Hotstard polymerase and 1X Pfu reaction buffer (Stratagene). The PCR product was then cloned into the pDFG-MoTMtag vector (see below) opened at the *Age*I site blunt-ended by Klenow treatment and the MluI site.

pDFG-MoTMtag was constructed by ligating pDFG-MoTM (Mangeney & Heidmann, 1998) opened with *Bsu*361 and *Xho*I and a PCR fragment (digested with the same enzymes) generated using primers 5' CGTCTTGTCTGCTGCAGCATC-3' (SEQ ID NO: 68) and 5'-CATACTCGAGTCAACGCGTAGAATCGAGACCGAGGAGAGGGTTAGGGATAGGCT TACCGTACGGCTCGTACTCTATAGGCTTC-3' (SEQ ID NO: 69) and pDFGMoTM as a template.

pDFG-envHERV-W was constructed by inserting the *Age*I*-Dra*I Klenow treated fragment from pCR2.1 pH74 (Blond et al. J. Virol. (2000) Vol 74: 3321-3329) into the pDFG-MoTMTag vector digested by *Age*I and BsiWI and Klenow treated.

### Establishment of envelope-expressing tumor Cells and in vivo assay

Retroviral expression vectors (1.75 µg) were packaged by transient cotransfection into 7.5 x 10⁵ 293T cells with 1.75 µg of CMV-gag-pol-MoMLV and 0.55 µg CMV-envAmpho, using the calcium phosphate method. Supernatants were recovered 2 days later, filtered and used for infection of 5 x 10⁵ MCA205 tumor cells in the presence of 4 µg/ml polybrene, as described in Mangeney & Heidmann, 1988. Cells were maintained in selective medium (400 units/ml hygromycin) for 2 weeks. For in vivo assays, tumor cells were trypsinized, centrifuged and resuspended in PBS at a concentration of 1 x 10⁷ cells/ml. 100 µl of each suspension were injected s.c. in the shaved right flank of 3 C57/BL6 and 8 to 10 BALB/c mice. Tumor establishment was determined by palpation and tumor area (in mm²) was determined by measuring perpendicular tumor diameters.

The immunosuppression index is defined as i = (Sₑₙᵥ-Sₙₒₙₑ)/Sₙₒₙₑ, wherein Sₑₙᵥ is the maximum area reached by a tumour expressing an envelop protein and Sₙₒₙₑ is the maximum area reached by a tumor which does not express any envelop protein (negative control).

### Results

The results of the immunosuppression assay are presented in the following table:

| **Envelope** | **Immunosuppression Index (i)** |
|---|---|
| HERV-FRD (Syncitin 2) . | + 0,64 ± 0,36 |
| HERV-W (Synctin 1) | - 0,30 ± 0,06 |

It appears that whereas Synctin 2 presents a strong immunosuppressive activity, Synctin 1 is devoid of such an activity. Syncitin 2 is the first HERV envelope described to date with both immunosuppressive and fusogenic activity.

## Claims

1. Use of a protein or a polypeptide, being constituted by:
• a sequence SEQ ID NO: 2 (Syncitin 2), or
• a homologous sequence derived from SEQ ID NO: 2 presenting at least 85% amino acid identity with SEQ ID NO: 2, provided that said homologous sequence presents a fusogenic activity similar to that of Syncitin 2, and an immunosuppressive activity similar to that of Syncitin 2 and an antigenic activity similar to that of Syncitin 2,
for the manufacture of a drug useful for the prevention and/or the treatment of a placental morphogenesis disorder.

2. Use of a nucleic acid, **characterized in that** it encodes a protein or a polypeptide as defined in claim 1, for the manufacture of a drug useful for the prevention and/or the treatment a placental morphogenesis disorder.

3. Use of a nucleic acid according to claim 2, wherein said nucleic acid comprises or is constituted by sequence SEQ ID NO: 1.

## Patentansprüche

1. Verwendung eines Proteins oder eines Polypeptids, das aus
. einer Sequenz SEQ ID NO: 2 (Syncitin 2), oder
. einer homologen Sequenz besteht, die von SEQ ID NO: 2 abgeleitet ist, die mindestens 85 % Aminosäureidentität mit SEQ ID NO: 2 aufweist, vorausgesetzt, dass die homologe Sequenz eine fusogene Aktivität, die mit der von Syncitin 2 ahnlich ist und eine immunsuppressive Aktivität, die mit der von Syncitin 2 ahnlich ist, und eine antigene Aktivität, die mit der von Syncitin 2 ahnlich ist, aufweist,
zur Herstellung eines Arzneimittels, das zur Vorbeugung und/oder Behandlung einer Störung der Plazenta-Morphogenese nützlich ist.

2. Verwendung einer Nukleinsäure, **dadurch gekennzeichnet, dass** sie ein Protein oder ein Polypeptid kodiert, wie in Anspruch 1 definiert, zur Herstellung eines Arzneimittels, das zur Vorbeugung und/oder Behandlung einer Störung der Plazenta-Morphogenese nützlich ist.

3. Verwendung einer Nukleinsäure nach Anspruch 2, wobei die Nukleinsäure Sequenz SEQ ID NO: 1 umfasst oder aus ihr besteht.

## Revendications

1. Utilisation d'une protéine ou d'un polypeptide constitué
- d'une séquence SEQ ID NO : 2 (Syncitine 2), ou
- d'une séquence homologue dérivée de SEQ ID NO/2 présentant au moins 85% d'identité en acides aminés avec SEQ ID NO :2, sous réserve que ladite séquence homologue présente une activité fusogénique similaire à celle de la Syncitine 2, et une activité immunosuppressive similaire à celle de la Syncitine 2 et une activité antigénique similaire à celle de la Syncitine 2,
pour la préparation d'un médicament utile pour la prévention et/ou le traitement d'un désordre de la morphogenèse du placenta.

2. Utilisation d'un acide nucléique, **caractérisé en ce qu'**il code une protéine ou un polypeptide tel que défini dans la revendication 1, pour la préparation d'un médicament utile pour la prévention et/ou le traitement d'un désordre de la morphogenèse du placenta.

3. Utilisation d'un acide nucléique selon la revendication 2, où ledit acide nucléique comprend ou est constitué de la séquence SEQ ID NO : 1.
